# EUROPEAN PATENT APPLICATION

(11) **EP 4 818 160 A1**
(43) Date of publication of application: **07.10.2026**
(21) Application number: 25168060.9
(22) Date of filing: 02.04.2025
(51) Int. Cl.: C07D 233/64, A61P 1/16, A61P 1/18, A61P 9/00, A61P 11/14, A61P 13/12, C07C 53/06, C07C 53/134, C07C 55/07, C07C 55/08, C07C 55/10, C07C 55/12, C07C 55/14, C07C 59/06, C07D 213/80, A61K 31/4172, A61K 31/4406

(54) **SALTS AND SALT HYDRATES OF N,N'-BIS[2-(1H-IMIDAZOL-4-YL)ETHYL] OXALAMIDE FOR THE TREATMENT OF COUGH AND FIBROSIS OF LUNGS, LIVER, KIDNEYS, PANCREAS, AND HEART**

(71) Applicant: Treamid Therapeutics GmbH, 13125 Berlin (DE)
(72) Inventor: NEBOLSIN, Vladimir Evgenievich, 13125 Berlin (DE)
(74) Representative: Croce, Valeria

(57) **Abstract**

The invention relates to the chemistry of organic compounds, pharmacology, and medicine, and concerns the prevention and treatment of fibrosis of the liver, kidneys, pancreas, heart (myocardial fibrosis), and lungs, preferably of idiopathic origin, as well as to cough, particularly subchronic and chronic cough, and more specifically, cough associated with idiopathic pulmonary fibrosis (IPF), by means of salt forms of N,N'-bis[2-(1H-imidazol-4-yl)ethyl]oxalamide of formula (I) 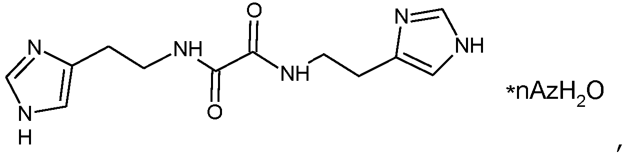 wherein A presents HCl, H₃PO₄, maleic acid, malonic acid, fumaric acid, HI, HBr, H₂SO₄, benzenesulfonic acid, tartaric acid, malic acid, p-toluenesulfonic acid, oxalic acid, glycolic acid, glutaric acid, camphorsulfonic acid, methanesulfonic acid, formic acid, ethanesulfonic acid, mandelic acid, nicotinic acid, succinic acid, and adipic acid; n represents 1 or 2, and z represents an integer from 0 to 3.

The present invention also relates to methods for preparing compounds of formula (I), pharmaceutical compositions comprising a therapeutically effective amount of the salt according to the invention, and methods of treatment.

## Description

### Field of the Invention

The present invention relates to the chemistry of organic compounds, pharmacology, and medicine, specifically to the salt forms of N,N'-bis[2-(1H-imidazol-4-yl)ethyl]oxalamide, hydrates and crystalline forms thereof.

More specifically, the invention relates to salts formed by N,N'-bis[2-(1H-imidazol-4-yl)ethyl]oxalamide and organic and inorganic acids, as well as hydrates and crystalline forms thereof, for the treatment of diseases accompanied by fibrotic formations and cough.

### Background of the Invention

Interstitial lung diseases (ILDs) represent a heterogeneous group of disorders characterized by different clinical courses and prognoses [N Engl J Med. 2020; 383(10):958-968]. However, studies of pulmonary fibrosis have established the existence of common mechanisms underlying its progression in various ILDs [Mol Aspects Med. 2019; 65:56-69]. Among chronic ILDs, idiopathic pulmonary fibrosis (IPF) is characterized by the most unfavorable disease course, with a median survival of no more than 3 years for 50% of patients [Lancet Respir Med. 2014; 2(7):566-572].

It was assumed that IPF is triggered by chronic inflammation, which promotes the recruitment of inflammatory cells and the release of cytokines, thereby leading to fibroblast proliferation and extracellular matrix deposition.

However, the use of anti-inflammatory agents in IPF has proven to be ineffective and sometimes even harmful [Curr Opin Pulm Med. 2012; 18:428-432; N Engl J. 2012; 366:1968-1977]. In the early stages of IPF pathogenesis, damage to type I alveolar cells occurs, leading to their apoptosis and the disruption of the alveolar epithelial layer [Springer Science. New York; 2014:161-205]. The surviving pneumocytes become pathologically activated and secrete profibrotic cytokines-transforming growth factor β (TGF-β), chemokines, and proteases, which act as mediators for the recruitment and activation of fibroblasts [Nat Rev Mol Cell Biol. 2006; 7(2):131-142].

The pathogenesis of IPF remains the subject of intensive research, with many aspects still unclear and its treatment far from being resolved [Am J Respir Crit Care Med. 2018; 198(5):e44-e68; Eur Respir J. 2018; 52(3):1801485]. Currently available medicaments for the treatment of IPF (pirfenidone and nintedanib) can only slow, but not stop, disease progression [Expert Opin Investig Drugs. 2020; 29(8):771-777]. Moreover, clinical observations of IPF patients indicate various adverse effects associated with the long-term use of these medicaments [Respir Investig. 2019; 57(4):300-311; Drug Healthc Patient Saf. 2020; 12:85-94; BMC Pulm Med. 2020; 20(1):3]. The administration of mesenchymal stem cells has, in rare cases, temporarily improved conditions of patients [Chest 2017; 151(5):971-981; Stem Cells Transl Med. 2020; 9(1):6-16]. However, cell therapy is associated with a high risk of neoplastic malignant transformation as well as significant temporary technological and financial burdens [Organogenesis 2011; 7:96-100]. The opportunities for lung transplantation are limited [Springer Science. New York; 2014:161-205] due to a shortage of donor organs, immune rejection, and potential surgical complications [Lancet Respir Med. 2019;7:271-282].

According to international studies, the prevalence of heart failure associated with myocardial fibrosis in developed countries is estimated at 1-2% of the adult population [Groenewegen A, Rutten FH, Mosterd A, Hoes AW. Epidemiology of heart failure. Eur J Heart Fail. 2020 Aug; 22(8):1342-1356. doi: 10.1002/ejhf.1858. Epub 2020 Jun 1. PMID: 32483830; PMCID: PMC7540043. Graziani F, Varone F, Crea F, Richeldi L. Treating heart failure with preserved ejection fraction: learning from pulmonary fibrosis. Eur J Heart Fail. 2018 Oct; 20(10):1385-1391. doi: 10.1002/ejhf.1286. Epub 2018 Aug 7. PMID: 30085383. Roger VL. Epidemiology of Heart Failure: A Contemporary Perspective. Circ Res. 2021 May 14; 128(10):1421-1434. doi: 10.1161/CIRCRESAHA.121.318172. Epub 2021 May 13. PMID: 33983838].

The prevalence of pancreatic fibrosis is steadily increasing, particularly in conjunction with the rising incidence of acute and chronic pancreatitis. Various studies report that pancreatic fibrosis is diagnosed in more than 40% of patients with pancreatic diseases. This condition is often a consequence of chronic pancreatitis, which may develop due to alcohol abuse, leading to significant replacement of the pancreatic parenchyma with connective tissue [Huang CT, Lin CK, Lee TH, Liang YJ. Pancreatic Fibrosis and Chronic Pancreatitis: Mini-Review of Non-Histologic Diagnosis for Clinical Applications. Diagnostics (Basel). 2020 Feb 7; 10(2):87. doi: 10.3390/diagnostics10020087. PMID: 32045995; PMCID: PMC7168241].

Currently, there is no specific antifibrotic therapy capable of directly targeting the process of cardiac and pancreatic fibrosis to prevent or substantially slow disease progression [Aghajanian, H., Kimura, T., Rurik, J.G. et al. Targeting cardiac fibrosis with engineered T cells. Nature 573, 430-433 (2019). https://doi.org/10.1038/s41586-019-1546-z, Shimizu, K. Mechanisms of pancreatic fibrosis and applications to the treatment of chronic pancreatitis. J Gastroenterol 43, 823-832 (2008). https://doi.org/10.1007/s00535-008-2249-7]. Existing therapeutic approaches focus on preserving organ function and reducing the risk of fatal outcomes; however, they do not directly affect the fibrotic process. In particular, the use of renin-angiotensin-aldosterone system blockers, diuretics, and cardiometabolic agents in the treatment of myocardial fibrosis is primarily aimed at improving cardiac function and reducing cardiovascular risk [Christian G Brilla, Renin-angiotensin-aldosterone system and myocardial fibrosis, Cardiovascular Research, Volume 47, Issue 1, July 2000, Pages 1-3, https://doi.org/10.1016/S0008-6363(00)00092-4, Morfino P, Aimo A, Castiglione V, Gálvez-Montón C, Emdin M, Bayes-Genis A. Treatment of cardiac fibrosis: from neuro-hormonal inhibitors to CAR-T cell therapy. Heart Fail Rev. 2023 Mar; 28(2):555-569. doi: 10.1007/s10741-022-10279-x. Epub 2022 Oct 11. PMID: 36221014; PMCID: PMC9553301]. Similarly, the use of enzyme preparations and dietary interventions is aimed at compensating for pancreatic insufficiency, but these measures do not promote tissue regeneration or reverse pancreatic fibrosis.

There is a need to develop new therapeutic strategies that can target the pathogenic mechanisms underlying fibrotic processes. Research in this field is ongoing, including studies of matrix metalloproteinase inhibitors and microRNA-132 for the treatment of myocardial fibrosis [DeLeon-Pennell KY, Meschiari CA, Jung M, Lindsey ML. Matrix Metalloproteinases in Myocardial Infarction and Heart Failure. Prog Mol Biol Transl Sci. 2017; 147:75-100. doi: 10.1016/bs.pmbts.2017.02.001. Epub 2017 Mar 18. PMID: 28413032; PMCID: PMC5576003, Ucar A, Gupta SK, Fiedler J, Erikci E, Kardasinski M, Batkai S, Dangwal S, Kumarswamy R, Bang C, Holzmann A, Remke J, Caprio M, Jentzsch C, Engelhardt S, Geisendorf S, Glas C, Hofmann TG, Nessling M, Richter K, Schiffer M, Carrier L, Napp LC, Bauersachs J, Chowdhury K, Thum T. The miRNA-212/132 family regulates both cardiac hypertrophy and cardiomyocyte autophagy. Nat Commun. 2012; 3:1078. doi: 10.1038/ncomms2090. PMID: 23011132; PMCID: PMC3657998], as well as antisense RNAs (siRNAgp46) and cholecystokinin receptor antagonists, for the treatment of pancreatic fibrosis [Ishiwatari H, Sato Y, Murase K, Yoneda A, Fujita R, Nishita H, Birukawa NK, Hayashi T, Sato T, Miyanishi K, Takimoto R, Kobune M, Ota S, Kimura Y, Hirata K, Kato J, Niitsu Y. Treatment of pancreatic fibrosis with siRNA against a collagen-specific chaperone in vitamin A-coupled liposomes. Gut. 2013 Sep; 62(9):1328-39. doi: 10.1136/gutjnl-2011-301746. Epub 2012 Nov 20. PMID: 23172890., Georgetown University. A Phase 1/ 2 Trial to Test the Safety of a CCK Receptor Antagonist, Proglumide, in Management of Chronic Pancreatitis Symptoms and Pain for 12 to 24 Months; Clinical trial registration NCT05551858; clinicaltrials.gov, 2024. https://clinicaltrials.gov/study/NCT05551858 (accessed 2025-02-28)].

At present, there is a significant unmet medical need for the development of an effective and safe medicament with a direct pathogenetic action that reduces collagen deposition in patients with fibrosis of various etiologies. For safety reasons, regardless of the route of administration, it is preferable that the medicament candidate exhibits low plasma exposure and preferential distribution to the target organs of fibrosis (lungs, liver, pancreas, and kidneys).

Experts estimate that the global prevalence of chronic cough (CC) approaches 10%; however, this figure is likely higher, as many epidemiological studies do not adequately capture symptoms and often fail to diagnose chronic cough. Chronic cough is associated with a substantial healthcare burden as well as reduced quality of life. Previously considered merely a symptom of comorbid conditions, chronic cough is now recognized as a distinct clinical entity. Therefore, enhanced education is needed to ensure that the diagnosis of chronic cough is not overlooked, and that appropriate treatment is provided. Unfortunately, there are few effective treatments for chronic cough, and many available therapies have serious side effects that limit their use. Most antitussive agents currently available act centrally for the treatment of pain or neuropathic conditions, and many are used off-label, exhibit limited efficacy, or cause significant adverse effects [Am J Manag Care. 2022; 28(suppl 9):S152-S158].

Chronic cough is observed in the majority of patients with idiopathic pulmonary fibrosis. It is a common symptom that is associated with reduced quality of life, an increased incidence of depression and anxiety, and disease progression. Currently, there is no pharmacological treatment for cough associated with IPF whose efficacy has been proven in clinical trials [Front. Rehabil. Sci., 18 October 2021, Sec. Pulmonary Rehabilitation, Volume 2-2021; https://doi.org/10.3389/fresc.2021.751798].

### SUMMARY OF THE INVENTION

The objective of the present invention is to develop a new medicament that is effective for the prevention and treatment of fibrosis in the liver, kidneys, heart (myocardial fibrosis), pancreas, and lungs, predominantly of idiopathic origin, and characterized by low variability in its pharmacokinetic parameters and preferential distribution to the target organs.

The technical result of the invention is the development and production of a highly effective medicament that exhibits high peak concentration and exposure in the fibrosis target organs (lungs, liver, heart, kidneys, pancreas), along with low variability in the mentioned pharmacokinetic parameters. This enables its use in fibrosis therapy.

An additional technical result is the development and manufacture of a medicament that maintains high stability during long-term storage at +25°C and 60% relative humidity, including under conditions of +30°C at 65% relative humidity and +45°C at 75% humidity.

Another technical result is the development and manufacture of a medicament for cough, particularly for subchronic and chronic cough, and more specifically for cough associated with idiopathic pulmonary fibrosis (IPF).

These technical results are achieved, by obtaining N,N'-bis[2-(1H-imidazol-4-yl)ethyl]oxalamide in the form of a salt of an acid and the base N,N'-bis[2-(1H-imidazol-4-yl)ethyl]oxalamide having the following structural Formula (I): 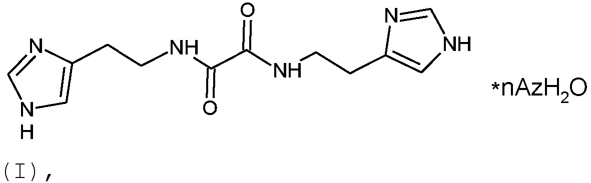 wherein A represents one of the following acids: HCl, H₃PO₄, maleic acid, malonic acid, fumaric acid, HI, HBr, H₂SO₄, benzenesulfonic acid, tartaric acid, malic acid, p-toluenesulfonic acid, oxalic acid, glycolic acid, glutaric acid, camphorsulfonic acid, methanesulfonic acid, formic acid, ethanesulfonic acid, mandelic acid, nicotinic acid, succinic acid, or adipic acid;
- n: represents 1 or 2; and
- z: represents an integer from 0 to 3.

Preferably, the compound of formula (I) is prepared as a pharmaceutically acceptable salt, which may be either anhydrous or in the form of a hydrate.

Preferably, the compound of formula (I) is produced in crystalline form.

As used herein, the term "hydrate" is understood as a product in which a water molecule(s) is associated with the salt. The hydrate may contain from 0.1 to 3 water molecules, preferably 0.5, 1, 1.5, 2, or 3 water molecules.

The compounds of general formula (I) possess the ability to slow down fibrotic processes in the organs and tissues of the body.

Other embodiments of the invention provide the dihydrate of the salt of hydrochloric acid and the base N,N'-bis[2-(1H-imidazol-4-yl)ethyl]oxalamide of Formula 1:

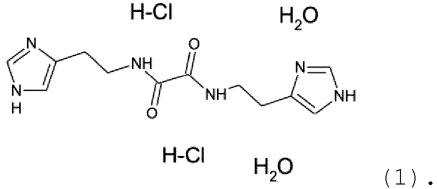

This crystalline form of N,N'-bis[2-(1H-imidazol-4-yl)ethyl]oxalamide dihydrochloride dihydrate is characterized by peak positions in the powder X-ray diffraction pattern at 2θ, deg. (± 0.2°): 21.7, 23.3, and 24.2. In a preferred embodiment, the crystalline form is characterized by peak positions in the powder X-ray diffraction pattern at 2θ, deg. (± 0.2°): 14.6, 21.1, 21.7, 23.3, 24.2, 27.3, 29.0, and 34.7. Additionally, this crystalline form is characterized by a monoclinic syngony, with unit cell edge lengths of 5.72 ± 0.50 Å, 24.20 ± 0.50 Å, 6.95 ± 0.50 Å, and an angle of 104.04° ± 5.00°.

The present technical problem is further solved, and the specified technical results are achieved, by a crystalline form of N,N'-bis[2-(1H-imidazol-4-yl)ethyl]oxalamide diphosphate of Formula 2:

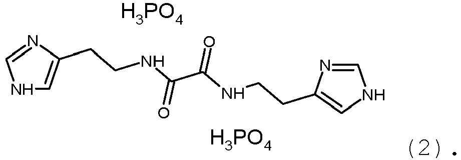

This form is characterized by peak positions at 2θ, deg. (±0.2°): 20.4, 23.3, and 24.7. In a preferred embodiment, the crystalline form is characterized by peak positions at 2θ, deg. (±0.2°): 6.5, 12.5, 20.2, 20.4, 23.3, 23.4, 23.8, and 24.7. Additionally, this crystalline form is characterized by a triclinic syngony, with unit cell edge lengths of 4.90 ± 0.50 Å, 7.43 ± 0.50 Å, and 14.02 ± 0.50 Å, and with angles of 103.84° ± 5.00°, 94.02° ± 5.00°, and 97.90° ± 5.00°.

The present technical problem is further solved, and the specified technical results are achieved, by a crystalline form of N,N'-bis[2-(1H-imidazol-4-yl)ethyl]oxalamide dimaleinate dihydrate of Formula 3:

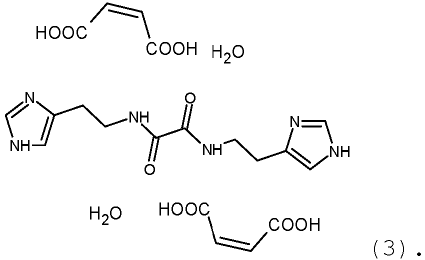

This form is characterized by peak positions at 2θ, deg., (±0.2°): 9.3, 25.4, and 29.9. In a preferred embodiment, the crystalline form is characterized by peak positions at 2θ, deg., (±0.2°): 9.3, 18.4, 18.6, 21.2, 22.2, 25.1, 25.4, and 29.9. Additionally, this crystalline form is characterized by a triclinic syngony, with unit cell edge lengths of 4.97 ± 0.50 Å, 10.32 ± 0.50 Å, and 13.53 ± 0.50 Å, and with angles of 109.72° ± 5.00°, 96.62° ± 5.00°, and 98.80° ± 5.00°.

The present technical problem is further solved, and the specified technical results are achieved, by a crystalline form of N,N'-bis[2-(1H-imidazol-4-yl)ethyl]oxalamide dimalonate of Formula 4:

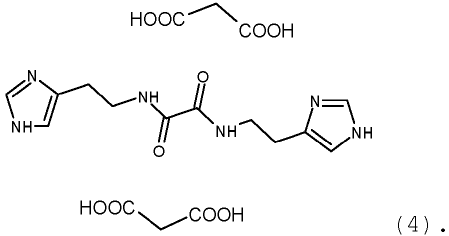

This form is characterized by peak positions at 2θ, degrees (±0.2°): 6.5, 23.3, and 31.1. In a preferred embodiment, the crystalline form is characterized by peak positions at 2θ, degrees (±0.2°): 6.5, 13.9, 19.7, 23.3, 23.5, 25.5, 26.2, 31.1, and 37.3. Additionally, the crystalline form is characterized by a triclinic syngony, with unit cell edge lengths of 4.59 ± 0.50 Å, 8.53 ± 0.50 Å, and 14.01 ± 0.50 Å, and with angles of 103.91° ± 5.00°, 92.18° ± 5.00°, and 96.82° ± 5.00°.

The present technical problem is further solved, and the specified technical results are achieved, by a crystalline form of N,N'-bis[2-(1H-imidazol-4-yl)ethyl]oxalamide fumarate of Formula 5:

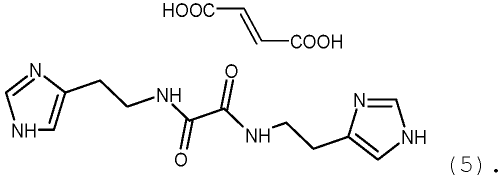

This form is characterized by peak positions at 2θ, degrees (±0.2°): 21.3, 23.6, and 27.5. In a preferred embodiment, the crystalline form is characterized by peak positions at 2θ, degrees (±0.2°): 16.5, 18.8, 18.9, 21.3, 21.4, 22.0, 22.5, 23.6, 24.3, 27.5, 28.1, and 28.6. Additionally, this crystalline form is characterized by a monoclinic syngony, with unit cell edge lengths of 12.87 ± 0.50 Å, 5.10 ± 0.50 Å, and 14.45 ± 0.50 Å, and with an angle of 104.36° ± 5.00°.

The present technical problem is further solved, and the specified technical results are achieved, by a crystalline form of N,N'-bis[2-(1H-imidazol-4-yl)ethyl]oxalamide dihydroiodide dihydrate of Formula 6:

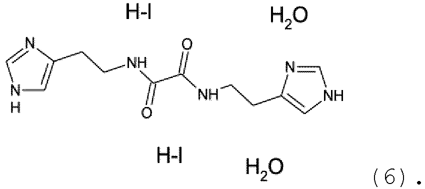

This form is characterized by peak positions at 2θ, degrees (±0.2°): 20.4, 22.6, and 26.2. In a preferred embodiment, the crystalline form is characterized by peak positions at 2θ, degrees (±0.2°): 18.2, 20.4, 22.6, 23.6, 26.2, 31.3, 33.3, and 36.5. Additionally, this crystalline form is characterized by a triclinic syngony, with unit cell edge lengths of 7.76 ± 0.50 Å, 8.04 ± 0.50 Å, and 8.99 ± 0.50 Å, and with angles of 88.05° ± 5.00°, 74.66° ± 5.00°, and 70.66° ± 5.00°.

The present technical problem is further solved, and the specified technical results are achieved, by a crystalline form of N,N'-bis[2-(1H-imidazol-4-yl)ethyl]oxalamide dihydrobromide dihydrate of Formula 7:

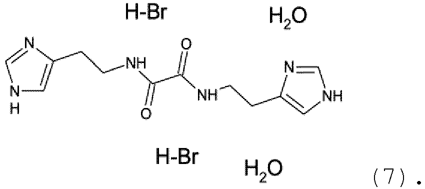

This form is characterized by peak positions at 2θ, degrees (±0.2°): 22.0, 22.8, and 25.0. In a preferred embodiment, the crystalline form is characterized by peak positions at 2θ, degrees (±0.2°): 12.0, 20.5, 21.2, 22.0, 22.8, 23.2, 23.7, 24.3, 25.0, 26.8, and 31.8. Additionally, this crystalline form is characterized by a triclinic syngony, with unit cell edge lengths of 5.11 ± 0.50 Å, 7.80 ± 0.50 Å, and 12.65 ± 0.50 Å, and with angles of 104.78° ± 5.00°, 96.31° ± 5.00°, and 100.38° ± 5.00°.

The present technical problem is further solved, and the specified technical results are achieved, by a crystalline form of N,N'-bis[2-(1H-imidazol-4-yl)ethyl]oxalamide sulfate trihydrate of Formula 8:

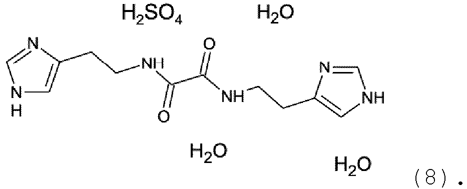

This form is characterized by peak positions at 2θ, degrees (±0.2°): 6.6, 21.3, and 24.5. In a preferred embodiment, the crystalline form is characterized by peak positions at 2θ, degrees (±0.2°): 6.3, 6.6, 11.9, 13.2, 15.9, 18.2, 21.1, 21.3, 22.9, 23.6, 24.5, 26.0, and 27.4. Additionally, this crystalline form is characterized by a monoclinic syngony, with unit cell edge lengths of 8.22 ± 0.50 Å, 8.77 ± 0.50 Å, and 27.13 ± 0.50 Å, and with an angle of 97.61° ± 5.00°.

The present technical problem is further solved, and the specified technical results are achieved, by a crystalline form of N,N'-bis[2-(1H-imidazol-4-yl)ethyl]oxalamide dibenzenesulfonate of Formula 9:

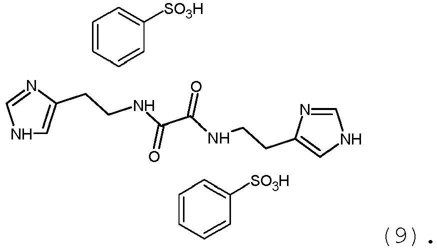

This form is characterized by peak positions at 2θ, degrees (±0.2°): 20.9, 21.6, and 22.4. In a preferred embodiment, the crystalline form is characterized by peak positions at 2θ, degrees (±0.2°): 13.3, 16.2, 17.9, 18.8, 20.9, 21.6, 22.4, 22.6, 25.0, 25.1, and 26.6. Additionally, this crystalline form is characterized by a monoclinic syngony, with unit cell edge lengths of 9.61 ± 0.50 Å, 10.74 ± 0.50 Å, and 12.99 ± 0.50 Å, and with an angle of 101.92° ± 5.00°.

The present technical problem is further solved, and the specified technical results are achieved, by a crystalline form of N,N'-bis[2-(1H-imidazol-4-yl)ethyl]oxalamide L,D-tartrate of Formula 10:

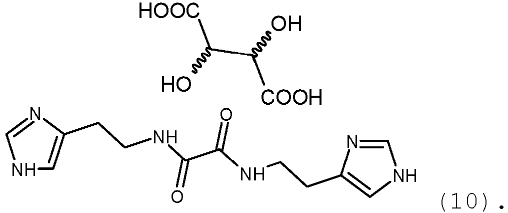

This form is characterized by peak positions at 2θ, degrees (±0.2°): 6.9, 19.9, and 23.6. In a preferred embodiment, the crystalline form is characterized by peak positions at 2θ, degrees (±0.2°): 6.9, 10.8, 18.4, 19.9, 21.1, 21.5, and 27.2. Additionally, this crystalline form is characterized by a triclinic syngony, with unit cell edge lengths of 9.00 ± 0.50 Å, 9.16 ± 0.50 Å, and 13.54 ± 0.50 Å, and with angles of 108.59° ± 5.00°, 91.87° ± 5.00°, and 112.10° ± 5.00°.

The present technical problem is further solved, and the specified technical results are achieved, by a crystalline form of N,N'-bis[2-(1H-imidazol-4-yl)ethyl]oxalamide L-malate of Formula 11:

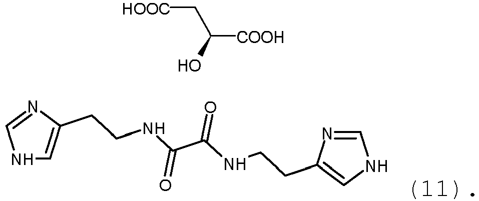

This form is characterized by peak positions at 2θ, degrees (±0.2°): 19.3, 24.0, and 25.3. In a preferred embodiment, the crystalline form is characterized by peak positions at 2θ, degrees (±0.2°): 7.0, 11.5, 17.1, 19.3, 21.4, 24.0, 24.8, 25.3, and 32.6. Additionally, this crystalline form is characterized by a triclinic syngony with, unit cell edge lengths of 4.68 ± 0.50 Å, 7.81 ± 0.50 Å, and 12.74 ± 0.50 Å, and with angles of 81.64° ± 5.00°, 81.79° ± 5.00°, and 80.66° ± 5.00°.

The present technical problem is further solved, and the specified technical results are achieved, by a crystalline form of N,N'-bis[2-(1H-imidazol-4-yl)ethyl]oxalamide di-p-toluenesulfonate of Formula 12:

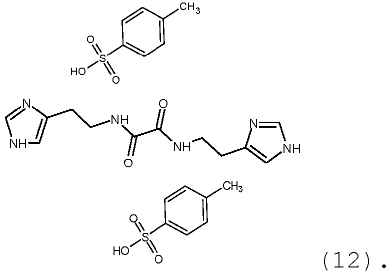

This form is characterized by peak positions at 2θ, degrees (±0.2°): 7.4, 15.7, and 23.1. In a preferred embodiment, the crystalline form is characterized by peak positions at 2θ, degrees (±0.2°): 7.4, 12.3, 13.2, 14.9, 15.7, 18.4, 18.5, 21.4, 22.3, 23.1, 24.6, 25.4, 25.7, 25.9, 26.7, 27.0, 27.6, 28.2, 30.2, 32.2, and 33.3. Additionally, this crystalline form is characterized by a monoclinic syngony, with unit cell edge lengths of 7.25 ± 0.50 Å, 8.06 ± 0.50 Å, and 23.86 ± 0.50 Å, and with angles of 90° ± 5.00°, 95.81° ± 5.00°, and 90° ± 5.00°.

The present technical problem is further solved, and the specified technical results are achieved, by a crystalline form of N,N'-bis[2-(1H-imidazol-4-yl)ethyl]oxalamide oxalate of Formula 13:

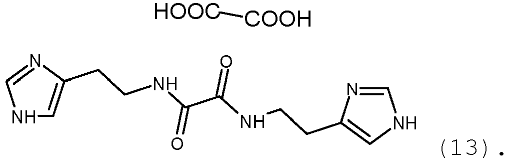

This form is characterized by peak positions at 2θ, degrees (±0.2°): 16.5, 16.6, and 18.7. In a preferred embodiment, the crystalline form This crystalline form is characterized by peak positions at 2θ, degrees (±0.2°): 10.4, 16.5, 16.6, 18.7, 20.8, 22.0, 25.1, 25.3, 26.4, 33.6, and 43.2. Additionally, this crystalline form is characterized by a monoclinic syngony, with unit cell edge lengths of 14.91 ± 0.50 Å, 21.31 ± 0.50 Å, and 22.43 ± 0.50 Å, and with an angle of 22.43° ± 5.00°.

The present technical problem is further solved, and the specified technical results are achieved, by a crystalline form of N,N'-bis[2-(1H-imidazol-4-yl)ethyl]oxalamide dioxalate of Formula 14:

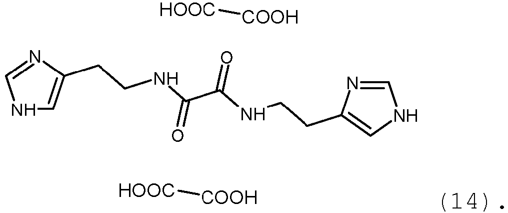

This form is characterized by peak positions at 2θ, degrees (±0.2°): 24.1, 25.4, and 27.5. In a preferred embodiment, the crystalline form is characterized by peak positions at 2θ, degrees (±0.2°): 19.3, 22.4, 24.1, 25.4, 27.5, and 30.0. Additionally, this crystalline form is characterized by a monoclinic syngony, with unit cell edge lengths of 10.84 ± 0.50 Å, 5.64 ± 0.50 Å, and 16.10 ± 0.50 Å, and with an angle of 100.51° ± 5.00°.

The present technical problem is further solved, and the specified technical results are achieved, by a crystalline form of N,N'-bis[2-(1H-imidazol-4-yl)ethyl]oxalamide diadipinate monohydrate of Formula 15:

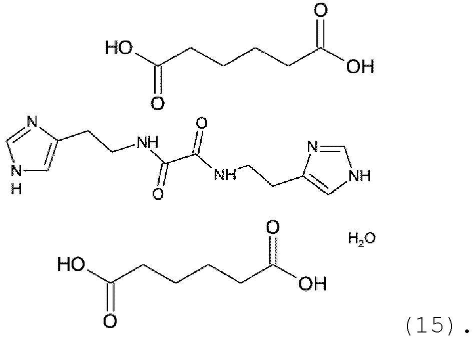

This form is characterized by peak positions at 2θ, degrees (±0.2°): 19.5, 23.7, and 25.8. In a preferred embodiment, the crystalline form is characterized by peak positions at 2θ, degrees (±0.2°): 14.4, 18.9, 19.5, 20.9, 22.7, 23.7, 25.8, and 36.2. Additionally, this crystalline form is characterized by triclinic syngony, with unit cell edge lengths of 4.99 ± 0.50 Å, 7.92 ± 0.50 Å, and 18.33 ± 0.50 Å, and with angles of 82.98° ± 5.00°, 88.26° ± 5.00°, and 77.81° ± 5.00°.

The present technical problem is further solved, and the specified technical results are achieved, by a crystalline form of N,N'-bis[2-(1H-imidazol-4-yl)ethyl]oxalamide dimethanesulfonate dihydrate of Formula 16:

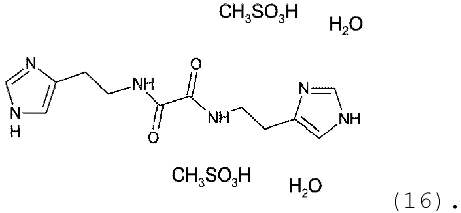

This form is characterized by peak positions at 2θ, degrees (±0.2°): 6.4, 22.5, and 23.9. In a preferred embodiment, the crystalline form is characterized by peak positions at 2θ, degrees (±0.2°): 6.4, 12.8, 19.0, 19.3, 22.3, 22.5, 22.9, 23.1, 23.9, 25.8, and 33.1. Additionally, this crystalline form is characterized by a triclinic syngony, with unit cell edge lengths of 5.85 ± 0.50 Å, 7.19 ± 0.50 Å, and 14.02 ± 0.50 Å, and with angles of 97.43° ± 5.00°, 98.30° ± 5.00°, and 102.48° ± 5.00°.

The present technical problem is further solved, and the specified technical results are achieved, by a crystalline form of N,N'-bis[2-(1H-imidazol-4-yl)ethyl]oxalamide succinate of Formula 17:

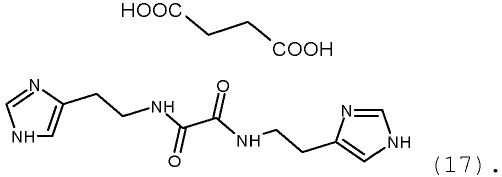

This form is characterized by peak positions at 2θ, degrees (±0.2°): 6.2, 21.2 and 26.3. In a preferred embodiment, the crystalline form is characterized by peak positions at 2θ, degrees (±0.2°): 6.2, 16.9, 18.4, 18.6, 19.4, 20.4, 21.2, 26.3, 24.4, and 25.6.

Additionally, this crystalline form is characterized by a monoclinic syngony, with unit cell edge lengths of 13.02 ± 0.50 Å, 5.08 ± 0.50 Å, and 14.51 ± 0.50 Å, and with an angle of 104.87° ± 5.00°.

The present technical problem is further solved, and the specified technical results are achieved, by a crystalline form of N,N'-bis[2-(1H-imidazol-4-yl)ethyl]oxalamide di-D-malate of Formula 18:

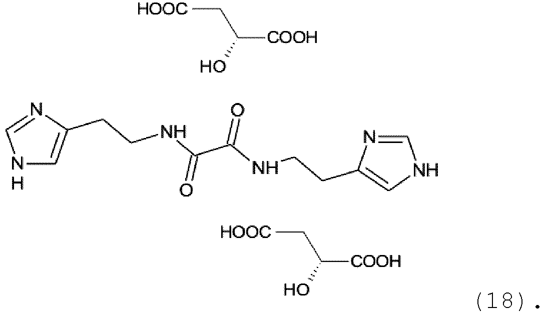

This form is characterized by peak positions at 2θ, degrees (±0.2°): 22.1, 22.9, and 23.6. In a preferred embodiment, the crystalline form is characterized by peak positions at 2θ, degrees (±0.2°): 5.9, 15.7, 19.5, 20.3, 21.4, 22.1, 22.9, 23.3, 23.6, 24.4, 28.0, and 32.3. Additionally, this crystalline form is characterized by triclinic syngony, with unit cell edge lengths of 5.03 ± 0.50 Å, 7.83 ± 0.50 Å, and 15.05 ± 0.50 Å, and with angles of 83.89° ± 5.00°, 84.35° ± 5.00°, and 78.31° ± 5.00°.

The present technical problem is further solved, and the specified technical results are achieved, by a crystalline form of N,N'-bis[2-(1H-imidazol-4-yl)ethyl]oxalamide D-malate of Formula 19:

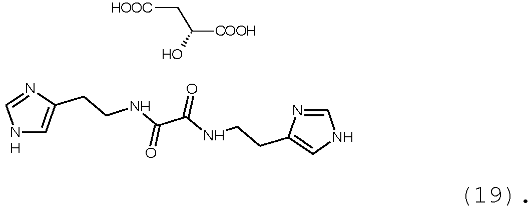

This form is characterized by peak positions at 2θ, degrees (±0.2°): 19.4, 23.5 and 24.1. In a preferred embodiment, the crystalline form is characterized by peak positions at 2θ, degrees (±0.2°): 17.1, 19.4, 21.5, 22.1, 23.0, 23.3, 23.5, 24.1, 24.8, 25.4, and 28.5. Additionally, this crystalline form is characterized by a monoclinic syngony, with unit cell edge lengths of 9.54 ± 0.50 Å, 11.86 ± 0.50 Å, and 16.02 ± 0.50 Å, and with an angle of 101.01° ± 5.00°.

The present invention also relates to the use of a salt of an acid and the base N,N'-bis[2-(1H-imidazol-4-yl)ethyl]oxalamide of Formula (I) as a medicament for the treatment and/or prevention of fibrosis of the liver, kidneys, pancreas, and heart (myocardial fibrosis). Furthermore, the invention relates to the use of a salt of an acid and the base N,N'-bis[2-(1H-imidazol-4-yl)ethyl]oxalamide of Formula (I) as a medicament for the prevention and treatment of lung fibrosis, preferably of idiopathic origin.

The present invention also relates to the use of a salt of an acid and the base N,N'-bis[2-(1H-imidazol-4-yl)ethyl]oxalamide of Formula (I) for the preparation of a pharmaceutical composition for the treatment and/or prevention of fibrosis of the liver, kidneys, pancreas, and heart (myocardial fibrosis). Furthermore, the invention relates to the use of a salt of an acid and the base N,N'-bis[2-(1H-imidazol-4-yl)ethyl]oxalamide of Formula (I) as a medicament for the prevention and treatment of lung fibrosis, preferably of idiopathic origin.

The present invention also relates to the use of a salt of an acid and the base N,N'-bis[2-(1H-imidazol-4-yl)ethyl]oxalamide of Formula (I) in the manufacture of a medicament for the treatment and/or prevention of fibrosis of the liver, kidneys, pancreas, and heart (myocardial fibrosis). Furthermore the invention relates to the use of a salt of an acid and the base N,N'-bis[2-(1H-imidazol-4-yl)ethyl]oxalamide of Formula (I) in the manufacture of a medicament for the prevention and treatment of lung fibrosis, preferably of idiopathic origin.

The present invention also relates to the use of a salt of an acid and the base N,N'-bis[2-(1H-imidazol-4-yl)ethyl]oxalamide of Formula (I) as a medicament for the treatment and/or prevention of cough, particularly subchronic and chronic cough, and more particularly cough associated with idiopathic pulmonary fibrosis (IPF).

The present invention also relates to the use of a salt of an acid and the base N,N'-bis[2-(1H-imidazol-4-yl)ethyl]oxalamide of Formula (I) for the preparation of a pharmaceutical composition for the treatment and/or prevention of cough, particularly subchronic and chronic cough, and more particularly cough associated with idiopathic pulmonary fibrosis (IPF).

The present invention also relates to the use of a salt of an acid and the base N,N'-bis[2-(1H-imidazol-4-yl)ethyl]oxalamide of Formula (I) in the manufacture of a medicament for the treatment and/or prevention of cough, particularly subchronic and chronic cough, and more particularly cough associated with idiopathic pulmonary fibrosis (IPF).

Furthermore, the invention provides a pharmaceutical composition for the treatment and/or prevention of fibrosis of the liver, kidneys, pancreas, heart (myocardial fibrosis), and lungs, preferably of idiopathic origin, wherein the pharmaceutical composition is characterized by comprising an effective amount of a salt of an acid and the base N,N'-bis[2-(1H-imidazol-4-yl)ethyl]oxalamide of Formula (I) and at least one pharmaceutically acceptable auxiliary substance. In some embodiments, the auxiliary substance is a pharmaceutically acceptable carrier and/or excipient.

Furthermore, the invention provides a pharmaceutical composition for the treatment and/or prevention of cough, particularly subchronic and chronic cough, and more particularly cough associated with IPF, wherein the pharmaceutical composition is characterized by comprising an effective amount of a salt of an acid and the base N,N'-bis[2-(1H-imidazol-4-yl)ethyl]oxalamide of Formula (I) and at least one pharmaceutically acceptable auxiliary substance. In some embodiments, the auxiliary substance is a pharmaceutically acceptable carrier and/or excipient.

The specified technical results are achieved by a medicament that comprises a salt of an acid and the base N,N'-bis[2-(1H-imidazol-4-yl)ethyl]oxalamide of Formula (I).

The invention also encompasses a method for treating and/or preventing fibrosis, particularly fibrosis of the liver, kidneys, pancreas, heart (myocardial fibrosis), and lungs, preferably of idiopathic origin, comprising administering said pharmaceutical composition or medicament to a subject.

The invention also provides a method for treating and/or preventing cough, particularly subchronic and chronic cough, and more particularly cough associated with IPF, comprising administering said pharmaceutical composition or medicament to a subject.

One embodiment of the invention provides a method for preparing a crystalline form of a compound of Formula (I), comprising the steps of:
a) providing N,N'-bis[2-(1H-imidazol-4-yl)ethyl]oxalamide;
b) preparing a solution of an acid in water and/or an organic solvent and/or a mixture of solvents;
c) mixing the components from steps (a) and (b) to obtain a solution of a salt of N,N'-bis[2-(1H-imidazol-4-yl)ethyl]oxalamide;
d) crystallizing the obtained salt by diluting the reaction mixture with an organic solvent;
e) isolating the obtained salt by removing the solvent from the reaction mixture under vacuum, dissolving the residue in isopropanol, and maintaining the resulting solution at +5°C until salt crystals precipitate;
f) isolating the obtained salt by crystallization from water; and
g) separating the salt crystals from the solvent.

In some embodiments of the method, the acid may be added at room temperature, or at heating or cooling each of the components; any order of mixing the components may also be used.

In some embodiments of the invention, the solvent used in step (b) as the medium for dissolving the acid is water.

In some embodiments of the invention, N,N'-bis[2-(1H-imidazol-4-yl)ethyl]oxalamide is introduced in step (a) in solid form.

In some embodiments of the invention, N,N'-bis[2-(1H-imidazol-4-yl)ethyl]oxalamide is introduced in step (a) as a suspension or a solution in an organic solvent.

In some embodiments of the invention, the solvent used in step (d) for precipitating the salt is acetone.

In some embodiments of the invention, the solvent used in step (e) for crystallizing the salt is isopropanol.

In some embodiments of the invention, the solvent used in step (f) for crystallizing the salt is water.

In some embodiments of the invention, after step (d), the salt undergoes additional recrystallization.

If the salt is obtained using a solution in step (a), after step (d), an additional step of crystal nucleation is performed. Crystal nucleation may be achieved, by introducing crystals of a salt of an acid and the base N,N'-bis[2-(1H-imidazol-4-yl)ethyl]oxalamide into the solution or by any other known method.

### BRIEF DESCRIPTION OF DRAWINGS

Fig. 1. General view of the unit cell of C₁₂H₁₆N₆O₂*2HCl*2H₂O. Experimental X-ray diffraction pattern of N,N'-bis[2-(1H-imidazol-4-yl)ethyl]oxalamide dihydrochloride dihydrate (C₁₂H₁₆N₂O₂*2HCl*2H₂O) .
Fig. 2. General view of the unit cell of C₁₂H₁₆N₆O₂*2H₃PO₄. Experimental X-ray diffraction pattern of N,N'-bis[2-(1H-imidazol-4-yl) ethyl]oxalamide diphosphate (C₁₂H₁₆N₆O₂*2H₃PO₄).
Fig. 3. General view of the unit cell of C₁₂H₁₆N₆O₂*2C₄H₄O₄*2H₂O. Experimental X-ray diffraction pattern of N,N'-bis[2-(1H-imidazol-4-yl) ethyl]oxalamide dimaleinate dihydrate (C₁₂H₁₆N₆O₂*2C₄H₄O₄*2H₂O).
Fig. 4. General view of the unit cell of C₁₂H₁₆N₆O₂*2C₃H₄O₄. Experimental X-ray diffraction pattern of N,N'-bis[2-(1H-imidazol-4-yl)ethyl]oxalamide dimalonate (C₁₂H₁₆N₆O₂*2C₃H₄O₄) .
Fig. 5. General view of the unit cell of C₁₂H₁₆N₆O₂*C₄H₄O₄. Experimental X-ray diffraction pattern of N,N'-bis[2-(1H-imidazol-4-yl)ethyl]oxalamide fumarate (C₁₂H₁₆N₆O₂*C₄H₄O₄).
Fig. 6. General view of the unit cell of N,N'-bis[2-(1H-imidazol-4-yl)ethyl]oxalamide dihydroiodide dihydrate (C₁₂H₁₆N₆O₂*2HI*2H₂O). Experimental X-ray diffraction pattern of N,N'-bis[2-(1H-imidazol-4-yl)ethyl]oxalamide dihydroiodide (C₁₂H₁₆N₆O₂*2HI).
Fig. 7. General view of the unit cell of N,N'-bis[2-(1H-imidazol-4-yl)ethyl]oxalamide dihydrobromide dihydrate (C₁₂H₁₆N₆O₂*2HBr*2H₂O) . Experimental X-ray diffraction pattern of N,N'-bis[2-(1H-imidazol-4-yl)ethyl]oxalamide dihydrobromide (C₁₂H₁₆N₆O₂*2HBr).
Fig. 8. General view of the unit cell of C₁₂H₁₆N₆O₂*H₂SO₄*3H₂O. Experimental X-ray diffraction pattern of N,N'-bis[2-(1H-imidazol-4-yl) ethyl]oxalamide sulfate trihydrate (C₁₂H₁₆N₆O₂*H₂SO₄*3H₂O).
Fig. 9. General view of the unit cell of C₁₂H₁₆N₆O₂*2C₆H₆O₃S. Experimental X-ray diffraction pattern of N,N'-bis[2-(1H-imidazol-4-yl) ethyl]oxalamide dibenzenesulfonate (C₁₂H₁₆N₆O₂*2C₆H₆O₃S).
Fig. 10. General view of the unit cell of C₁₂H₁₆N₆O₂*C₄H₆O₆. Experimental X-ray diffraction pattern of N,N'-bis[2-(1H-imidazol-4-yl)ethyl]oxalamide D,L-tartrate (C₁₂H₁₆N₆O₂*C₄H₆O₆).
Fig. 11. General view of the unit cell of C₁₂H₁₆N₆O₂*C₄H₆O₅. Experimental X-ray diffraction pattern of N,N'-bis[2-(1H-imidazol-4-yl) ethyl]oxalamide L-malate (C₁₂H₁₆N₆O₂*C₄H₆O₅).
Fig. 12. General view of the unit cell of C₁₂H₁₆N₆O₂*2C₇H₈O₃S. Experimental X-ray diffraction pattern of N,N'-bis[2-(1H-imidazol-4-yl) ethyl]oxalamide di-p-toluenesulfonate (C₁₂H₁₆N₆O₂*2C₇H₈O₃S).
Fig. 13. General view of the unit cell of C₁₂H₁₆N₆O₂*C₂H₂O₄. Experimental X-ray diffraction pattern of N,N'-bis[2-(1H-imidazol-4-yl)ethyl]oxalamide oxalate (C₁₂H₁₆N₆O₂*C₂H₂O₄).
Fig. 14. General view of the unit cell of C₁₂H₁₆N₆O₂*2C₂H₂O₄. Experimental X-ray diffraction pattern of N,N'-bis[2-(1H-imidazol-4-yl)ethyl]oxalamide dioxalate (C₁₂H₁₆N₆O₂*2C₂H₂O₄).
Fig. 15. General view of the unit cell of C₁₂H₁₆N₆O₂*2C₆H₁₀O₄*H₂O. Experimental X-ray diffraction pattern of N,N'-bis[2-(1H-imidazol-4-yl)ethyl]oxalamide diadipinate monohydrate (C₁₂H₁₆N₆O₂*2C₆H₁₀O₄*H₂O).
Fig. 16. General view of the unit cell of C₁₂H₁₆N₆O₂*C₄H₆O₄. Experimental X-ray diffraction pattern of N,N'-bis[2-(1H-imidazol-4-yl) ethyl] oxalamide succinate (C₁₂H₁₆N₆O₂*C₄H₆O₄).
Fig. 17. General view of the unit cell of C₁₂H₁₆N₆O₂*C₄H₆O₅*2H₂O. Experimental X-ray diffraction pattern of N,N'-bis[2-(1H-imidazol-4-yl)ethyl]oxalamide dimethanesulfonate dihydrate (C₁₂H₁₆N₆O₂*2CH₄O₃S*2H₂O).
Fig. 18. General view of the unit cell of C₁₂H₁₆N₆O₂*2C₄H₆O₅. Experimental X-ray diffraction pattern of N,N'-bis[2-(1H-imidazol-4-yl)ethyl]oxalamide di-D-malate (C₁₂H₁₆N₆O₂*2C₄H₆O₅).
Fig. 19. General view of the unit cell of C₁₂H₁₆N₆O₂*C₄H₆O5. Experimental X-ray diffraction pattern of N,N'-bis[2-(1H-imidazol-4-yl) ethyl] oxalamide D-malate (C₁₂H₁₆N₆O₂*C₄H₆O₅).
Fig. 20. Experimental X-ray diffraction pattern of N,N'-bis[2-(1H-imidazol-4-yl)ethyl]oxalamide disulfate (C₁₂H₁₆N₆O₂*2H₂SO₄).
Fig. 21. Experimental X-ray diffraction pattern of N,N'-bis[2-(1H-imidazol-4-yl)ethyl]oxalamide L-tartrate (C₁₂H₁₆N₆O₂*C₄H₆O₆).
Fig. 22. Experimental X-ray diffraction pattern of N,N'-bis[2-(1H-imidazol-4-yl)ethyl]oxalamide D-tartrate (C₁₂H₁₆N₆O₂*C₄H₆O₆).
Fig. 23. Experimental X-ray diffraction pattern of N,N'-bis[2-(1H-imidazol-4-yl)ethyl]oxalamide diglycolate (C₁₂H₁₆N₆O₂*2C₂H₄O₃).
Fig. 24. Experimental X-ray diffraction pattern of N,N'-bis[2-(1H-imidazol-4-yl)ethyl]oxalamide glutarate dihydrate (C₁₂H₁₆N₆O₂*C₅H₈O₄*2H₂O).
Fig. 25. Experimental X-ray diffraction pattern of N,N'-bis[2-(1H-imidazol-4-yl)ethyl]oxalamide dicamphorsulfonate (C₁₂H₁₆N₆O₂*2C₁₀H₁₆O₄S).
Fig. 26. Experimental X-ray diffraction pattern of N,N'-bis[2-(1H-imidazol-4-yl)ethyl]oxalamide diformiate (C₁₂H₁₆N₆O₂*2CH₂O₂).
Fig. 27. Experimental X-ray diffraction pattern of N,N'-bis[2-(1H-imidazol-4-yl)ethyl]oxalamide diethanesulfonate (C₁₂H₁₆N₆O₂*2C₂H₆O₃S).
Fig. 28. Experimental X-ray diffraction pattern of N,N'-bis[2-(1H-imidazol-4-yl)ethyl]oxalamide malonate dihydrate (C₁₂H₁₆N₆O₂*C₃H₄O₄*2H₂O).
Fig. 29. Experimental X-ray diffraction pattern of N,N'-bis[2-(1H-imidazol-4-yl)ethyl]oxalamide dimandelate (C₁₂H₁₆N₆O₂*2C₈H₈O₃).
Fig. 30. Experimental X-ray diffraction pattern of N,N'-bis[2-(1H-imidazol-4-yl)ethyl]oxalamide dinicotinate (C₁₂H₁₆N₆O₂*2C₆H₅NO₂).
Fig. 31. Effect of N,N'-bis[2-(1H-imidazol-4-yl)ethyl]oxalamide dihydrochloride dihydrate on the hydroxyproline level in a bleomycin-induced pulmonary fibrosis model.
Fig. 32. Effect of N,N'-bis[2-(1H-imidazol-4-yl)ethyl]oxalamide dihydrochloride dihydrate on lung fibrosis in a bleomycin-induced pulmonary fibrosis model as assessed by a modified Ashcroft scale.
Fig. 33. Effect of intranasal administration of an N,N'-bis[2-(1H-imidazol-4-yl)ethyl]oxalamide dihydrochloride dihydrate solution on the number of coughs in a capsaicin inhalation-induced cough model in guinea pigs.
Fig. 34. Effect of intrapharyngeal administration of an N,N'-bis[2-(1H-imidazol-4-yl)ethyl]oxalamide dihydrochloride dihydrate solution on the number of coughs in a capsaicin inhalation-induced acute cough model in guinea pigs.

### DETAILED DESCRIPTION OF THE INVENTION

The preparation of the free base N,N'-bis[2-(1H-imidazol-4-yl)ethyl]oxalamide, as well as a number of other chemical compounds, is described in patent application RU 2013/116822. The same application discloses the ability of N,N'-bis[2-(1H-imidazol-4-yl)ethyl]oxalamide to chelate metal ions, as well as its use in antiviral and anti-inflammatory therapy, and for the treatment of a number of diseases associated with the chelating properties of the claimed compounds.

Furthermore, the activity of N,N'-bis[2-(1H-imidazol-4-yl)ethyl]oxalamide against human secretory glutamyl cyclase and Golgi-resident glutamyl cyclase, as well as its related use in the treatment of various inflammatory diseases is described in patent RU 2665688 (published 04.09.2018).

However, during the study of the pharmaceutical properties of N,N'-bis[2-(1H-imidazol-4-yl)ethyl]oxalamide, the Applicant encountered a number of issues.

Firstly, N,N'-bis[2-(1H-imidazol-4-yl)ethyl]oxalamide has low aqueous solubility, which limits its effective use as a pharmaceutical substance on its own.

The development of a liquid pharmaceutical formulation based on N,N'-bis[2-(1H-imidazol-4-yl)ethyl]oxalamide was assumed to require the preparation and use of a solution of a salt (with an organic or inorganic acid) that would be stable in water, as the compound N,N'-bis[2-(1H-imidazol-4-yl)ethyl]oxalamide itself is insoluble in water, which significantly complicates the creation of liquid dosage forms. However, based on the experimental results, the inventors discovered that this is not a trivial task.

It was unexpectedly found that most aqueous salt solutions exhibit an increase in impurities during storage (see Table 34 below), which is likely to prevent the development of a stable liquid formulation based on them, whereas Compound 1 and certain other salts were found to be stable in solution, such as:
N,N'-bis[2-(1H-imidazol-4-yl)ethyl]oxalamide di-p-toluenesulfonate of Formula 12,
N,N'-bis[2-(1H-imidazol-4-yl)ethyl]oxalamide dimandelate,
N,N'-bis[2-(1H-imidazol-4-yl)ethyl]oxalamide dicamphorsulfonate, and
N,N'-bis[2-(1H-imidazol-4-yl)ethyl]oxalamide disulfate.

Secondly, *in vivo* studies revealed that the pharmacokinetics of N,N'-bis[2-(1H-imidazol-4-yl)ethyl]oxalamide is characterized by high intergroup variability of key pharmacokinetic parameters.

For example, in several independent experiments, it was shown that after a single oral administration of N,N'-bis[2-(1H-imidazol-4-yl)ethyl]oxalamide to Sprague-Dawley rats, the maximum and minimum values of peak concentration and exposure of N,N'-bis[2-(1H-imidazol-4-yl)ethyl]oxalamide varied by more than threefold within the same group of animals. The mean and median values of exposure and peak concentrations, determined in independent pharmacokinetic studies of N,N'-bis[2-(1H-imidazol-4-yl)ethyl]oxalamide following a single oral dose of 13 mg/kg, differed by more than fourfold (see Biological Example 1 below for details).

The therapeutic or prophylactic use of a medicament with high variability in pharmacokinetic parameters requires individualized dose selection, which precludes the use of N,N'-bis[2-(1H-imidazol-4-yl)ethyl]oxalamide for treating diseases associated with the development of critical conditions or rapidly progressing pathologies.

Thus, the pharmacokinetic characteristics of N,N'-bis[2-(1H-imidazol-4-yl)ethyl]oxalamide complicate its clinical use as a medicament for the treatment of rapidly progressing life-threatening diseases such as idiopathic pulmonary fibrosis, fibrosis of the liver, kidneys, heart (myocardial fibrosis), and pancreas.

In the course of further studies of the properties of N,N'-bis[2-(1H-imidazol-4-yl)ethyl]oxalamide, a number of salt forms of Formula (I) were obtained, comprising a pharmacologically acceptable counterion and exhibiting high aqueous solubility. Several salt forms of N,N'-bis[2-(1H-imidazol-4-yl)ethyl]oxalamide were identified that demonstrated crystallinity, compositional stability, and high purity.

Subsequent studies unexpectedly demonstrated that the salts of Formula (I) exhibit improved stability under storage conditions, both in solid form and as an aqueous solution.

The subsequent studies also unexpectedly revealed that the salts of Formula (I) exhibit preferential distribution to the lungs, as well as to the target-organs of hepatic, renal, and pancreatic fibrosis, and demonstrate lower variability in pharmacokinetic characteristics compared to the free base.

Moreover, it was unexpectedly found that, in comparison with the free base, the salts of Formula (I) are characterized by lower peak concentration and drug exposure levels in the blood plasma of experimental animals. Thus, the therapeutic use of the salts of Formula (I) is associated with a reduced risk of adverse side effects.

Subsequent efficacy studies unexpectedly revealed that the salts of Formula (I) exhibit activity in suppressing collagen synthesis and fibrotic processes in tissues both *in vivo* and *in vitro.*

Further studies also unexpectedly demonstrated that the salts of Formula (I) are effective in the treatment of cough.

Thus, the salts of Formula (I) developed in the course of the present invention exhibit high aqueous solubility, crystallinity, compositional stability, and high purity.

Further studies unexpectedly revealed that, in comparison with the free base, the salts of Formula (I) exhibit preferential distribution to the target organs of pulmonary, hepatic, pancreatic, and renal fibrosis, as well as low variability in pharmacokinetic characteristics. They also demonstrate high therapeutic efficacy against fibrosis in specific organs, which cannot be explained based on previously disclosed information about the structure and properties of N,N'-bis[2-(1H-imidazol-4-yl)ethyl]oxalamide. Furthermore, the previously disclosed properties of N,N'-bis[2-(1H-imidazol-4-yl)ethyl]oxalamide do not suggest the efficacy of the salts of Formula (I) in the treatment of cough, as identified in the present invention.

### Terms and definitions

As used in the present document, the term "N,N'-bis[2-(1H-imidazol-4-yl)ethyl]oxalamide" refers to the free base of N,N'-bis[2-(1H-imidazol-4-yl)ethyl]oxalamide, represented by the structural formula:

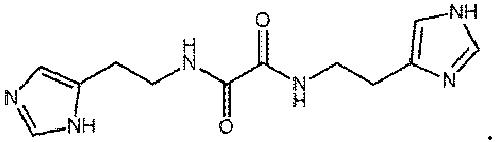

N,N'-bis[2-(1H-imidazol-4-yl)ethyl]oxalamide can exist in the form of mesomeric structures, which are presented in the Table below.

### Mesomeric Structures

| | |
|---|---|
| Mesomers of imidazole groups: | 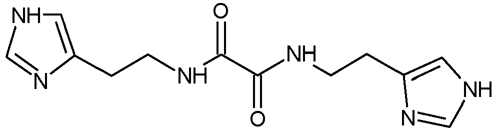 |
| | 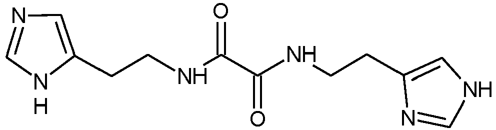 |
| | 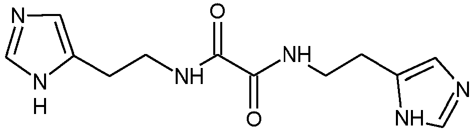 |
| Mesomers of amide groups: | 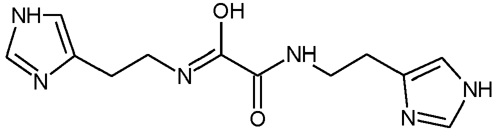 |
| | 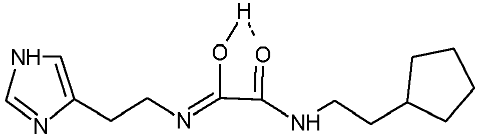 |
| | 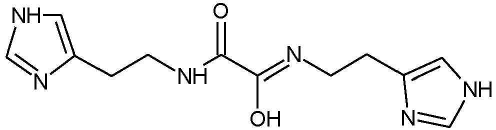 |
| | 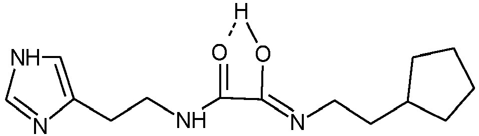 |
| | 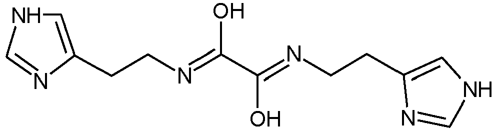 |

The term "mesomer" (or "mesomeric structure") is understood as a structure in chemical resonance theory that arises due to the conjugation of multiple bonds and/or lone electron pairs within a molecule. In N,N'-bis[2-(1H-imidazol-4-yl)ethyl]oxalamide, the mesomeric effect is possible in both imidazole and both amide groups.

The following names may also describe the structural formula above, taking into account the mesomeric effect possible for the heterocyclic fragments of the molecule:
Bis-1,2-(Nβ-histaminyl)oxalic acid;
N,N'-Bis-[2-(1H-imidazol-4-yl)ethyl]oxalamide;
N,N'-Bis-[2-(1H-imidazol-5-yl)ethyl]oxalamide;
N,N'-bis-[2-(1H-imidazol-4-yl)ethyl]ethanediamide; and
N,N'-bis-[2-(1H-imidazol-5-yl)ethyl]ethanediamide.

The term "C", when used in reference to temperature, denotes the centigrade scale or Celsius temperature scale.

The term "suspension" refers to a solid substance suspended in a liquid medium, typically water or an organic solvent.

The term "polymorphic modification" refers to the solid-phase form of a substance that appears in multiple distinct forms due to differences in the arrangement and/or conformation of molecules within the crystal lattice. Polymorphic modifications typically exhibit different chemical and physical properties. Furthermore, the term "polymorphic modification" also refers to solvates and hydrates (i.e., crystalline forms containing a solvent or water), as well as to different non-solvated and non-hydrated crystalline forms of a compound.

The term "solvate" is used to describe a molecular complex comprising a compound according to the invention and one or more molecules of a pharmaceutically acceptable solvent, such as ethanol. The term "hydrate" is used when the specified solvent is water.

The term "powder X-ray diffraction pattern" or "PXRD pattern" refers to an experimentally observed diffractogram or the parameters derived therefrom. Typically, powder X-ray diffraction patterns are characterized by peak position (on the x-axis) and peak intensity (on the y-axis). The term "peak intensity" refers to the relative intensity of a signal in a given X-ray diffraction pattern. Factors influencing the relative peak intensity include sample thickness and preferred orientation (i.e., the distribution of crystalline particles is not random). The term "peak position", as used in the present document, refers to the position of the X-ray reflection measured and observed in powder diffraction experiments. Peak positions are directly related to the unit cell dimensions. Peaks, identified by their corresponding positions, are obtained based on the diffraction pattern for various polymorphic forms of N,N'-bis[2-(1H-imidazol-4-yl)ethyl]oxalamide salts.

The term "2-theta value" or "2θ" refers to the peak position in degrees, based on experimental X-ray diffraction data, and primarily represents the unit of measurement on the x-axis of diffraction patterns. θ is the angle at which the diffraction condition is satisfied for a given wavelength. The angle between the diffracted beam direction and the primary beam is 2θ. It should be understood that any reference in the present application to specific 2θ values for a particular polymorphic form implies 2θ values in degrees, measured under the experimental X-ray diffraction conditions disclosed in the present application.

The term "auxiliary substance" means any pharmaceutically acceptable inorganic or organic substance that constitutes a medicament or used in the process of its manufacture to impart the necessary physicochemical properties to the medicament.

The terms "exposure" and "AUC" (area under the curve) refer to a pharmacokinetic parameter that characterizes the total concentration of a medicament in blood plasma over the entire observation period. Mathematically, it is defined as the integral from 0 to ∞ of the medicament concentration function (pharmacokinetic curve) in blood plasma over time and is equal to the area enclosed by the pharmacokinetic curve and the coordinate axes.

The terms "treatment" and "therapy" encompass the treatment of pathological conditions in mammals, preferably in humans, and include: (a) reduction, (b) blocking (halting) the progression of a disease, (c) alleviation of disease severity (i.e., inducing disease regression), and (d) reversal of the disease or condition to which the term applies, or one or more symptoms thereof.

The term "peak concentration" refers to a pharmacokinetic parameter that characterizes the maximum concentration of a medicament in blood plasma over the entire observation period.

The terms "prophylaxis" and "prevention" encompass the elimination of risk factors, as well as the preventive treatment of subclinical stages of a disease in mammals, preferably in humans, aimed at reducing the likelihood of progression of clinical stages of the disease. Patients for preventive therapy are selected based on factors that, according to known data, are associated with an increased risk of developing clinical stages of the disease compared to the general population. Preventive therapy includes (a) primary prevention and (b) secondary prevention. Primary prevention is defined as the preventive treatment of patients who have not yet developed the clinical stage of the disease, while secondary prevention refers to prevention of recurrence of the same or a related clinical condition.

### Salt form

Solid salt forms are generally preferred for oral medicaments, as they tend to exhibit desirable physical properties; however, different salt forms vary significantly in their ability to confer the desired properties (such as storage stability, ease of production and purification, pharmacokinetic parameters), and these properties cannot be predicted with sufficient accuracy [Berge et al., Pharmaceutical Salts// Journal Pharm. Sci., 1977, Vol.66, No.1; Verbeeck et al. Generic substitution: The use of medicinal products containing different salts and implications for safety and efficacy // EP Journal Pharm. Sci, 28, 2006, 1-6].

For example, some salts exist as solids at ambient temperature, while others are liquids, viscous oils, or resins. Furthermore, certain salt forms are stable under extreme heat and light exposure, whereas others readily degrade under much milder conditions. Thus, the development of an appropriate salt form of the base pharmaceutical substance for use in a pharmaceutical composition is an extremely important and often unpredictable process.

Pharmacokinetic parameters are among the most critical characteristics determining the suitability of a solid salt form (or a specific polymorphic modification) for use as a pharmaceutical substance. The average daily and maximum concentration of a medicament in the blood of animals and humans may significantly depend on the composition of the salt form and its polymorphic modification.

The feasibility of large-scale production of the selected salt form of a pharmaceutical substance and the purity (or complexity of purification) of the resulting product are also extremely important characteristics that significantly depend on the selected salt form. Furthermore, since pharmaceutical compositions comprising the pharmaceutical substance must have an adequate shelf life, suitable salt forms should not exhibit significant changes in their physicochemical characteristics (chemical composition, water content, density, hygroscopicity, solubility, etc.) over substantial storage period.

Solid substances, including pharmaceutically active compounds, often exist in more than one crystalline form, known as polymorphs. Polymorphism occurs when a compound crystallizes into multiple distinct solid phases that differ in crystal packing. Typically, polymorphic modifications (polymorphs) exhibit different physical characteristics, including solubility as well as physical and chemical stability. Moreover, different solid salt forms of the same pharmaceutical substance, and even different polymorphs of the same solid salt form, may vary in drug release rate, solid-state stability, and suitability for pharmaceutical formulation.

Thus, in the production of pharmaceutically acceptable compositions, it is important, whenever possible, to present the pharmaceutical substance in a crystalline and stable form(s).

### Pharmaceutical compositions

The invention also relates to pharmaceutical compositions that comprise the compounds according to the invention and one or more pharmaceutically acceptable carriers, adjuvants, solvents, and/or fillers, which can be administered to a patient together with the compound of the invention (constituting the essence of the present invention) and which do not affect the pharmacological activity of the compound and are non-toxic when administered in doses sufficient to deliver a therapeutically effective amount of the compound.

The pharmaceutical compositions claimed herein comprise the active compound, as described above, together with pharmaceutically acceptable carriers. These carriers may include any solvents, diluents, dispersions or suspensions, surfactants, isotonic agents, thickeners and emulsifiers, preservatives, binders, lubricants, etc., suitable for the specific dosage form. Materials that may serve as pharmaceutically acceptable carriers include, but are not limited to, mono- and oligosaccharides and their derivatives; gelatin; talc; excipients such as cocoa butter and waxes for suppositories; oils such as hydrogenated castor oil, peanut oil, cottonseed oil, safflower oil, sesame oil, olive oil, corn oil, and soybean oil; glycols such as propylene glycol; esters such as ethyl oleate and ethyl laurate; agar; buffering agents such as magnesium hydroxide and aluminum hydroxide; alginic acid; pyrogen-free water; isotonic solutions, Ringer's solution; ethanol; and phosphate-buffered solutions. Additionally, the composition may comprise other non-toxic, compatible lubricants, such as sodium lauryl sulphate and magnesium stearate, as well as colorants, partition liquids, film formers, sweeteners, taste-masking agents and flavoring additives, preservatives, and antioxidants.

The present invention also relates to dosage forms, a class of pharmaceutical compositions whose formulation is optimized for a specific route of administration in a therapeutically effective dose, for example, for oral, topical, inhalation (e.g., as an inhalation spray), intravenous, intranasal, subcutaneous, intramuscular, and infusion administration in the recommended dosages.

Dosage forms according to the present invention may include formulations prepared using liposomal technology, microencapsulation methods, methods of nanoparticle formulation, or other methods known in the pharmaceutical field.

In the preparation of a composition, for example, in the form of a tablet, the active compound is mixed with one or more pharmaceutical excipients, such as gelatin, starch, lactose, magnesium stearate, talc, silica, gum arabic, mannitol, microcrystalline cellulose, hypromellose, or similar compounds.

Tablets may be coated with sucrose, a cellulose derivative, or other substances suitable for coating. Tablets can be produced using various methods, such as direct compression, dry or wet granulation, or hot-melt processing.

A pharmaceutical composition in the form of a gelatin capsule can be prepared by mixing the active compound with a solvent and filling soft capsules with the resulting mixture.

In one embodiment, a pharmaceutical composition is particularly prepared in the form of a capsule mass for filling hard gelatin capsules by sequentially performing the following technological operations: triturating a mixture of the active substance with anhydrous lactose, mixing the triturate with anhydrous lactose and pregelatinized starch, and dusting the resulting mixture with hydrogenated castor oil.

In yet another embodiment, a pharmaceutical composition is prepared in the form of a capsule mass for filling hard gelatin capsules by sequentially performing the following technological operations: mixing the active substance with lactose monohydrate or anhydrous lactose and pregelatinized starch followed by homogenization, mixing the resulting mixture with magnesium stearate, followed by further homogenization.

For parenteral administration, aqueous suspensions, isotonic saline solutions, or sterile injection solutions are used, which comprise pharmaceutically compatible agents, such as propylene glycol or butylene glycol.

### Method for therapeutic use of the compounds

The present invention also relates to the administration of a therapeutically effective amount of the compound according to the invention to a subject in need of such treatment. A "therapeutically effective amount" refers to an amount of the compound that, when administered or delivered to a patient, is most likely to elicit the desired therapeutic (or prophylactic) response. The exact required amount may vary from subject to subject depending on factors such as age, body weight, overall patient condition, disease severity, method of administration, combination therapy with other drugs, and other factors.

A salt of N,N'-bis[2-(1H-imidazol-4-yl)ethyl]oxalamide or a pharmaceutical composition comprising the salt of N,N'-bis[2-(1H-imidazol-4-yl)ethyl]oxalamide can be administered in any amount. Preferably, the daily dose of the active compound is up to 1.5 g per patient per day, and most preferably the daily dose ranges from 50 to 1000 mg/day, administered by any route (preferably oral) that is effective for treating or preventing a disease.

After mixing the active compound with a specific suitable pharmaceutically acceptable carrier in the desired dosage, the compositions according to the invention may be administered to humans or animals orally, parenterally, topically, etc.

Administration may be performed as a single dose or multiple times per day, per week (or any other time interval), or intermittently. Additionally, the active compound may be administered to a patient daily for a specific period (e.g., 2-10 days) followed by a drug-free period (e.g., 1-30 days).

When the active compound is used as part of a combination therapy regimen, the dose of each component of the combination therapy is administered over the required treatment period. The compounds constituting the combination therapy may be administered to the patient either simultaneously in a single dosage form comprising all components or as individual dosages of each component.

### Combination Therapy

Although the salt form of N,N'-bis[2-(1H-imidazol-4-yl)ethyl]oxalamide according to the present invention can be administered as an individual active compound, it may also be used in combination with one or more other agents. In particular, another agent may be a nonsteroidal anti-inflammatory drug (NSAID) or another anti-inflammatory agent, pirfenidone, nintedanib, or another drug for fibrosis therapy. When co-administered orally, the therapeutic agents may be in different dosage forms, which are administered simultaneously or sequentially at different times, or the therapeutic agents may be combined into a single dosage form.

The term "combination therapy" in relation to the active compound according to the present invention in combination with other active compounds, refers to the simultaneous or sequential administration of all agents, which in some way provides a beneficial effect from the drug combination. Co-administration implies, in particular, the combined delivery, for example, in a single tablet, capsule, injection, or another form containing a fixed ratio of active compounds, as well as simultaneous delivery in several separate dosage forms, each comprising an individual compound.

Thus, the administration of the active compound according to the present invention may be carried out in combination with additional treatment methods known to specialists in the prevention and treatment of the relevant diseases, such as an anti-inflammatory agent, pirfenidone, nintedanib, or another drug for fibrosis therapy.

If the dosage form is a fixed dose, the combination uses the dose of the active compound within an acceptable range. The active compound according to the invention may also be administered sequentially with other agents when combination of these drugs is not feasible. The invention is not limited by the sequence of administration; the active compound according to the invention may be administered concurrently with, before, or after the administration of another pharmaceutical substance.

### EXAMPLES

The present invention is further illustrated but is not intended to be limited by the following examples.

### Experimental Conditions

The quantitative determination of N,N'-bis[2-(1H-imidazol-4-yl)ethyl]oxalamide was performed using HPLC.

### Chromatographic Conditions

| | |
|---|---|
| Column | 250 × 4.6 mm, packed with C18 sorbent, 5 µm (Gemini NX-C18 5 µm, cat. no. 00G-4454-E0, Phenomenex, USA) |
| | An alternative column that complies with the suitability requirements of the chromatographic system may be used; |
| Mobile phase | Acetonitrile: buffer solution at pH 7.5 (20:80); |
| Flow rate | 0.8 mL/min; |
| Column temperature | 25°C; |
| Autosampler temperature | 20°C; |
| Detector | UV at 210 nm; |
| Injection volume | 20 µL; |
| Elution mode | isocratic |

### Preparation of Solutions

*0.035M Phosphate Buffer:* 4.76 g of potassium dihydrogen phosphate is dissolved in 900 mL of chromatography-grade water. The pH value of the resulting solution is adjusted to 7.5 ± 0.05 potentiometrically using a 20% potassium hydroxide solution. The volume of the solution is brought to 1000.0 mL using chromatography-grade water, then mixed and degassed by any convenient method.

*Mobile Phase:* A 1000.0 mL volumetric flask is filled with 20 mL of chromatography-grade acetonitrile, and the volume is adjusted to the mark using 0.035M phosphate buffer. The solution is degassed by any convenient method.

*Sample Solvent:* A 1000.0 mL volumetric flask is filled with 20 mL of chromatography-grade acetonitrile, and the volume is adjusted to the mark with chromatography-grade water.

*Standard Solution:* About 18.0 mg (accurately weighed) of N,N'-bis[2-(1H-imidazol-4-yl)ethyl]oxalamide is placed in a 50.0 mL volumetric flask, about 30 mL of solvent is added, and the mixture is stirred. The volume of the solution is then adjusted to the mark using the same solvent and stirred.

*Test Solution:* About 25.0 mg (accurately weighed) of an N,N'-bis[2-(1H-imidazol-4-yl)ethyl]oxalamide salt sample is placed in a 50.0 mL volumetric flask, about 30 mL of solvent is added, and the mixture is stirred. The volume of the solution is then adjusted to the mark using the same solvent and stirred.

### Calculation of the Quantitative Content of N,N'-bis[2-(1H-imidazol-4-yl)ethyl]oxalamide

The calculation of the N,N'-bis[2-(1H-imidazol-4-yl)ethyl]oxalamide base content (*xᵢ*) in percentage (%) is performed using the following formula: ${x}_{i} \left(%\right) = \frac{{S}_{i} \times {a}_{0} \times 100 \times P \times 100}{{S}_{0} \times {a}_{i} \times 100 \times \left(100-ω\right)} = \frac{{S}_{i} \times {a}_{0} \times P \times 100}{{S}_{0} \times {a}_{i} \times \left(100-ω\right)} ,$ wherein:
*Sᵢ* is the peak area of N,N'-bis[2-(1H-imidazol-4-yl)ethyl]oxalamide in the chromatogram of a test solution;
*S₀* is the peak area of N,N'-bis[2-(1H-imidazol-4-yl)ethyl]oxalamide in the chromatogram of the standard solution;
*a₀* is the weight of an N,N'-bis[2-(1H-imidazol-4-yl)ethyl]oxalamide substance sample, mg;
*aᵢ* is the weight of the test sample, mg;
*ω* is the water content in a test sample, %;
*P* is the content of N,N'-bis[2-(1H-imidazol-4-yl)ethyl]oxalamide in the substance sample, %.

The quantitative determination of the acid residue was performed by acid-base titration.

About 100.0 mg (accurately weighed) of an N,N'-bis[2-(1H-imidazol-4-yl)ethyl]oxalamide salt sample is placed in a 150.0 mL beaker, dissolved in 100 mL of chromatography-grade water, and titrated with a 0.1M sodium hydroxide solution.

### Calculation of the Quantitative Content of the Acid Residue

The calculation of the content of the acid residue in the N,N'-bis[2-(1H-imidazol-4-yl)ethyl]oxalamide salt, expressed as a percentage (%) in mg, is performed using the following formula: ${x}_{acid} = \frac{\left({V}_{i}-{V}_{k}\right) ⋅ {M}_{eq} ⋅ k ⋅ 100}{{m}_{i} ⋅ χ ⋅ \left(100-ω\right)} ,$ wherein:
*mᵢ* is the mass of a weighed test sample, g;
*M_{eq}* is the amount of acid ions equivalent to 1 mL of a 0.1M sodium hydroxide solution, g/mL;
*Vₖ* is the volume of titrant used for the titration of the control test, mL;
*Vᵢ* is the volume of titrant used for the titration of the test sample, mL;
*X* is the stoichiometric coefficient, %; and
*ω* is the water content, %.

The chemical structure of the obtained compounds was confirmed by X-ray phase and X-ray structural analysis.

X-ray phase analysis was performed using a MiniFlex 600 diffractometer (Rigaku, Japan), operating in Bragg-Brentano (θ/2θ) geometry with a 1D d/TeX Ultra silicon strip detector and a Kβ filter. Data were collected in the range of 2° to 50° 2θ with a step size of 0.02° and a scan speed of 1° min⁻¹, using a CuKα radiation source. All experiments were conducted at ambient temperature (293(2) K). Additionally, X-ray phase analysis was performed using a Bruker D8 Advance diffractometer (using CuKα radiation) equipped with a LynxEye position-sensitive detector, in reflection geometry with sample rotation. Data collection was performed using the Bruker DIFFRACplus software suite, and analysis was performed using the EVA and Topas V5.0 software.

Single-crystal X-ray diffraction (SCXRD) experiments were performed using an XtaLAB Synergy-I diffractometer equipped with a HyPix3000 hybrid pixel array detector and a PhotonJet-I (Cu Kα) microfocus X-ray source. The crystal structure model was solved and refined using the SHELXT and SHELXL software. All experiments were conducted at ambient temperature (293(2) K).

Single-crystal X-ray diffraction studies of the compounds were carried out using a Bruker APEX II diffractometer (Mo Kα radiation, graphite monochromator, ω-scan). The structures were solved using the dual-space algorithm and refined by least-squares (LS) minimization in a full-matrix anisotropic approach on F²hkl. Hydrogen atoms were placed geometrically and refined with constraints applied to bond lengths and valence angles. All calculations were performed using SHELXT and SHELXL software.

### Synthesis of N,N'-bis[2-(1H-imidazol-4-yl)ethyl]oxalamide and salts thereof

### Example 1. Synthesis of N,N'-bis[2-(1H-imidazol-4-yl)ethyl]oxalamide

The synthesis of N,N'-bis[2-(1H-imidazol-4-yl)ethyl]oxalamide is described in patent application RU 2013/116822 (published 20.10.2014). The same application describes the ability of N,N'-bis[2-(1H-imidazol-4-yl)ethyl]oxalamide to form complexes or chelate metal ions. The biological activity of N,N'-bis[2-(1H-imidazol-4-yl)ethyl]oxalamide with respect to human secretory glutamyl cyclase and Golgi-resident glutamyl cyclase is described in patent RU 2665688 (published 04.09.2018).

The obtained product has the following characteristics:
¹H NMR spectrum (400 MHz, DMSO-d₆) δ: 11.82 (s, 1H, NH_{imidazole},), 8.09 (t, *J*=5.6 Hz, 1H, CONH), 7.51 (s, 1H, H²), 6.79 (s, 1H, H⁵), 3.28 (q, *J*=7.2 Hz, 2H, H^{α}), 3.01 (s, 2H, COCH₂CO), 2.63 (t, *J*=7.2 Hz, 2H, H^{β}).
¹³C NMR spectrum (400 MHz, D₂O) δ: 166.9 (CO), 134.7 (C⁵), 134.3 (C⁵), 116.9 (C²), 43.5 (CH₂), 39.0 (C^{α}) , 26.8 (C^{β}).
Mass spectrum, m/z: 277.14071 ([M+nH]⁺).

### Example 2. Synthesis of N,N'-bis[2-(1H-imidazol-4-yl)ethyl]oxalamide Dihydrochloride Dihydrate

To 4.2 mL (50 mmol) of concentrated hydrochloric acid, 7.2 mL of water was added. The resulting solution was heated to 50 °C and 5.9 g (21 mmol) of N,N'-bis[2-(1H-imidazol-4-yl)ethyl]oxalamide was introduced. The resulting suspension was stirred at the same temperature until a homogeneous solution was formed, after which the solution was filtered, and 9.0 mL of acetone was added to the filtrate. The mixture was cooled to 15 °C and stirred for 30 minutes. The product was isolated by filtration, washed with two portions of acetone, and dried at 60 °C for 8 hours to give 7.2 g of N,N'-bis[2-(1H-imidazol-4-yl)ethyl]oxalamide dihydrochloride dihydrate as a white crystalline powder (yield 87%).

¹H NMR spectrum (400 MHz, DMSO-d₆) δ: 2.85 (t, J=6.8Hz, 4H, β-CH₂-HA), 3.43 (q, J=6.3Hz, 4H, α-CH₂-HA), 7.40 (d, J=1.0Hz, 2H, -C=CH-), 8.89 (dd, J₁=6.2Hz J₂=5.7Hz, 2H, -NH-CO-), 9.05 (d, J=1.0Hz, 2H, -NH-CH=N-).

¹³C NMR spectrum (400 MHz, DMSO-d₆) δ: 23.9 (C^{β}), 37.6 (C^{α}), 115.8 (C²), 130.6 (C⁵), 133.4 (-CH₂-), 159.9 (-CO-).

Mass spectrum, m/z: 277.14071 ([M+nH]⁺).

Retention time of the individual peak for C₁₂H₁₆N₆O₂: 3.9 min.

Quantitative determination of the base C₁₂H₁₆N₆O₂: 71.7% calculated, 72.7% determined by HPLC (internal normalization method), recalculated on an anhydrous substance.

Quantitative determination of the acidic residue in the salt: 18.9% calculated, 19.0% determined by acid-base titration, recalculated on an anhydrous substance.

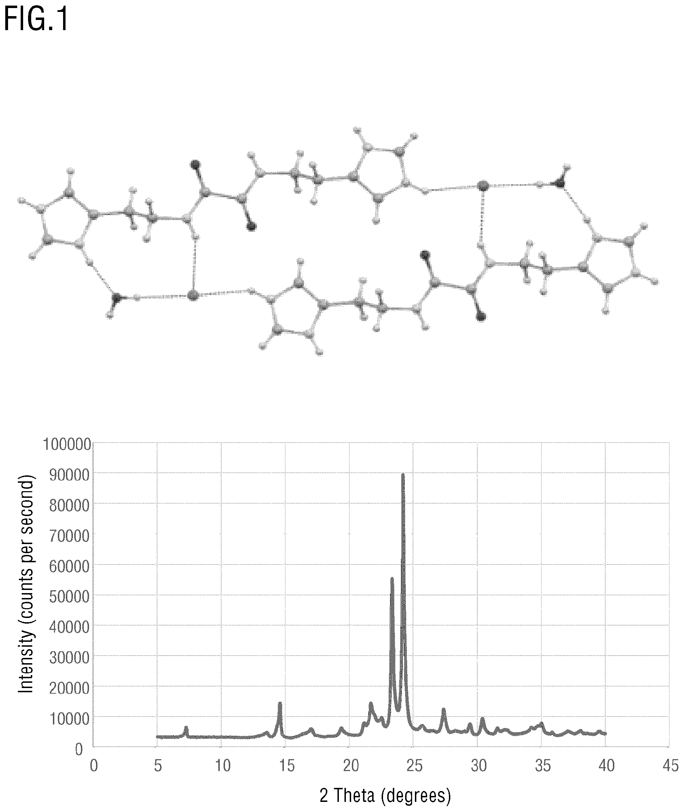

Next, an X-ray diffraction pattern of the obtained crystalline powder was recorded. Table 1 lists the 2θ positions and their relative intensities. Figure 1 shows the powder X-ray diffraction pattern of the obtained crystalline form. Single-crystal analysis revealed that the substance crystallizes in a monoclinic unit cell with the following parameters: space group P21/c, Å: a = 5.7218(2) Å, b = 24.1996(6) Å, c = 6.9536(2) Å, β = 104.043 (3) °, and V = 934.06(5) Å³. Based on the unit cell volume and symmetry, the crystal composition was determined as C₁₂H₁₆N₆O₂·2HCl·2H₂O.

**Table 1. Intensity and 2θ positions of some peaks with relative intensities greater than 5% for the sample.**

| Position [°2θ] | Relative Intensity [%] |
|---|---|
| 7.2862 | 3.13 |
| 13.4999 | 4.14 |
| 14.5701 | 13.64 |
| 16.5675 | 6.67 |
| 17.0415 | 2.78 |
| 19.3376 | 6.61 |
| 21.1355 | 10.54 |
| 21.6650 | 21.35 |
| 22.5622 | 5.28 |
| 23.3096 | 58.80 |
| 24.1904 | 100.00 |
| 25.6055 | 4.71 |
| 27.3308 | 11.45 |
| 28.2508 | 1.10 |
| 28.9654 | 5.76 |
| 29.3081 | 0.34 |
| 29.4467 | 3.59 |
| 30.4151 | 7.06 |
| 31.5645 | 1.83 |
| 32.2215 | 4.04 |
| 34.1911 | 2.01 |
| 34.7110 | 9.42 |
| 34.9739 | 3.39 |
| 35.8779 | 1.64 |
| 37.0783 | 2.78 |
| 38.0239 | 1.88 |
| 39.4938 | 0.82 |

In the X-ray diffraction pattern of C₁₂H₁₆N₆O₂·2HCl·2H₂O, the following intense peaks (maxima) were observed at diffraction angles 2θ (± 0.2°): 14.6, 21.1, 21.7, 23.3, 24.2, 27.3, 29.0, and 34.7. The most intense maxima were displayed at 21.7, 23.3, and 24.2.

### Example 3. Synthesis of N,N'-bis[2-(1H-imidazol-4-yl)ethyl]oxalamide Diphosphate (Molar Ratio 1:2)

To a solution of 4.20 g (0.04 mol) of phosphoric acid in 17.3 mL of water, 5.00 g (0.02 mol) of N,N'-bis[2-(1H-imidazol-4-yl)ethyl]oxalamide was added with stirring. The resulting suspension was stirred at 90 °C until a solution was obtained. The reaction mixture was cooled to 20-25 °C, 33 mL of acetone was added, and the mixture was stirred for 2 hours. The formed precipitate was filtered, washed with acetone (2 × 10.0 mL), and dried under vacuum at 60 °C to give a white crystalline powder.

Yield: 7.39 g (86.4%).

Retention time of the individual peak for C₁₂H₁₆N₆O₂: 4.1 min.

Quantitative determination of the base C₁₂H₁₆N₆O₂ : 58.5% calculated, 58.4% determined by HPLC (internal normalization method), recalculated on an anhydrous substance.

Quantitative determination of the acidic residue in the salt: 41.5% calculated, 41.5% determined by acid-base titration, recalculated on an anhydrous substance.

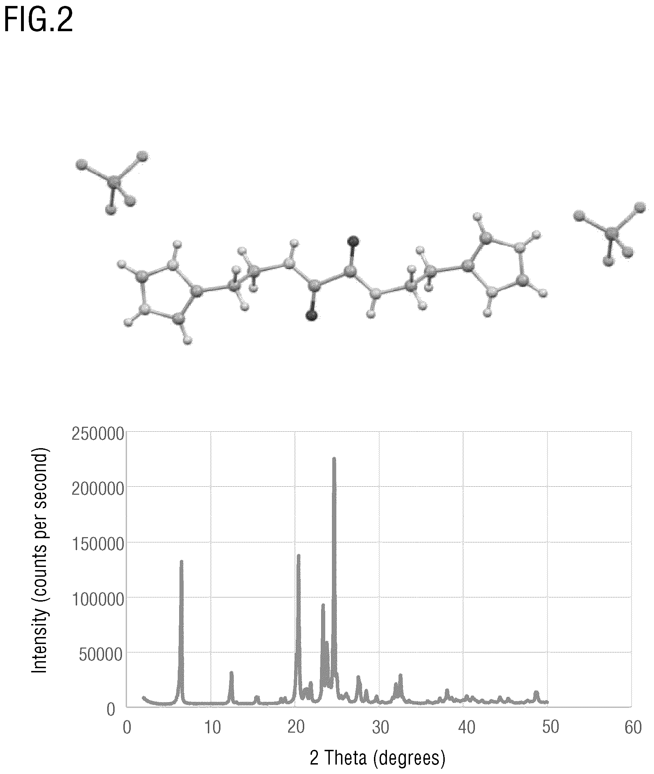

Next, an X-ray diffraction pattern of the obtained crystalline powder was recorded. Table 2 lists the 2θ positions and their relative intensities. Figure 2 shows the powder X-ray diffraction pattern of the obtained crystalline form. Single-crystal analysis showed that the substance crystallizes in a triclinic unit cell with the following parameters: space group P-1, Å: a = 4.89980(10) Å, b = 7.4302(3) Å, c = 14.0207(6) Å, α = 103.836(4)°, β = 94.022(3)°, γ = 97.898(3)°, and V = 488.11(3) Å³. Based on the unit cell volume and symmetry, the crystal composition was determined as C₁₂H₁₆N₆O₂·2H₃PO₄.

**Table 2. Intensity and 2θ positions of some peaks with relative intensities greater than 5% for the sample.**

| Position [°2θ] | Relative Intensity [%] |
|---|---|
| 6.4699 | 49.87 |
| 6.5401 | 46.32 |
| 12.4803 | 24.68 |
| 20.1678 | 35.31 |
| 20.4353 | 91.57 |
| 21.4014 | 8.83 |
| 21.8668 | 14.56 |
| 23.3413 | 36.12 |
| 23.3678 | 65.85 |
| 23.7566 | 28.39 |
| 24.6587 | 100 |
| 24.952 | 34.2 |
| 27.6561 | 20.72 |
| 28.4602 | 10.58 |
| 31.9726 | 5.44 |
| 32.5218 | 11.41 |
| 48.6252 | 8.41 |

In the X-ray diffraction pattern of C₁₂H₁₆N₆O₂·H₃PO₄, the following intense peaks (maxima) were observed at diffraction angles 2θ (± 0.2°): 6.5, 12.5, 20.2, 20.4, 23.3, 23.4, 23.8, and 24.7. The most intense maxima were displayed at 20.4, 23.3, and 24.7.

### Example 4. Synthesis of N,N'-bis[2-(1H-imidazol-4-yl)ethyl]oxalamide dimaleinate dihydrate (Molar Ratio 1:2)

To a solution of 3.36 g (0.030 mol) of maleic acid in 36.3 mL of water, 4.00 g (0.015 mol) of N,N'-bis[2-(1H-imidazol-4-yl)ethyl]oxalamide was added with stirring. The resulting suspension was stirred at 80 °C until a solution was obtained. The reaction mixture was cooled to 5 °C, 33 mL of acetone was added, and the mixture was stirred for 30 minutes. The formed precipitate was filtered, washed with acetone (2 × 10.0 mL), and dried under vacuum at 60 °C to give a white crystalline powder.

Yield: 8.03 g (81.5%).

Retention time of the individual peak for C₁₂H₁₆N₆O₂: 4.0 min.

Quantitative determination of the base C₁₂H₁₆N₆O₂: 50.7% calculated, 50.5% determined by HPLC (internal normalization method), recalculated on an anhydrous substance.

Quantitative determination of the acidic residue in the salt: 42.6% calculated, 42.2% determined by acid-base titration, recalculated on an anhydrous substance.

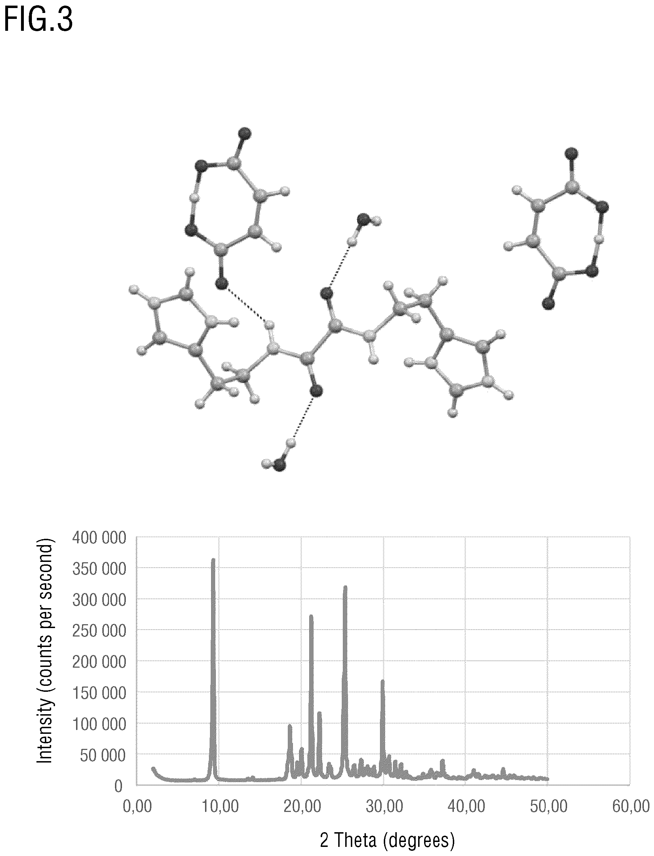

Next, an X-ray diffraction pattern of the obtained crystalline powder was recorded. Table 3 lists the 2θ positions and their relative intensities. Figure 3 shows the powder X-ray diffraction pattern of the obtained crystalline form. Single-crystal analysis revealed that the substance crystallizes in a triclinic unit cell with the following parameters: space group P-1 Å: a = 4.96553(10) Å, b = 10.3177(3) Å, c = 13.5280(3) Å, α = 109.724(2)°, β = 96.6220(18)°, γ = 98.803(2)°, and V = 634.26(43) Å³.

Based on the unit cell volume and symmetry, the crystal composition was determined as C₁₂H₁₆N₆O₂·2C₄H₄O₄·2H₂O.

**Table 3. Intensity and 2θ positions of some peaks with relative intensities greater than 5% for the sample.**

| Position [°2θ] | Relative Intensity [%] |
|---|---|
| 9.2801 | 100 |
| 9.401 | 12.31 |
| 18.3522 | 16.89 |
| 18.6179 | 17.6 |
| 18.7748 | 7.41 |
| 19.5142 | 6.32 |
| 20.0524 | 15.55 |
| 21.2221 | 72.79 |
| 22.22 | 30.04 |
| 23.3893 | 5.92 |
| 23.6089 | 7.07 |
| 25.1428 | 41.08 |
| 25.3628 | 80.9 |
| 26.4727 | 3.97 |
| 27.3094 | 8.58 |
| 28.0963 | 4.07 |
| 28.8673 | 3.6 |
| 29.9013 | 44.02 |
| 30.6838 | 10.72 |
| 31.4385 | 7.97 |
| 32.1577 | 6.94 |
| 37.2518 | 10.18 |

In the X-ray diffraction pattern of C₁₂H₁₆N₆O₂·2C₄H₄O₄·2H₂O, the following intense peaks (maxima) were observed at diffraction angles 2θ (± 0.2°): 9.3, 18.4, 18.6, 21.2, 22.2, 25.1, 25.4, and 29.9. The most intense maxima were displayed at 9.3, 25.4, and 29.9.

### Example 5. Synthesis of N,N'-bis[2-(1H-imidazol-4-yl)ethyl]oxalamide dimalonate (molar ratio 1:2)

To a solution of 3.77 g (0.04 mol) of malonic acid in 6.3 mL of water, 5.00 g (0.02 mol) of N,N'-bis[2-(1H-imidazol-4-yl)ethyl]oxalamide was added with stirring. The resulting suspension was stirred at 50 °C until a solution was obtained. The reaction mixture was cooled to 20-25 °C, 33 mL of acetone was added, and the mixture was stirred for 2 hours. The formed precipitate was filtered, washed with acetone (2 × 15.0 mL), and dried in vacuum at 60 °C to give a white crystalline powder.

Yield: 7.4 g (84.4%).

Retention time of the individual peak for C₁₂H₁₆N₆O₂: 3.9 min.

Quantitative determination of the base C₁₂H₁₆N₆O₂: 57.0% calculated, 58.2% determined by HPLC (internal normalization method), recalculated on an anhydrous substance.

Quantitative determination of the acidic residue in the salt: 43.8% calculated, 43.8% determined by acid-base titration, recalculated on an anhydrous substance.

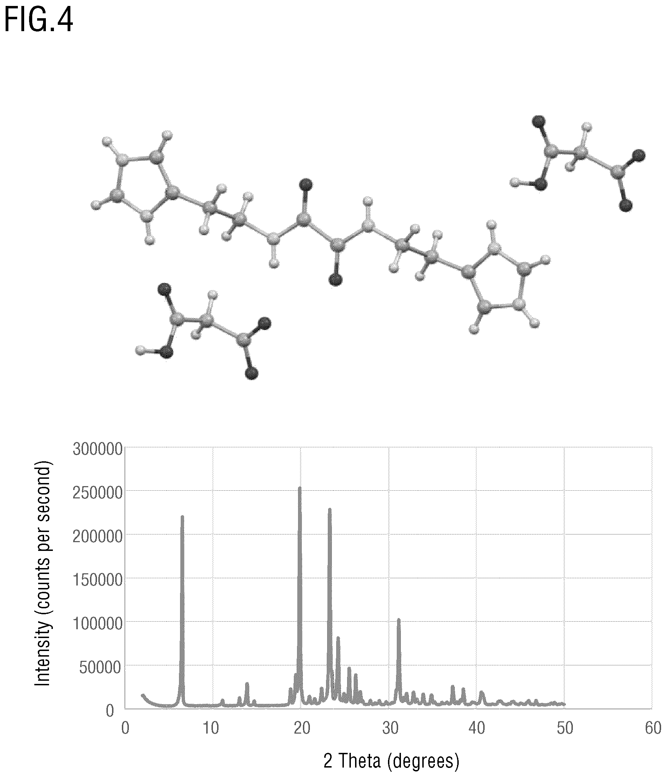

Next, an X-ray diffraction pattern of the obtained crystalline powder was recorded. Table 4 lists the 2θ positions and their relative intensities. Figure 4 shows the powder X-ray diffraction pattern of the obtained crystalline form. Single-crystal analysis revealed that the substance crystallizes in a triclinic unit cell with the following parameters: space group P-1, Å: a = 4.59430(10) Å, b = 8.5343(2) Å, c = 14.0094(3) Å, α = 103.911(2)°, β = 92.1761(19)°, γ = 96.818(2)°, and a volume of V = 528.15(4) Å³.

Based on the unit cell volume and symmetry, the crystal composition was determined as C₁₂H₁₆N₆O₂·2C₃H₄O₄.

**Table 4. Intensity and 2θ positions of some peaks with relative intensities greater than 5% for the sample.**

| Position [°2θ] | Relative Intensity [%] |
|---|---|
| 6.5216 | 100 |
| 13.8947 | 15.35 |
| 18.8862 | 8.73 |
| 19.4658 | 10.88 |
| 19.9119 | 95.4 |
| 19.9681 | 41.29 |
| 22.394 | 8.47 |
| 23.3193 | 84.08 |
| 23.5091 | 36.42 |
| 24.2556 | 39.9 |
| 25.5007 | 17.99 |
| 26.2438 | 13.35 |
| 26.7657 | 6.39 |
| 30.8627 | 9.48 |
| 31.1436 | 45.79 |
| 32.0292 | 5.36 |
| 32.8351 | 7.83 |
| 33.9926 | 6.41 |
| 37.3295 | 12.69 |
| 38.5363 | 9.89 |
| 6.5216 | 100 |
| 13.8947 | 15.35 |

In the X-ray diffraction pattern of C₁₂H₁₆N₆O₂·2C₃H₄O₄·2H₂O, the following intense peaks (maxima) were observed at diffraction angles 2θ (± 0.2°): 6.5, 13.9, 19.7, 23.3, 23.5, 25.5, 26.2, 31.1, and 37.3. The most intense maxima were displayed at 6.5, 23.3, and 31.1.

### Example 6. Synthesis of N,N'-bis[2-(1H-imidazol-4-yl)ethyl]oxalamide fumarate (molar ratio 1:1)

To a solution of 3.36 g (0.03 mol) of fumaric acid in 77.3 mL of water, 8.00 g (0.03 mol) of N,N'-bis[2-(1H-imidazol-4-yl)ethyl]oxalamide was added with stirring. The resulting suspension was stirred at 50 °C until a solution was obtained. The reaction mixture was cooled to 20 °C, 53 mL of acetone was added, and the mixture was stirred for 2 hours. The formed precipitate was filtered, washed with acetone (2 × 20.0 mL), and dried in vacuum at 60 °C to give a white crystalline powder.

Yield: 10.96 g (96.5%).

Retention time of the individual peak for C₁₂H₁₆N₆O₂: 3.9 min.

Quantitative determination of the base C₁₂H₁₆N₆O₂: 70.4% calculated, 70.0% determined by HPLC (internal normalization method), recalculated on an anhydrous substance.

Quantitative determination of the acidic residue in the salt: 29.6% calculated, 29.4% determined by acid-base titration, recalculated on an anhydrous substance.

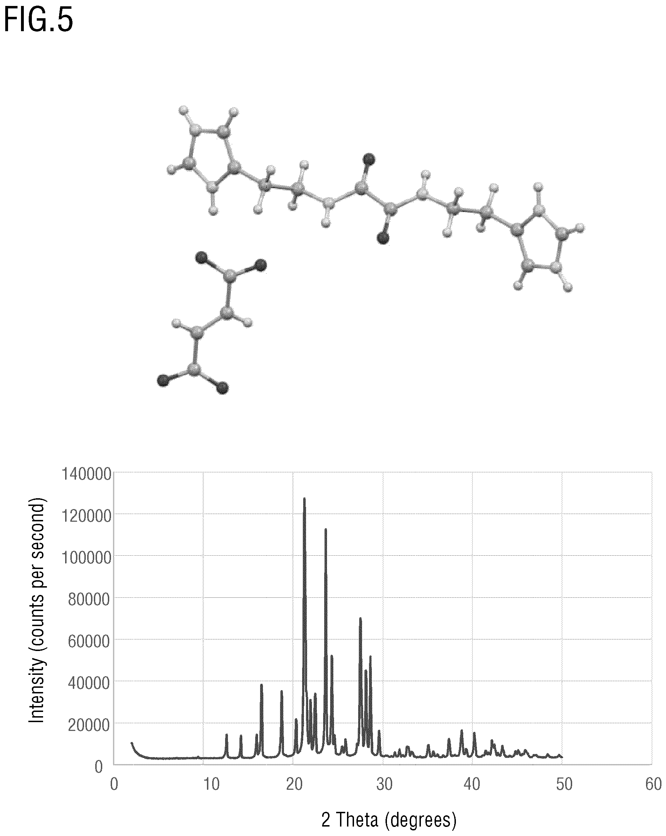

Next, an X-ray diffraction pattern of the obtained crystalline powder was recorded. Table 5 lists the 2θ positions and their relative intensities. Figure 5 shows the powder X-ray diffraction pattern of the obtained crystalline form. Single-crystal analysis revealed that the substance crystallizes in a monoclinic syngony, with the following parameters: space group P21/c, Å: a = 12.8718(8) Å, b = 5.0966(2) Å, c = 14.4484(9) Å, β = 104.362(6)°, and a volume of V = 918.3(4) Å³.

Based on the cell volume and symmetry, the crystal composition was determined as C₁₂H₁₆N₆O₂·C₄H₄O₄.

**Table 5. Intensity and 2θ positions of some peaks with relative intensities greater than 5% for the sample.**

| Position [°2θ] | Relative Intensity [%] |
|---|---|
| 9.4328 | 0.38 |
| 12.6094 | 9.71 |
| 14.2032 | 6.89 |
| 16.4862 | 27.92 |
| 18.755 | 23.71 |
| 18.9089 | 3.67 |
| 20.3607 | 13.46 |
| 21.2577 | 100 |
| 21.3684 | 35.27 |
| 21.5152 | 8.59 |
| 21.9646 | 17.25 |
| 22.462 | 29.26 |
| 23.6556 | 87.62 |
| 24.3245 | 41.14 |
| 25.4724 | 7.38 |
| 25.8143 | 8.17 |
| 27.1437 | 3.26 |
| 27.4876 | 53.73 |
| 28.0908 | 36.47 |
| 28.5378 | 8.46 |
| 28.6209 | 24.15 |
| 29.568 | 10.17 |
| 30.2432 | 0.85 |
| 30.495 | 0.66 |
| 31.3312 | 1.95 |
| 31.8614 | 2.95 |
| 32.2347 | 0.85 |
| 32.703 | 3.81 |
| 33.2222 | 2.16 |
| 33.3623 | 1.06 |
| 34.231 | 0.47 |
| 35.0383 | 3.64 |
| 35.1469 | 2.54 |
| 36.1497 | 1.49 |
| 37.3912 | 8.56 |
| 38.0404 | 0.69 |
| 38.7821 | 14.13 |
| 39.3356 | 3.97 |
| 40.2153 | 10.97 |
| 41.7991 | 1.29 |
| 42.1797 | 5.35 |
| 42.4608 | 6.2 |
| 42.8545 | 1.25 |
| 43.3328 | 3.85 |
| 43.5156 | 1.56 |
| 45.5941 | 1.11 |
| 47.1592 | 1.04 |
| 48.4055 | 1.59 |

In the X-ray diffraction pattern of C₁₂H₁₆N₆O₂·C₄H₄O₄, the following intense peaks (maxima) were observed at diffraction angles 2θ (± 0.2°): 16.5, 18.8, 18.9, 21.3, 21.4, 22.0, 22.5, 23.6, 24.3, 27.5, 28.1, and 28.6. The most intense maxima were displayed at 21.3, 23.6, and 27.5.

### Example 7. Synthesis of N,N'-bis[2-(1H-imidazol-4-yl)ethyl]oxalamide dihydroiodide (molar ratio 1:2)

To a solution of 8.93 g (0.04 mol) of a 57% hydroiodic acid solution in 6.3 mL of water, 5.00 g (0.02 mol) of N,N'-bis[2-(1H-imidazol-4-yl)ethyl]oxalamide was added with stirring. The resulting suspension was stirred at 50 °C until a solution was obtained. The reaction mixture was cooled to 20-25 °C, 100 mL of acetone was added, and the mixture was stirred for 2 hours. The formed precipitate was filtered, washed with acetone (2 × 15.0 mL), and dried in vacuum at 60 °C to give a white crystalline powder.

Yield: 6.0 g (88.7%).

Retention time of the individual peak for C₁₂H₁₆N₆O₂: 3.9 min.

Quantitative determination of the base C₁₂H₁₆N₆O₂: 51.9% calculated, 52.0% determined by HPLC (internal normalization method), recalculated on an anhydrous substance.

Quantitative determination of the acidic residue in the salt: 48.1% calculated, 48.1% determined by acid-base titration, recalculated on an anhydrous substance.

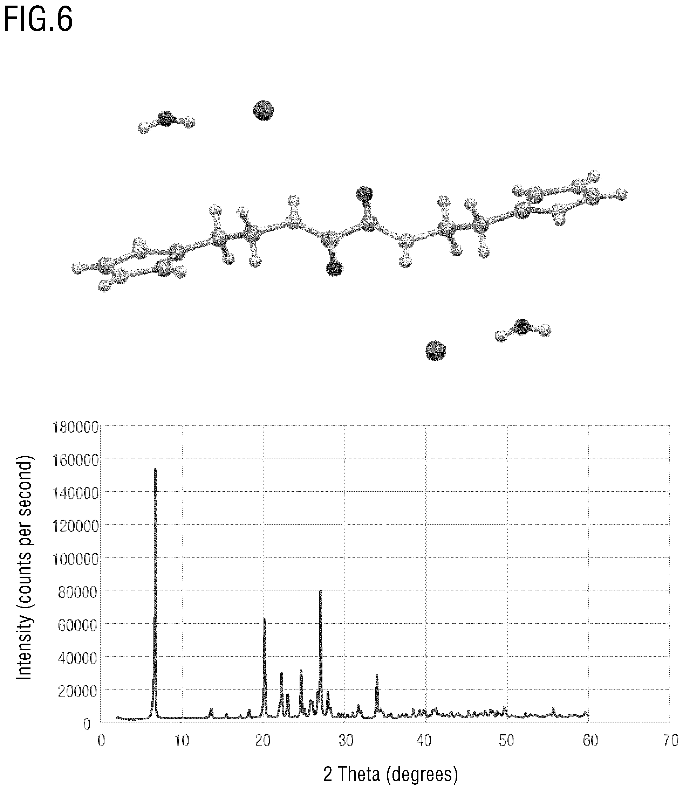

Next, an X-ray diffraction pattern of the obtained crystalline powder was recorded. Table 6 lists the 2θ positions and their relative intensities. Figure 6 shows the powder X-ray diffraction pattern of the obtained crystalline form.

**Table 6. Intensity and 2θ positions of some peaks with relative intensities greater than 5% for the sample.**

| Position [°2θ] | Relative Intensity [%] |
|---|---|
| 6.7009 | 100 |
| 12.9393 | 0.99 |
| 13.5193 | 3.85 |
| 15.4908 | 3.09 |
| 17.1338 | 2.91 |
| 18.2302 | 7.47 |
| 20.1739 | 52.26 |
| 20.8474 | 3.45 |
| 21.9487 | 4.62 |
| 22.2324 | 25.75 |
| 22.9623 | 14.26 |
| 25.0738 | 5.06 |
| 25.7418 | 10.23 |
| 25.8627 | 3.93 |
| 26.6734 | 13.55 |
| 27.0133 | 58.07 |
| 27.9221 | 17.66 |
| 28.3076 | 4.17 |
| 30.9705 | 2.86 |
| 31.7013 | 8.84 |
| 31.9967 | 5.25 |
| 33.9497 | 20.52 |
| 34.2502 | 6.11 |
| 34.6716 | 3.38 |
| 34.985 | 0.41 |
| 35.6916 | 2.53 |
| 36.5976 | 1.07 |
| 37.635 | 1.56 |
| 38.4389 | 4.52 |
| 38.9853 | 0.94 |
| 39.2426 | 2.59 |
| 39.7029 | 3.48 |
| 39.9685 | 2.09 |
| 40.8678 | 6.31 |
| 41.8718 | 0.55 |
| 43.0847 | 2.79 |
| 44.2865 | 0.98 |
| 45.2337 | 3.58 |
| 46.5042 | 0.54 |
| 46.7052 | 1.61 |
| 47.3162 | 2.22 |
| 49.2014 | 0.91 |
| 50.5099 | 1.34 |
| 52.8986 | 4.19 |
| 53.3889 | 0.94 |
| 55.1228 | 3.14 |
| 55.3223 | 0.43 |
| 55.9361 | 1.89 |
| 56.4565 | 1.31 |
| 59.8977 | 0.28 |

In the X-ray diffraction pattern of C₁₂H₁₆N₆O₂·2HI, the following intense peaks (maxima) were observed at diffraction angles 2θ (± 0.2°): 6.7, 20.2, 22.2, 22.2, 23.0, 25.7, 26.7, 27.0, 27.9, and 33.9. The most intense maxima were displayed at 6.7, 20.2, and 27.0.

### Example 8. Synthesis of N,N'-bis[2-(1H-imidazol-4-yl)ethyl]oxalamide dihydroiodide dihydrate (molar ratio 1:2)

To a solution of 8.93 g (0.04 mol) of a 57% hydroiodic acid solution in 6.3 mL of water, 5.00 g (0.02 mol) of N,N'-bis[2-(1H-imidazol-4-yl)ethyl]oxalamide was added in 20 mL of water with stirring. The resulting suspension was stirred at 70 °C until a solution was obtained. The reaction mixture was cooled to +25 °C and left until a precipitate formed. The formed precipitate was filtered, washed with water, and dried in vacuum to give a white crystalline powder.

Yield: 6.0 g (88.7%).

Retention time of the individual peak for C₁₂H₁₆N₆O₂: 3.9 min.

Quantitative determination of C₁₂H₁₆N₆O₂: 48.6% calculated, 58.7% determined by HPLC (internal normalization method), recalculated on an anhydrous substance.

Quantitative determination of the acidic residue in the salt: 45.0% calculated, 44.6% determined by acid-base titration, recalculated on an anhydrous substance.

Single-crystal analysis revealed that the substance crystallizes in a triclinic unit cell with the following parameters: space group P-1, Å: a = 7.7582(5) Å, b = 8.0438(5) Å, c = 8.9915 (5) Å, α = 88.047(5)°, β = 74.657(6)°, γ = 70.657(6)°, and a volume of V = 509.69(6) Å³.

Next, an X-ray diffraction pattern of the obtained crystalline powder was recorded. Table 7 lists the 2θ positions and their relative intensities.

Based on the cell volume and symmetry, the crystal composition was determined as C₁₂H₁₆N₆O₂·2HI·2H₂O.

**Table 7. Intensity and 2θ positions of some peaks with relative intensities greater than 5% for the sample.**

| Position [°2θ] | Relative Intensity [%] |
|---|---|
| 11.68 | 17.41 |
| 15.98 | 9.76 |
| 18.22 | 59.86 |
| 18.88 | 33.29 |
| 20.4 | 100 |
| 20.98 | 3.69 |
| 22.56 | 86.27 |
| 23.04 | 24.43 |
| 23.58 | 41.08 |
| 24.56 | 12.6 |
| 25.08 | 21.37 |
| 26.2 | 88.35 |
| 27.08 | 6.49 |
| 28.12 | 17.95 |
| 28.48 | 13.97 |
| 30.96 | 10.78 |
| 31.34 | 26.38 |
| 31.56 | 10.48 |
| 31.92 | 16.59 |
| 32.06 | 12.8 |
| 33.3 | 19.62 |
| 36.46 | 17.68 |
| 36.92 | 7.98 |
| 46.16 | 8.39 |
| 47.08 | 4.38 |
| 48.3 | 4.68 |
| 49.16 | 4.78 |

In the X-ray diffraction pattern of C₁₂H₁₆N₆O₂·2HI·2H₂O, the following intense peaks (maxima) were observed at diffraction angles 2θ (± 0.2°): 18.2, 20.4, 22.6, 23.6, 26.2, 31.3, 33.3, and 36.5. The most intense maxima were displayed at 20.4, 22.6, and 26.2.

### Example 9. Synthesis of N,N'-bis[2-(1H-imidazol-4-yl)ethyl]oxalamide dihydrobromide (molar ratio 1:2)

To a solution of 4.92 mL (0.04 mol) of a 47% hydrobromic acid solution in 4.8 mL of water, 5.00 g (0.02 mol) of N,N'-bis[2-(1H-imidazol-4-yl)ethyl]oxalamide was added with stirring. The resulting suspension was stirred at 50°C until a solution was obtained. The reaction mixture was cooled to 20-25°C, 100 mL of acetone was added, and the mixture was stirred for 2 hours. The formed precipitate was filtered, washed with acetone (2 × 15.0 mL), and dried under vacuum at 60 °C to give a white crystalline powder.

Yield: 6.0 g (75.7%).

Retention time of the individual peak for C₁₂H₁₆N₆O₂: 4.0 min.

Quantitative determination of the base C₁₂H₁₆N₆O₂: 63.1% calculated, 62.2% determined by HPLC (internal normalization method), recalculated on an anhydrous substance.

Quantitative determination of the acidic residue in the salt: 36.9% calculated, 44.6% determined by acid-base titration, recalculated on an anhydrous substance.

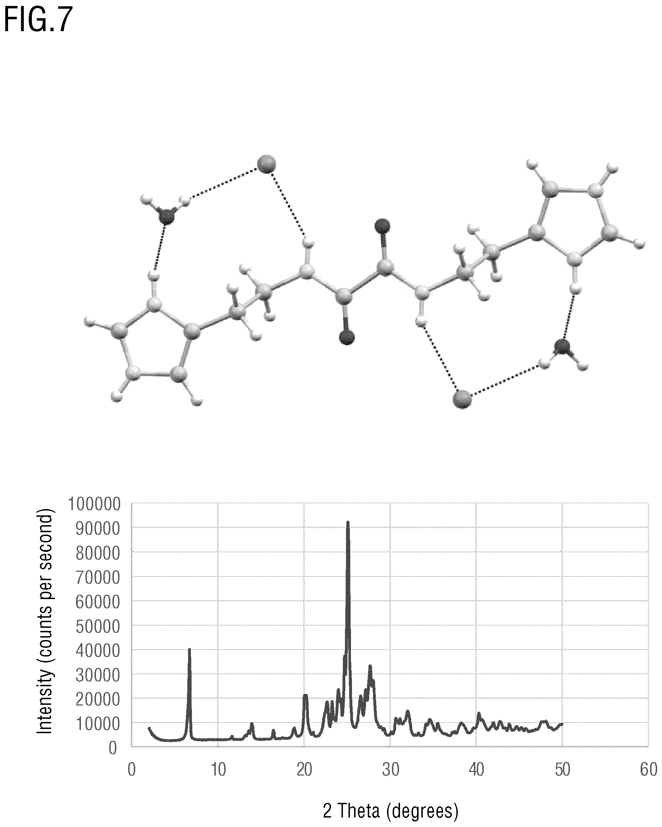

Next, an X-ray diffraction pattern of the obtained crystalline powder was recorded. Table 8 lists the 2θ positions and their relative intensities. Figure 7 shows the powder X-ray diffraction pattern of the obtained crystalline form.

**Table 8. Intensity and 2θ positions of some peaks with relative intensities greater than 5% for the sample.**

| Position [°2θ] | Relative Intensity [%] |
|---|---|
| 6.7318 | 38.57 |
| 11.6587 | 1.93 |
| 13.2065 | 1.6 |
| 13.6226 | 3.98 |
| 13.9692 | 10.36 |
| 16.464 | 3.46 |
| 18.8672 | 6.45 |
| 20.0543 | 6.51 |
| 20.2881 | 32.03 |
| 22.5081 | 25.17 |
| 22.7071 | 5.25 |
| 23.2731 | 9.22 |
| 23.4901 | 8.44 |
| 23.9446 | 38.74 |
| 24.6963 | 30.04 |
| 25.0749 | 100 |
| 26.5986 | 27.33 |
| 27.1509 | 18.91 |
| 27.6787 | 36.64 |
| 28.0095 | 33.46 |
| 30.6956 | 33.46 |
| 32.1208 | 19.76 |
| 34.1169 | 3.56 |
| 34.6087 | 21.72 |
| 35.4658 | 9.16 |
| 37.2273 | 0.67 |
| 37.524 | 0.95 |
| 38.2162 | 9.64 |
| 38.728 | 2.83 |
| 39.6708 | 6.2 |
| 40.3299 | 4.26 |
| 40.6744 | 12.66 |
| 41.7398 | 1.89 |
| 41.9705 | 3.77 |
| 42.7341 | 7.06 |
| 43.8366 | 2.24 |
| 44.7067 | 1.67 |
| 45.2291 | 1.07 |
| 45.7698 | 0.7 |
| 47.8356 | 18.83 |
| 49.6065 | 2.09 |

In the X-ray diffraction pattern of C₁₂H₁₆N₆O₂·2HBr, the following intense peaks (maxima) were observed at diffraction angles 2θ (± 0.2°): 6.7, 20.3, 22.5, 23.9, 24.7, 25.1, 26.6, 27.7, 28.0, 32.1, and 34.6. The most intense maxima were displayed at 6.7, 23.9, and 25.0.

### Example 10. Synthesis of N,N'-bis[2-(1H-imidazol-4-yl)ethyl]oxalamide dihydrobromide dihydrate (molar ratio 1:2)

To a solution of 4.92 mL (0.04 mol) of a 57% hydrobromic acid solution in 6.3 mL of water, 5.00 g (0.02 mol) of N,N'-bis[2-(1H-imidazol-4-yl)ethyl]oxalamide was added in 20 mL of water with stirring. The resulting suspension was stirred at 70°C until a solution was obtained. The reaction mixture was cooled to +25°C and left until a precipitate formed. The formed precipitate was filtered, washed with water, and dried under vacuum to give a white crystalline powder.

Yield: 6.0 g (88.7%).

Retention time of the individual peak for C₁₂H₁₆N₆O₂: 4.0 min.

Quantitative determination of the base C₁₂H₁₆N₆O₂: 58.3% calculated, 57.6% determined by HPLC (internal normalization method), recalculated on an anhydrous substance.

Quantitative determination of the acidic residue in the salt: 34.1% calculated, 33.6% determined by acid-base titration, recalculated on an anhydrous substance.

Single-crystal analysis showed that the substance crystallizes in a triclinic unit cell with the following parameters: space group P-1, Å: a = 5.11280(10) Å, b = 7.7959(2) Å, c = 12.6514(3) Å, α = 104.781(2)°, β = 96.311(2)°, γ = 100.375(2)°, and a volume of V = 473.13(2) Å³.

Next, an X-ray diffraction pattern of the obtained crystalline powder was recorded. Table 9 lists the 2θ positions and their relative intensities.

Based on the cell volume and symmetry, the crystal composition was determined as C₁₂H₁₆N₆O₂·2HBr·2H₂O.

**Table 9. Intensity and 2θ positions of some peaks with relative intensities greater than 5% for the sample.**

| Position [°2θ] | Relative Intensity [%] |
|---|---|
| 7.32 | 21.59 |
| 12.02 | 38.44 |
| 14.66 | 12.47 |
| 16.16 | 4.56 |
| 20.48 | 32.42 |
| 21.22 | 36.99 |
| 21.5 | 16.94 |
| 21.98 | 97.93 |
| 22.82 | 100 |
| 23.18 | 41.13 |
| 23.68 | 38.79 |
| 24.32 | 42.55 |
| 24.58 | 23.6 |
| 25.02 | 56.94 |
| 26.12 | 16.34 |
| 26.82 | 40.74 |
| 27.22 | 3.64 |
| 28.88 | 22.5 |
| 29.58 | 24.84 |
| 29.82 | 28.8 |
| 31.22 | 4.41 |
| 31.84 | 50.83 |
| 32.18 | 5.56 |
| 32.38 | 5.04 |
| 32.7 | 11.83 |
| 35.16 | 16.05 |
| 36.18 | 15.64 |
| 36.6 | 11.72 |
| 37.22 | 24.82 |
| 39.4 | 19.43 |
| 40.04 | 5.95 |
| 41.64 | 11.27 |
| 41.82 | 8.87 |
| 42.34 | 11.85 |
| 49.88 | 5.3 |

In the X-ray diffraction pattern of C₁₂H₁₆N₆O·2HBr·2H₂O, the following intense peaks (maxima) were observed at diffraction angles 2θ (± 0.2°): 12.0, 20.5, 21.2, 22.0, 22.8, 23.2, 23.7, 24.3, 25.0, 26.8, and 31.8. The most intense maxima were displayed at 22.0, 22.8, and 25.0.

### Example 11. Synthesis of N,N'-bis[2-(1H-imidazol-4-yl)ethyl]oxalamide sulfate trihydrate (molar ratio 1:1)

To a solution of 2.96 mL (0.03 mol) of sulfuric acid (96%) in 26.1 mL of water, 8.00 g (0.03 mol) of N,N'-bis[2-(1H-imidazol-4-yl)ethyl]oxalamide was added with stirring. The resulting suspension was stirred at 50°C until a solution was obtained. The reaction mixture was cooled to 20-25 °C, 53 mL of acetone was added, and the mixture was stirred for 2 hours. The formed precipitate was filtered, washed with acetone (2 × 15.0 mL), and dried under vacuum at 60°C to give a white crystalline powder.

Yield: 11.3 g (99.1%).

Retention time of the individual peak for C₁₂H₁₆N₆O: 4.1 min.

Quantitative determination of the base C₁₂H₁₆N₆O: 67.3% calculated; 68.7% determined by HPLC (internal normalization method), recalculated on an anhydrous substance.

Quantitative determination of the acidic residue in the salt: 23.9% calculated; 23.8% determined by acid-base titration, recalculated on an anhydrous substance.

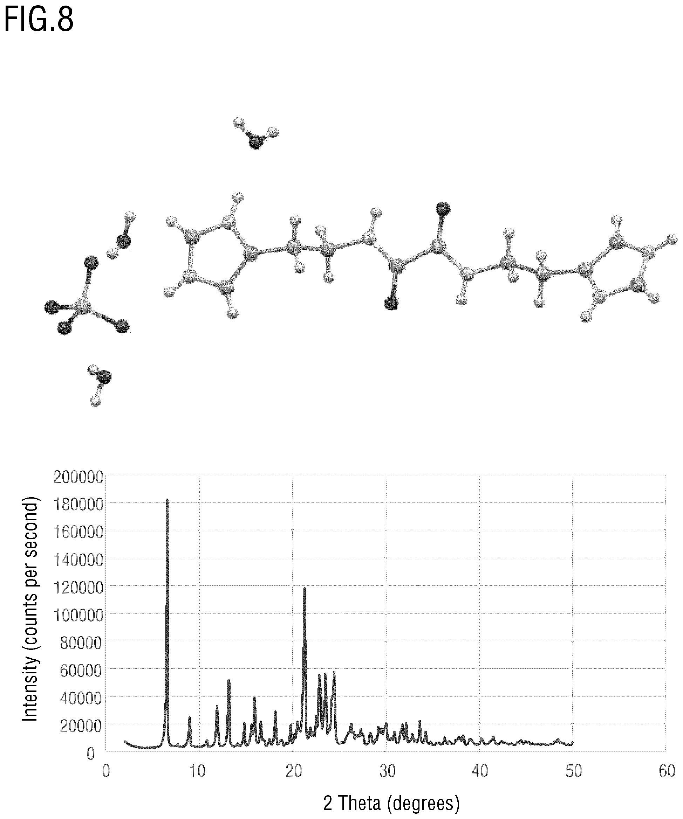

Next, an X-ray diffraction pattern of the obtained crystalline powder was recorded. Table 10 lists the 2θ positions and their relative intensities s. Figure 8 shows the powder X-ray diffraction pattern of the obtained crystalline form.

Single-crystal analysis showed that the substance crystallizes in a monoclinic unit cell with the following parameters: space group P21/c, Å: a = 8.22400(13) Å, b = 8.76891(14) Å, c = 27.1346(4) Å, β = 97.6144(16)°, and a volume of V = 1939.57(5) Å³.

Based on the cell volume and symmetry, the crystal composition was determined as C₁₂H₁₆N₆O·H₂SO₄·3H₂O.

**Table 10. Intensity and 2θ positions of some peaks with relative intensities greater than 5% for the sample.**

| Position [°2θ] | Relative Intensity [%] |
|---|---|
| 6.3414 | 17.72 |
| 6.5872 | 100.00 |
| 7.7273 | 2.25 |
| 8.9801 | 19.56 |
| 10.8516 | 6.26 |
| 11.8554 | 10.18 |
| 11.9202 | 19.05 |
| 13.1626 | 38.54 |
| 14.1100 | 2.50 |
| 14.8228 | 13.24 |
| 15.6347 | 15.52 |
| 15.9235 | 22.08 |
| 16.5847 | 10.30 |
| 17.5380 | 2.76 |
| 18.1753 | 17.93 |
| 18.6813 | 1.10 |
| 19.8039 | 8.68 |
| 20.2184 | 4.01 |
| 20.5030 | 12.02 |
| 20.6859 | 10.85 |
| 21.1044 | 23.55 |
| 21.2994 | 71.86 |
| 21.8998 | 6.69 |
| 22.1546 | 5.01 |
| 22.5294 | 11.50 |
| 22.8630 | 43.61 |
| 23.0258 | 6.73 |
| 23.5646 | 38.96 |
| 24.1538 | 7.01 |
| 24.4654 | 68.50 |
| 26.0090 | 26.96 |
| 26.2507 | 7.54 |
| 26.5141 | 1.79 |
| 27.3477 | 17.73 |
| 28.2848 | 8.11 |
| 29.7359 | 10.67 |
| 31.5532 | 9.42 |
| 33.6460 | 12.17 |
| 35.4079 | 0.85 |
| 35.6447 | 1.72 |
| 36.7856 | 1.20 |
| 37.9284 | 4.38 |
| 38.2576 | 5.64 |
| 38.9457 | 2.49 |
| 39.2618 | 1.87 |
| 40.2312 | 2.41 |
| 41.2305 | 3.90 |
| 41.4072 | 1.49 |
| 42.4101 | 1.89 |
| 44.9166 | 2.12 |

In the X-ray diffraction pattern of C₁₂H₁₆N₆O·H₂SO₄·3H₂O, the following intense peaks (maxima) were observed at diffraction angles 2θ (± 0.2°): 6.3, 6.6, 11.9, 13.2, 15.9, 18.2, 21.1, 21.3, 22.9, 23.6, 24.5, 26.0, and 27.4. The most intense maxima were displayed at 6.6, 21.3, and 24.5.

### Example 12. Synthesis of N,N'-bis[2-(1H-imidazol-4-yl)ethyl]oxalamide dibenzenesulfonate (molar ratio 1:2)

To a solution of 5.73 g (0.04 mol) of malonic acid in 6.3 mL of water, 5.00 g (0.02 mol) of N,N'-bis[2-(1H-imidazol-4-yl)ethyl]oxalamide was added with stirring. The resulting suspension was stirred at 50°C until a solution was obtained. The reaction mixture was cooled to 20-25°C, 163 mL of acetone was added, and the mixture was stirred for 2 hours. The formed precipitate was filtered, washed with acetone (2 × 15.0 mL), and dried under vacuum at 60°C to give a white crystalline powder.

Yield: 9.3 g (86.9%).

Retention time of the individual peak for C₁₂H₁₆N₆O: 4.0 min.

Quantitative determination of the base C₁₂H₁₆N₆O: 46.6% calculated, 45.8% determined by HPLC (internal normalization method), recalculated on an anhydrous substance.

Quantitative determination of the acidic residue in the salt: 53.4% calculated, 52.5% determined by acid-base titration, recalculated on an anhydrous substance.

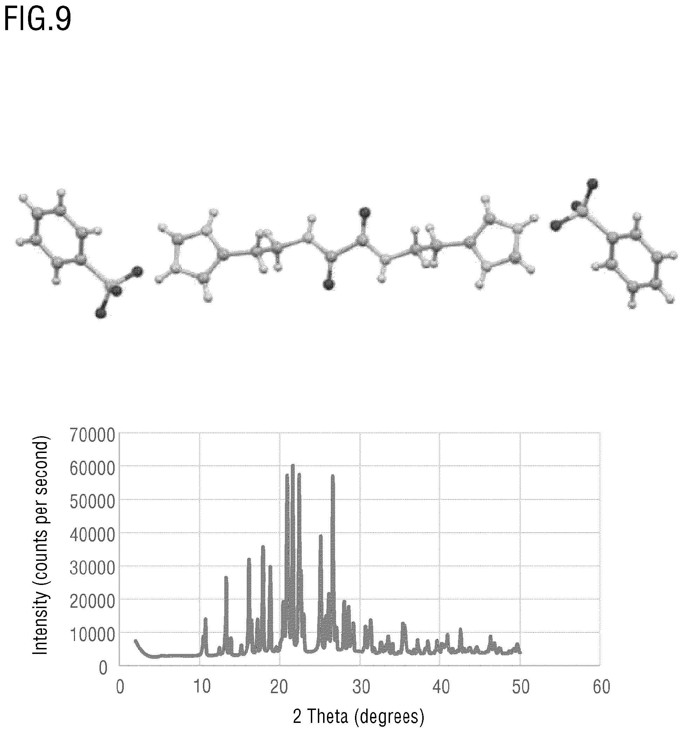

Next, an X-ray diffraction pattern of the obtained crystalline powder was recorded. Table 11 lists the 2θ positions and their relative intensities. Figure 9 shows the powder X-ray diffraction pattern of the obtained crystalline form.

Single-crystal analysis showed that the substance crystallizes in a monoclinic unit cell with the following parameters: space group P21/c, Å: a = 9.6118(2) Å, b = 10.7375(2) Å, c = 12.9924(2) Å, β = 101.923(2)°, and a volume of V = 1311.97(4) Å³.

Based on the cell volume and symmetry, the crystal composition was determined as C₁₂H₁₆N₆O·2C₆H₆O₃S.

**Table 11. Intensity and 2θ positions of some peaks with relative intensities greater than 5% for the sample.**

| Position [°2θ] | Relative Intensity [%] |
|---|---|
| 10.4656 | 6.97 |
| 10.7678 | 15.56 |
| 13.3215 | 32.28 |
| 13.9121 | 7.99 |
| 15.2326 | 6.42 |
| 16.1817 | 37.87 |
| 16.4704 | 13.31 |
| 17.2202 | 13.4 |
| 17.9023 | 42.81 |
| 18.8427 | 34.69 |
| 20.1315 | 16.24 |
| 20.4203 | 14.32 |
| 20.8974 | 100 |
| 21.6262 | 68.95 |
| 22.4261 | 66.54 |
| 22.6284 | 41.14 |
| 22.97 | 13.73 |
| 24.9932 | 21.03 |
| 25.1388 | 45.84 |
| 25.7667 | 14.94 |
| 26.1021 | 20.15 |
| 26.5918 | 65.35 |
| 26.7416 | 10.79 |
| 27.065 | 11.24 |
| 28.0162 | 22.44 |
| 28.5655 | 17.81 |
| 28.9174 | 7.68 |
| 29.1838 | 11.44 |
| 30.7166 | 6.72 |
| 31.0537 | 6.02 |
| 31.3256 | 14.74 |
| 32.5923 | 4.58 |
| 33.5351 | 6.08 |
| 34.0666 | 4.06 |
| 35.3424 | 16.45 |
| 35.6492 | 13.16 |
| 37.225 | 5.12 |
| 38.513 | 5.37 |
| 39.6512 | 4.94 |
| 40.2711 | 3.79 |
| 40.6907 | 4.11 |
| 40.9579 | 6.68 |
| 42.558 | 10.94 |
| 46.3467 | 3.27 |
| 46.6898 | 4.51 |

In the X-ray diffraction pattern of C₁₂H₁₆N₆O·2C₆H₆O₃S, the following intense peaks (maxima) were observed at diffraction angles 2θ (± 0.2°): 13.3, 16.2, 17.9, 18.8, 20.9, 21.6, 22.4, 22.6, 25.0, 25.1, and 26.6. The most intense maxima were displayed at 20.9, 21.6, and 22.4.

### Example 13. Synthesis of N,N'-bis[2-(1H-imidazol-4-yl)ethyl]oxalamide L,D-tartrate (molar ratio 1:1)

To a solution of 2.72 g (0.03 mol) of L,D-tartaric acid in 6.3 mL of water, 5.00 g (0.03 mol) of N,N'-bis[2-(1H-imidazol-4-yl)ethyl]oxalamide was added with stirring. The resulting suspension was stirred at 75°C until a solution was obtained. The reaction mixture was cooled to 20-25 °C, 33 mL of acetone was added, and the mixture was stirred for 2 hours. The formed precipitate was filtered, washed with acetone (2 × 15.0 mL), and dried under vacuum at 60°C to give a white crystalline powder.

Yield: 6.4 g (82.8%).

Retention time of the individual peak for C₁₂H₁₆N₆O: 3.7 min.

Quantitative determination of the base C₁₂H₁₆N₆O: 64.8 % calculated as, 64.5% determined by HPLC (internal normalization method), recalculated on an anhydrous substance.

Quantitative determination of the acidic residue in the salt: 35.2% calculated, 35.1% determined by acid-base titration, recalculated on an anhydrous substance.

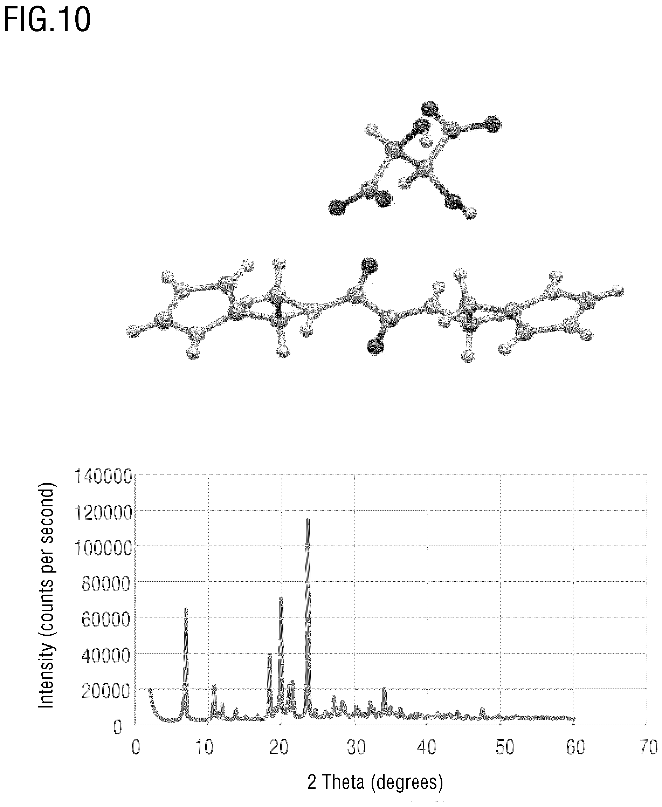

Next, an X-ray diffraction pattern of the obtained crystalline powder was recorded. Table 12 lists the 2θ positions and their relative intensities. Figure 10 shows the powder X-ray diffraction pattern of the obtained crystalline form. Single-crystal analysis revealed that the substance crystallizes in a triclinic unit cell with the following parameters: space group P-1, Å: a = 9.0057 (2) Å, b = 9.1627(2) Å, c = 13.5418(2) Å, α = 108.5901(18)°, β = 91.8688(17)°, γ = 112.104(2)°, and a volume of V = 966.36(4) Å³.

Based on the cell volume and symmetry, the crystal composition was determined as C₁₂H₁₆N₆O·C₄H₆O₆.

**Table 12. Intensity and 2θ positions of some peaks with relative intensities greater than 5% for the sample.**

| Position [°2θ] | Relative Intensity [%] |
|---|---|
| 6.9658 | 57.25 |
| 10.8103 | 17.53 |
| 11.8871 | 8.63 |
| 13.7874 | 5.85 |
| 18.3903 | 31.43 |
| 19.9564 | 54.93 |
| 20.8593 | 10.03 |
| 21.0836 | 10.33 |
| 21.4613 | 17.73 |
| 21.6992 | 6.02 |
| 23.6055 | 100 |
| 27.1934 | 10.07 |
| 27.4768 | 4.94 |
| 28.1738 | 6.22 |
| 28.3746 | 3.81 |
| 30.2181 | 4.14 |
| 32.0979 | 8.42 |
| 34.0428 | 14.4 |

In the X-ray diffraction pattern of C₁₂H₁₆N₆O·C₄H₆O₆, the following intense peaks (maxima) were observed at diffraction angles 2θ (± 0.2°): 7.0, 10.8, 18.4, 20.0, 21.08, 21.5, and 27.2. The most intense maxima were displayed at 7.0, 20.0, and 23.6.

### Example 14. Synthesis of N,N'-bis[2-(1H-imidazol-4-yl)ethyl]oxalamide L-malate (molar ratio 1:1)

To a solution of 3.4 g (0.03 mol) of L-malic acid in 22 mL of water, 7.00 g (0.03 mol) of N,N'-bis[2-(1H-imidazol-4-yl)ethyl]oxalamide was added with stirring. The resulting suspension was stirred at 50°C until a solution was obtained. The reaction mixture was cooled to 5°C, 58 mL of acetone was added, and the mixture was stirred for 2 hours. The formed precipitate was filtered, washed with acetone (2 × 15.0 mL), and dried under vacuum at 60°C to give a white crystalline powder.

Yield: 8.3 g (79.5%).

Retention time of the individual peak for C₁₂H₁₆N₆O: 3.7 min.

Quantitative determination of the base C₁₂H₁₆N₆O: 67.3% calculated as, 67.6% determined by HPLC (internal normalization method), recalculated on an anhydrous substance.

Quantitative determination of the acidic residue in the salt: 32.7% calculated, 32.8% determined by acid-base titration, recalculated on an anhydrous substance.

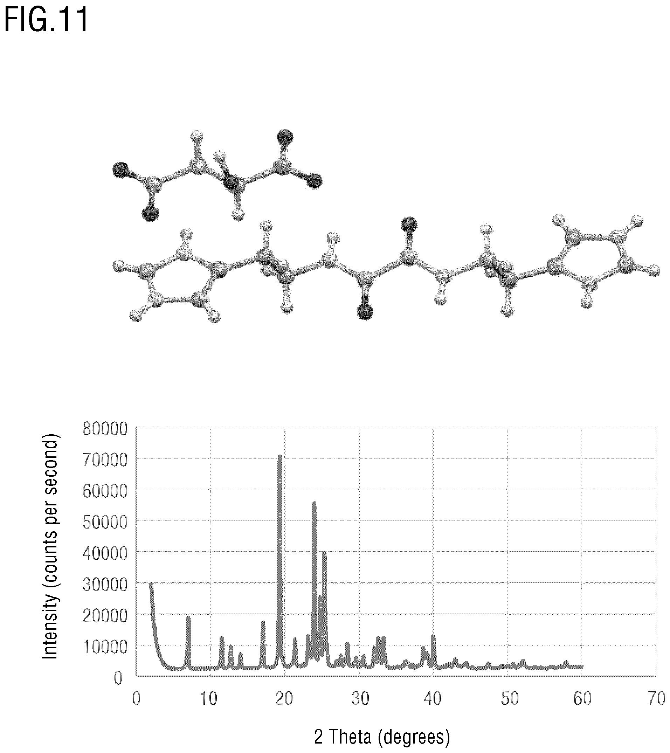

Next, an X-ray diffraction pattern of the obtained crystalline powder was recorded. Table 13 lists the 2θ positions and their relative intensities. Figure 11 shows the powder X-ray diffraction pattern of the obtained crystalline form. Single-crystal analysis revealed that the substance crystallizes in a triclinic unit cell with the following parameters: space group P-1, Å: a = 4.67918(18) Å, b = 7.8122(3) Å, c = 12.7423(4) Å, α = 81.635(3)°, β = 81.774(3)°, γ = 80.656(3)°, and a volume of V = 451.30(3) Å³.

Based on the cell volume and symmetry, the crystal composition was determined as C₁₂H₁₆N₆O·C₄H₆O₅.

**Table 13. Intensity and 2θ positions of some peaks with relative intensities greater than 5% for the sample.**

| Position [°2θ] | Relative Intensity [%] |
|---|---|
| 7.0001 | 23.34 |
| 11.5289 | 16.75 |
| 12.7364 | 11.46 |
| 14.0648 | 7.11 |
| 17.0676 | 24.26 |
| 19.3304 | 100 |
| 21.3996 | 19.72 |
| 23.1461 | 15.82 |
| 23.4253 | 6.73 |
| 23.9967 | 80.38 |
| 24.7517 | 34.06 |
| 25.3198 | 64.63 |
| 25.6816 | 9.48 |
| 28.4178 | 12.89 |
| 30.6002 | 5.98 |
| 31.9882 | 13.15 |
| 32.5608 | 17.96 |
| 33.2298 | 12.97 |
| 38.6649 | 13.27 |
| 39.0633 | 9.04 |
| 39.2987 | 5.01 |
| 40.0385 | 16.1 |

In the X-ray diffraction pattern of C₁₂H₁₆N₆O·C₄H₆O₅, the following intense peaks (maxima) were observed at diffraction angles 2θ (± 0.2°): 67.0, 11.5, 17.1, 19.3, 21.4, 24.0, 24.8, 25.3, and 32.6. The most intense maxima were displayed at 19.3, 24.0, and 25.3.

### Example 15. Synthesis of N,N'-bis[2-(1H-imidazol-4-yl)ethyl]oxalamide di-p-toluenesulfonate (molar ratio 1:2)

To a solution of 6.9 g (0.04 mol) of p-toluenesulfonic acid in 6.3 mL of water 5.00 g (0.02 mol) of N,N'-bis[2-(1H-imidazol-4-yl)ethyl]oxalamide was added with stirring. The resulting suspension was stirred at 50 °C until a clear solution was obtained. The reaction mixture was cooled to 20-25°C, 33 mL of acetone was added, and the mixture was stirred for 2 hours. The formed precipitate was filtered, washed with acetone (2 × 15.0 mL), and dried under vacuum at 60°C to give a white crystalline powder.

Yield: 7.5 g (66.5%).

Retention time of the individual peak for C₁₂H₁₆N₆O: 4.1 min.

Quantitative determination of the base C₁₂H₁₆N₆O: 42.1% calculated, 41.6% determined by HPLC (internal normalization method), recalculated on an anhydrous basis.

Quantitative determination of the acid residue in the salt: 57.9% calculated, 57.3% determined by acid-base titration, recalculated on an anhydrous basis.

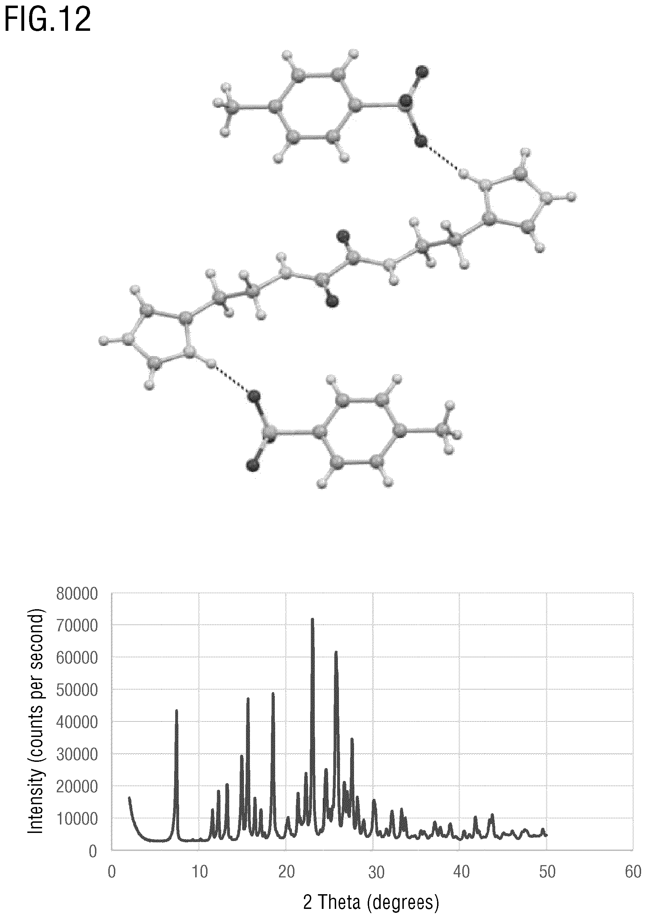

Next, an X-ray diffraction pattern of the crystalline powder was recorded. Table 14 lists the 2θ positions and their relative intensities. Figure 12 shows the powder X-ray diffraction pattern of the obtained crystalline form. Single-crystal analysis revealed that the compound crystallizes in a monoclinic unit cell with the following parameters: space group P21/c, Å: a = 7.25360(10) Å, b = 8.06360(10) Å, c = 23.8621(4) Å, α = 90°, β = 95.812(2)°, γ = 90°, with a cell volume of V = 451.30(3) Å³.

Based on the cell volume and symmetry, the crystal composition was determined as C₁₂H₁₆N₆O₂·2C₇H₈O₃S.

**Table 14. Intensity and 2θ positions of some peaks with relative intensities greater than 5% for the sample.**

| Position [°2θ] | Relative Intensity [%] |
|---|---|
| 7.4373 | 59.08 |
| 11.5623 | 14.24 |
| 12.2483 | 22.94 |
| 13.2444 | 25.38 |
| 14.9447 | 37.12 |
| 15.6572 | 65.08 |
| 16.4604 | 18.32 |
| 17.5670 | 2.32 |
| 18.3505 | 10.11 |
| 18.5431 | 56.27 |
| 20.0950 | 4.60 |
| 20.4811 | 1.28 |
| 21.3959 | 17.70 |
| 22.3127 | 27.98 |
| 23.0795 | 100.00 |
| 24.4896 | 14.03 |
| 24.6334 | 26.37 |
| 25.0209 | 9.63 |
| 25.7095 | 57.78 |
| 25.8935 | 58.86 |
| 26.7150 | 22.05 |
| 27.6056 | 43.67 |
| 28.1877 | 16.32 |
| 28.9379 | 7.00 |
| 30.0961 | 7.27 |
| 30.2067 | 14.62 |
| 31.5770 | 3.65 |
| 32.2002 | 12.26 |
| 33.2880 | 14.13 |
| 33.7249 | 9.77 |
| 34.8323 | 1.28 |
| 36.6300 | 1.21 |
| 37.1679 | 7.11 |
| 38.9565 | 6.82 |
| 40.5168 | 3.82 |
| 41.8405 | 10.41 |
| 43.1547 | 0.47 |
| 43.7517 | 11.16 |
| 46.1012 | 2.66 |
| 47.4872 | 3.39 |
| 48.5268 | 0.62 |
| 49.5746 | 4.02 |

In the X-ray diffraction pattern of C₁₂H₁₆N₆O₂·2C₇H₈O₃S, the following intense peaks (maxima) were observed at diffraction angles 2θ (± 0.2°): 7.4, 12.3, 13.2, 14.9, 15.7, 18.3, 18.5, 21.4, 22.3, 23.1, 24.6, 25.4, 25.7, 25.9, 26.7, 27.0, 27.6, 28.2, 30.2, 32.2, and 33.3. The most intense maxima were displayed at 7.4, 15.7, and 23.1.

### Example 16. Synthesis of N,N'-bis[2-(1H-imidazol-4-yl)ethyl]oxalamide oxalate (molar ratio 1:1)

To a solution of 2.28 g (0.02 mol) of oxalic acid dihydrate in 23.3 mL of water, 5.00 g (0.02 mol) of N,N'-bis[2-(1H-imidazol-4-yl)ethyl]oxalamide was added with stirring. The resulting suspension was stirred at 90°C until a clear solution was formed. The reaction mixture was cooled to 20-25°C, 25 mL of acetone was added, and the mixture was stirred for 2 hours. The formed precipitate was filtered, washed with acetone (2 × 15.0 mL), and dried under vacuum at 60°C to give a white crystalline powder.

Yield: 6.0 g (91.0%).

Retention time of the individual peak for C₁₂H₁₆N₆O₂: 3.7 min.

Quantitative determination of the base C₁₂H₁₆N₆O₂: 68.7% calculated, 67.5% determined by HPLC (internal normalization method), recalculated on an anhydrous basis.

Quantitative determination of the acid residue in the salt: 31.3% calculated, 30.8% determined by acid-base titration, recalculated on an anhydrous basis.

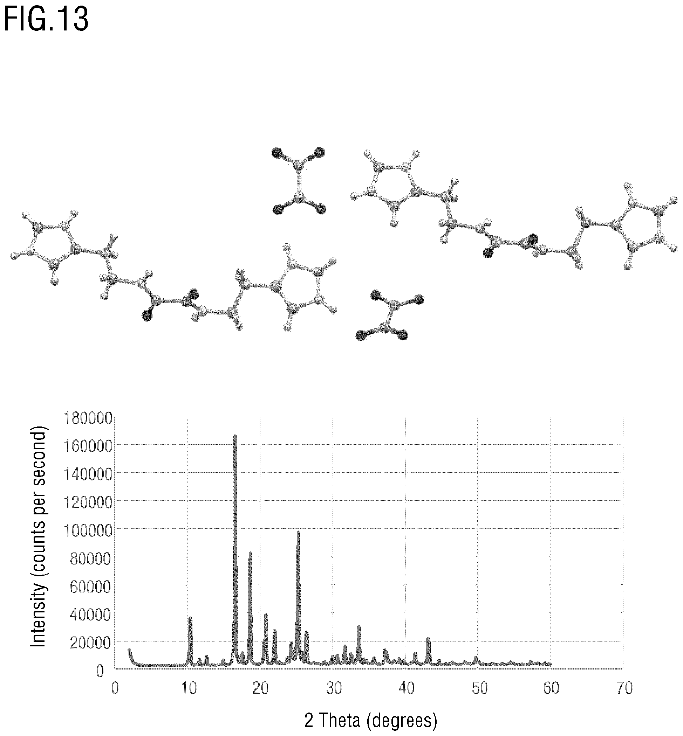

The X-ray diffraction pattern of the crystalline powder was then recorded. Table 15 lists the 2θ positions and their relative intensities. Figure 13 shows the powder X-ray diffraction pattern of the obtained crystalline form. Single-crystal analysis revealed that the compound crystallizes in a monoclinic unit cell with the following parameters: space group I2/a, Å: a = 14.90632(17) Å, b = 21.3104(2) Å, c = 22.4265(3) Å, β = 22.4265(3)° [note: this β value appears unusual], with a cell volume of V = 6785.83(14) Å³.

Based on the cell volume and symmetry, the crystal composition was determined as C₁₂H₁₆N₆O₂·C₂H₂O₄.

**Table 15. Intensity and 2θ positions of some peaks with relative intensities greater than 5% for the sample.**

| Position [°2θ] | Relative Intensity [%] |
|---|---|
| 10.4398 | 45.18 |
| 12.6713 | 11.56 |
| 16.5438 | 94.84 |
| 16.6224 | 95.82 |
| 17.5831 | 10.74 |
| 18.6619 | 100 |
| 20.5774 | 16.44 |
| 20.8217 | 43.86 |
| 22.0268 | 28.34 |
| 24.2751 | 10.1 |
| 25.0727 | 53.4 |
| 25.2877 | 66.96 |
| 25.8497 | 5.9 |
| 26.4029 | 23.09 |
| 30.5998 | 7.7 |
| 31.6579 | 13.97 |
| 32.4915 | 8.64 |
| 33.6132 | 26.39 |
| 37.1738 | 9.86 |
| 37.3728 | 9.24 |
| 41.3852 | 9.32 |
| 43.1459 | 21.85 |

In the X-ray diffraction pattern of C₁₂H₁₆N₆O₂·C₂H₂O₄, the following intense peaks (maxima) were observed at diffraction angles 2θ (± 0.2°): 10.4, 16.5, 16.6, 18.7, 20.8, 22.0, 25.1, 25.3, 26.4, 33.6, and 43.2. The most intense maxima were displayed at 16.5, 16.6, and 18.7.

### Example 17. Synthesis of N,N'-bis[2-(1H-imidazol-4-yl)ethyl]oxalamide dioxalate (molar ratio 1:2)

To a solution of 4.56 g (0.04 mol) of oxalic acid dihydrate in 20.3 mL of water, 5.00 g (0.02 mol) of N,N'-bis[2-(1H-imidazol-4-yl)ethyl]oxalamide was added with stirring. The resulting suspension was stirred at 80°C until a solution was obtained. The reaction mixture was cooled to 20-25 °C, 33 mL of acetone was added, and the mixture was stirred for 2 hours. The formed precipitate was filtered, washed with acetone (2 × 15.0 mL), and dried under vacuum at 60°C to give a white crystalline powder.

Yield: 7.4 g (89.5%).

Retention time of the individual peak for C₁₂H₁₆N₆O₂: 4.1 min.

Quantitative determination of the base C₁₂H₁₆N₆O₂: 52.3% calculated, 51.8% determined by HPLC (internal normalization method), recalculated on an anhydrous basis.

Quantitative determination of the acid residue in the salt: 47.7% calculated, 47.2% determined by acid-base titration, recalculated on an anhydrous basis.

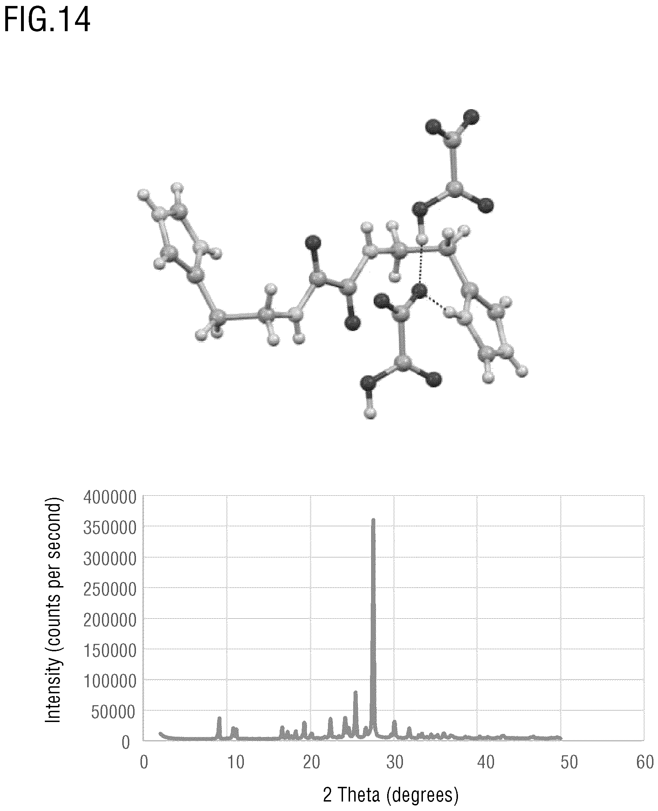

The X-ray diffraction pattern of the crystalline powder was recorded. Table 16 lists the 2θ positions and their relative intensities. Figure 14 shows the powder X-ray diffraction pattern of the obtained crystalline form. Single-crystal analysis revealed that the compound crystallizes in a monoclinic unit cell with the following parameters: space group P21/n, Å: a = 10.8403(2) Å, b = 5.64120(10) Å, c = 16.0993(3) Å, β = 100.507(2)°, with a cell volume of V = 968.0(3) Å³.

Based on the cell volume and symmetry, the crystal composition was determined as C₁₂H₁₆N₆O₂·2C₂H₂O₄.

**Table 16. Intensity and 2θ positions of some peaks with relative intensities greater than 5% for the sample.**

| Position [°2θ] | Relative Intensity [%] |
|---|---|
| 9.1011 | 8.59 |
| 19.2651 | 8.83 |
| 22.4015 | 8.71 |
| 24.1319 | 12.02 |
| 25.3891 | 22.4 |
| 27.5252 | 100 |
| 30.0428 | 9.73 |

In the X-ray diffraction pattern of C₁₂H₁₆N₆O₂·2C₂H₂O₄, the following intense peaks (maxima) were observed at diffraction angles 2θ (± 0.2°): 19.3, 22.4, 24.1, 25.4, 27.5, and 30.0. The most intense maxima were displayed at 24.1, 25.4, and 27.5.

### Example 18. Synthesis of N,N'-bis[2-(1H-imidazol-4-yl)ethyl]oxalamide diadipinate monohydrate (molar ratio 1:2)

To a solution of 0.215 g (1.45 mmol) of adipic acid in 3.5 mL of water, 0.200 g (0.72 mmol) of N,N'-bis[2-(1H-imidazol-4-yl)ethyl]oxalamide was added with stirring. The resulting suspension was stirred at 70 °C until a clear solution was obtained. The reaction mixture was cooled to +25°C and left until a precipitate formed. The formed precipitate was filtered, washed with water, and dried under vacuum to give a white crystalline powder.

Yield: 0.40 g (98.0%).

Retention time of the individual peak for C₁₂H₁₆N₆O₂: 3.7 min.

Quantitative determination of the base C₁₂H₁₆N₆O₂: 48.6% calculated, 48.4% determined by HPLC (internal normalization method), recalculated on an anhydrous basis.

Quantitative determination of the acid residue in the salt: 51.2% calculated, 50.9% determined by acid-base titration, recalculated on an anhydrous basis.

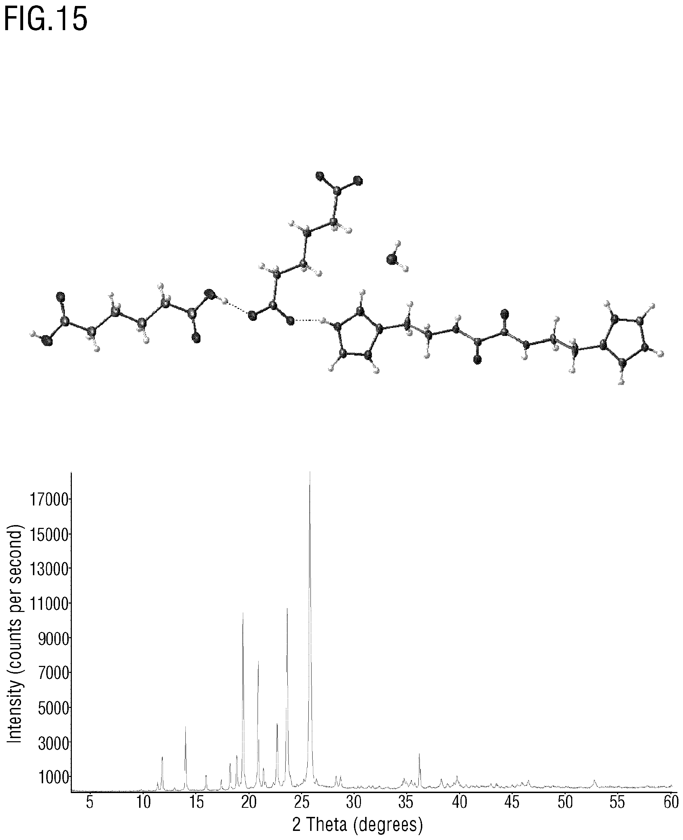

The X-ray diffraction pattern of the crystalline powder was recorded. Table 17 lists the 2θ positions and their relative intensities. Figure 15 shows the powder X-ray diffraction pattern of the obtained crystalline form.

**Table 17. Intensity and 2θ positions of some peaks with relative intensities greater than 5% for the sample.**

| Position [°2θ] | Relative Intensity [%] |
|---|---|
| 9.852 | 0.40 |
| 11.406 | 2.40 |
| 11.835 | 9.20 |
| 12.987 | 0.80 |
| 14.048 | 19.20 |
| 15.983 | 4.40 |
| 17.413 | 3.20 |
| 18.246 | 8.00 |
| 18.884 | 10.30 |
| 19.485 | 53.10 |
| 20.91 | 38.90 |
| 21.411 | 6.70 |
| 21.598 | 1.90 |
| 22.36 | 1.40 |
| 22.711 | 23.10 |
| 23.674 | 54.20 |
| 24.6 | 1.10 |
| 25.035 | 1.20 |
| 25.237 | 2.40 |
| 25.797 | 100.00 |
| 26.43 | 2.50 |
| 28.306 | 3.60 |
| 28.718 | 3.80 |
| 30.348 | 0.70 |
| 30.677 | 0.80 |
| 31.4 | 0.70 |
| 31.788 | 0.40 |
| 32.411 | 0.80 |
| 33.22 | 0.70 |
| 34.485 | 1.30 |
| 34.769 | 2.70 |
| 34.99 | 2.10 |
| 35.452 | 2.40 |
| 35.777 | 1.80 |
| 36.204 | 13.20 |
| 37.211 | 0.70 |
| 38.288 | 2.80 |
| 38.907 | 1.20 |
| 39.488 | 1. 90 |
| 39.78 | 4.00 |
| 40.62 | 1.00 |
| 41.15 | 0.50 |
| 41.627 | 1.00 |
| 42.224 | 0.30 |
| 42.999 | 1.30 |
| 43.5 | 1.70 |
| 43.965 | 0.50 |
| 44.461 | 0.40 |
| 45.021 | 1.00 |
| 45.473 | 1.10 |
| 45.926 | 1.70 |
| 46.562 | 2.20 |
| 48.072 | 0.50 |
| 48.896 | 0.30 |
| 49.337 | 0.60 |
| 52.532 | 0.60 |
| 52.781 | 2.50 |
| 55.095 | 0.50 |
| 57.797 | 0.70 |

In the X-ray diffraction pattern of C₁₂H₁₆N₆O₂·C₄H₁₀O₄·H₂O, the following intense peaks (maxima) were observed at diffraction angles 2θ (± 0.2°): 14.1, 18.9, 19.5, 20.9, 22.7, 23.7, 25.8, and 36.2. The most intense maxima were displayed at 19.5, 23.57, and 25.8.

### Example 19. Synthesis of N,N'-bis[2-(1H-imidazol-4-yl)ethyl]oxalamide succinate (molar ratio 1:1)

To a solution of 2.14 g (0.02 mol) of succinic acid in 31.3 mL of water, 5.00 g (0.02 mol) of N,N'-bis[2-(1H-imidazol-4-yl)ethyl]oxalamide was added with stirring. The resulting suspension was stirred at 80°C until a solution was obtained. The reaction mixture was cooled to 20-25 °C, 33 mL of acetone was added, and the mixture was stirred for 2 hours. The formed precipitate was filtered, washed with acetone (2 × 15.0 mL), and dried under vacuum at 60°C to give a white crystalline powder.

Yield: 5.74 g (85.6%).

Retention time of the individual peak for C₁₂H₁₆N₆O₂: 4.0 min.

Quantitative determination of the base C₁₂H₁₆N₆O₂: 52.4% calculated, 53.2% determined by HPLC (internal normalization method), recalculated on an anhydrous basis.

Quantitative determination of the acid residue in the salt: 47.1% calculated, 47.4% determined by acid-base titration, recalculated on an anhydrous basis.

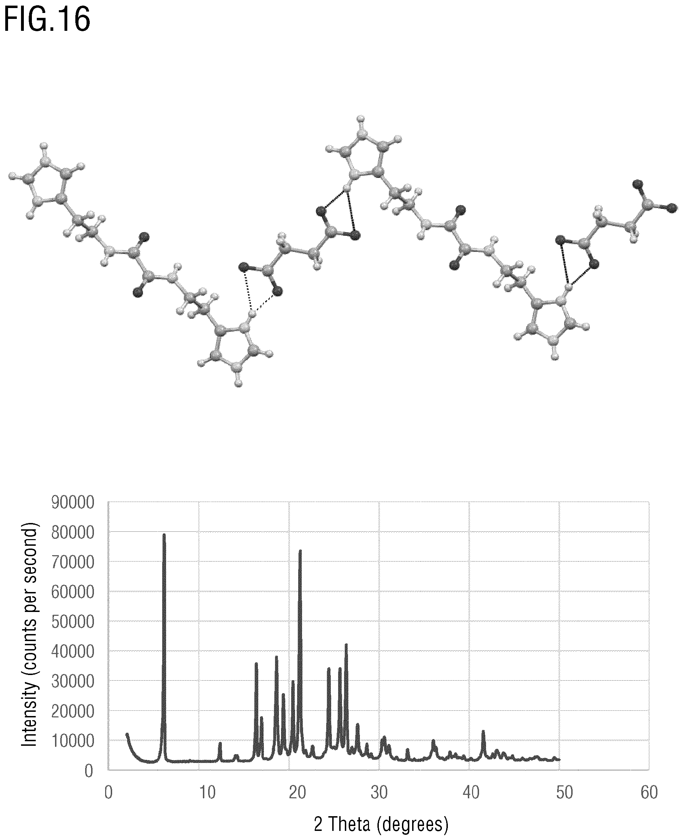

Next, an X-ray diffraction pattern of the crystalline powder was recorded. Table 18 lists the 2θ positions and their relative intensities. Figure 16 shows the powder X-ray diffraction pattern of the obtained crystalline form. Single-crystal analysis revealed that the compound crystallizes in a monoclinic unit cell with the following parameters: space group P2(1), Å: a = 13.0213(4) Å, b = 5.08060(10) Å, c = 14.5124(5) Å, α = 90°, β = 104.871(3)°, γ = 90°, with a cell volume of V = 927.93(5) Å³.

Based on the cell volume and symmetry, the crystal composition was determined as C₁₂H₁₆N₆O₂·C₄H₆O₄.

**Table 18. Intensity and 2θ positions of some peaks with relative intensities greater than 5% for the sample.**

| Position [°2θ] | relative intensity [%] |
|---|---|
| 6.18 | 100.00 |
| 12.34 | 6.36 |
| 14.02 | 1.90 |
| 14.30 | 1.79 |
| 16.38 | 42.94 |
| 16.94 | 17.53 |
| 18.64 | 44.19 |
| 19.38 | 27.33 |
| 19.72 | 3.65 |
| 20.24 | 7.15 |
| 20.44 | 34.08 |
| 21.20 | 84.42 |
| 21.82 | 4.05 |
| 22.62 | 5.58 |
| 23.92 | 2.88 |
| 24.42 | 38.69 |
| 25.02 | 1.12 |
| 25.64 | 38.00 |
| 26.32 | 47.03 |
| 26.96 | 4.68 |
| 27.58 | 13.63 |
| 27.88 | 1.37 |
| 28.36 | 2.43 |
| 28.64 | 6.88 |
| 29.12 | 2.48 |
| 30.34 | 7.58 |
| 30.60 | 6.91 |
| 31.06 | 5.84 |
| 31.90 | 1.13 |
| 33.16 | 4.58 |
| 33.92 | 0.50 |
| 35.04 | 0.79 |
| 36.06 | 7.55 |
| 36.38 | 3.79 |
| 36.68 | 1.13 |
| 37.02 | 0.90 |
| 37.52 | 0.90 |
| 37.88 | 3.25 |
| 38.24 | 1. 68 |
| 38.50 | 2.65 |
| 39.10 | 0.97 |
| 39.42 | 1.75 |
| 40.22 | 0.70 |
| 41.14 | 0.57 |
| 41.40 | 0.00 |
| 41.58 | 11.05 |
| 41.70 | 4.21 |
| 42.04 | 1.58 |
| 42.66 | 2.46 |
| 43.06 | 3.90 |
| 43.32 | 1.59 |
| 43.84 | 2.59 |
| 44.84 | 1.64 |
| 45.92 | 0.78 |
| 46.76 | 0.51 |
| 47.36 | 1.15 |
| 47.62 | 0.97 |
| 49.48 | 0.95 |

In the X-ray diffraction pattern of C₁₂H₁₆N₆O₂·C₄H₆O₄, the following intense peaks (maxima) were observed at diffraction angles 2θ (± 0.2°): 6.2, 16.9, 18.4, 18.6, 19.4, 20.4, 21.2, 26.3, 24.4, 25.6.

### Example 20. Synthesis of N,N'-bis[2-(1H-imidazol-4-yl)ethyl]oxalamide dimethanesulfonate dihydrate (molar ratio 1:2)

To a solution of 3.95 mL (0.06 mol) of methanesulfonic acid in 10 mL of water, 8.00 g (0.03 mol) of N,N'-bis[2-(1H-imidazol-4-yl)ethyl]oxalamide was added with stirring. The resulting suspension was stirred at 50 °C until a clear solution was obtained. The reaction mixture was cooled to +25°C and left until a precipitate formed. The formed precipitate was filtered, washed with water, and dried under vacuum to give a white crystalline powder.

Yield: 11.1 g (81.6%).

Retention time of the individual peak for C₁₂H₁₆N₆O₂: 4.0 min.

Quantitative determination of the base C₁₂H₁₆N₆O₂: 54.8% calculated, 55.3% determined by HPLC (internal normalization method), recalculated on an anhydrous basis.

Quantitative determination of the acid residue in the salt: 38.1% calculated, 38.5% determined by acid-base titration recalculated on an anhydrous basis.

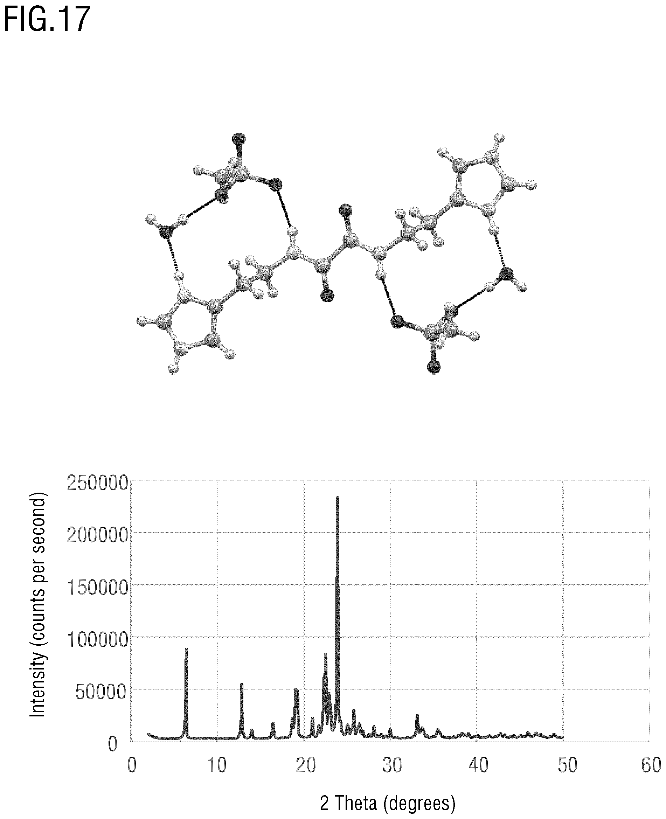

Next, an X-ray diffraction pattern of the crystalline powder was recorded. Table 19 lists the 2θ positions and their relative intensities. Figure 17 shows the powder X-ray diffraction pattern of the obtained crystalline form. Single-crystal analysis revealed that the compound crystallizes in a triclinic unit cell with the following parameters: space group P-1, Å: a = 5.8467(2) Å, b = 7.1877(4) Å, c = 14.0179(6) Å, α = 97.429(4)°, β = 98.303(3)°, γ = 102.482(4)°, with a cell volume of V = 561.23(4) Å³.

Based on the cell volume and symmetry, the crystal composition was determined as C₁₂H₁₆N₆O₂·2CH₄O₃S·2H₂O.

**Table 19. Intensity and 2θ positions of some peaks with relative intensities greater than 5% for the sample.**

| Position [°2θ] | Relative Intensity [%] |
|---|---|
| 6.4020 | 36.14 |
| 12.8018 | 23.10 |
| 12.9883 | 1.80 |
| 13.9876 | 3.75 |
| 16.4133 | 6.52 |
| 18.6580 | 7.13 |
| 19.0226 | 18.20 |
| 19.2655 | 15.98 |
| 20.9914 | 8.80 |
| 21.7182 | 4.09 |
| 22.3415 | 20.79 |
| 22.5095 | 33.26 |
| 22.9198 | 9.69 |
| 23.0887 | 10.82 |
| 23.8730 | 100.00 |
| 24.2264 | 4.88 |
| 25.0476 | 4.28 |
| 25.4946 | 2.31 |
| 25.7626 | 10.56 |
| 26.1968 | 3.12 |
| 26.4132 | 5.02 |
| 26.8261 | 2.29 |
| 27.5210 | 1.11 |
| 28.1155 | 4.55 |
| 28.5054 | 0.60 |
| 28.9695 | 1.46 |
| 29.4934 | 0.76 |
| 29.9887 | 3.73 |
| 30.2238 | 0.34 |
| 32.8445 | 0.64 |
| 33.1160 | 8.67 |
| 33.5021 | 1.55 |
| 33.7030 | 3.86 |
| 33.8771 | 1.79 |
| 34.2502 | 0.81 |
| 34.9475 | 0.41 |
| 35.5024 | 2.93 |
| 35.7295 | 2.05 |
| 36.0952 | 0.50 |
| 37.3715 | 0.44 |
| 37.8778 | 1.00 |
| 38.2550 | 1.72 |
| 38.4073 | 0.60 |
| 38.7004 | 1.09 |
| 39.0896 | 2.14 |
| 39.7376 | 0.53 |
| 40.1653 | 0.95 |
| 41.2344 | 0.36 |
| 41.4772 | 0.60 |
| 41.6417 | 0.45 |
| 42.1522 | 0.21 |
| 42.4834 | 0.78 |
| 42.7466 | 1. 68 |
| 43.2708 | 1.12 |
| 43.9040 | 0.35 |
| 44.3092 | 0.94 |
| 45.0674 | 0.69 |
| 45.9436 | 2.39 |
| 46.8756 | 1.82 |
| 47.4273 | 1.11 |
| 48.4176 | 0.32 |
| 48.8870 | 1.44 |
| 49.0938 | 1.18 |

In the X-ray diffraction pattern of C₁₂H₁₆N₆O₂·2CH₄O₃S·2H₂O, the following intense peaks (maxima) were observed at diffraction angles 2θ (± 0.2°): 6.4, 12.8, 19.0, 19.3, 22.3, 22.5, 22.9, 23.1, 23.9, 25.8, and 33.1. The most intense maxima were displayed at 6.4, 23.9, and 22.5.

### Example 21. Synthesis of N,N'-bis[2-(1H-imidazol-4-yl)ethyl]oxalamide di-D-malate (molar ratio 1:2)

To a solution of 0.198 g (0.72 mmol) of D-malic acid in 5 mL of water, 0.200 g (0.72 mmol) of N,N'-bis[2-(1H-imidazol-4-yl)ethyl]oxalamide was added with stirring. The resulting suspension was stirred at 90 °C until a clear solution was obtained. The reaction mixture was cooled to +25°C and left until a precipitate formed. The formed precipitate was filtered, washed with water, and dried under vacuum to give a white crystalline powder.

Yield: 0.39 g (98.0%).

Retention time of the individual peak for C₁₂H₁₆N₆O₂: 3.7 min.

Quantitative determination of the base C₁₂H₁₆N₆O₂: 50.7% calculated, 50.5% determined by HPLC (internal normalization method), recalculated on an anhydrous basis.

Quantitative determination of the acid residue in the salt: 49.3% calculated, 50.0% determined by acid-base titration, recalculated on an anhydrous basis.

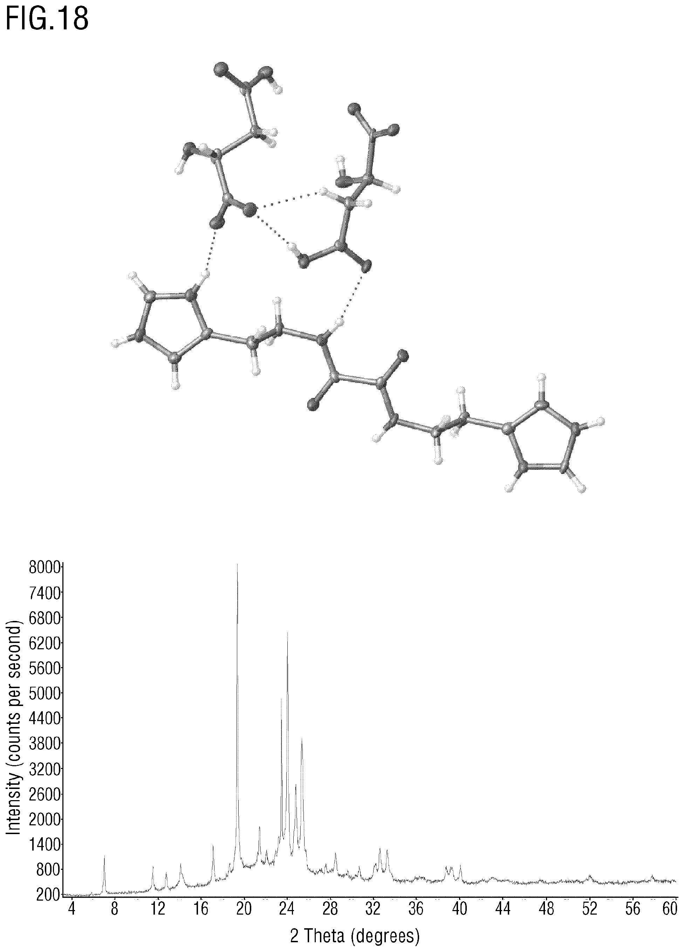

Next, an X-ray diffraction pattern of the crystalline powder was recorded. Table 20 lists the 2θ positions and their relative intensities. Figure 18 shows the powder X-ray diffraction pattern of the obtained crystalline form. Single-crystal analysis revealed that the compound crystallizes in a triclinic unit cell with the following parameters: space group P1, Å: a = 5.031(4) Å, b = 7.829(6) Å, c = 15.047(11) Å, α = 83.89(2)°, β = 84.35(2)°, γ = 78.31(2)°, with a cell volume of V = 575.2(8) Å³.

Based on the cell volume and symmetry, the crystal composition was determined as C₁₂H₁₆N₆O₂·2C₄H₆O₅.

**Table 20. Intensity and 2θ positions of some peaks with relative intensities greater than 5% for the sample.**

| Position [°2θ] | Relative Intensity [%] |
|---|---|
| 5.89 | 28.00 |
| 11.48 | 13.00 |
| 15.72 | 29.00 |
| 17.26 | 16.00 |
| 17.76 | 30.00 |
| 19.53 | 33.00 |
| 20.27 | 32.00 |
| 21.43 | 52.00 |
| 22.12 | 100.00 |
| 22.94 | 87.00 |
| 23.3 | 44.00 |
| 23.64 | 81.00 |
| 24.38 | 46.00 |
| 25.0 | 60.00 |
| 27.97 | 32.00 |
| 28.88 | 19.00 |
| 32.25 | 32.00 |
| 33.07 | 20.00 |
| 33.79 | 23.00 |
| 34.91 | 15.00 |
| 36.06 | 24.00 |
| 36.82 | 14.00 |
| 37.54 | 14.00 |
| 37.84 | 17.00 |
| 39.17 | 14.00 |
| 39.79 | 17.00 |
| 41.25 | 14.00 |
| 42.22 | 23.00 |

In the X-ray diffraction pattern of C₁₂H₁₆N₆O₂·2C₄H₆O₅, the following intense peaks (maxima) were observed at diffraction angles 2θ (± 0.2°): 5.9, 15.7, 19.5, 20.3, 21.4, 22.1, 22.9, 23.3, 23.6, 24.4, 27.8, and 32.3. The most intense maxima were displayed at 22.1, 22.9, and 23.6.

### Example 22. Synthesis of N,N'-bis[2-(1H-imidazol-4-yl)ethyl]oxalamide D-malate (molar ratio 1:1)

To a solution of 0.100 g (0.72 mmol) of D-malic acid in 5 mL of water, 0.200 g (0.72 mmol) of N,N'-bis[2-(1H-imidazol-4-yl)ethyl]oxalamide was added with stirring. The resulting suspension was stirred at 90°C until a solution was obtained. The reaction mixture was then cooled to +25°C and left until a precipitate formed. The formed precipitate was filtered, washed with water, and dried under vacuum to give a white crystalline powder.

Yield: 0.29 g (98.0%).

Retention time of the individual peak for C₁₂H₁₆N₆O: 3.7 min.

Quantitative determination of the base C₁₂H₁₆N₆O: 67.3% calculated, 67.2% determined by HPLC (internal normalization method), recalculated on an anhydrous basis.

Quantitative determination of the acid residue in the salt: 32.7% calculated, 32.5% determined by acid-base titration, recalculated on an anhydrous basis.

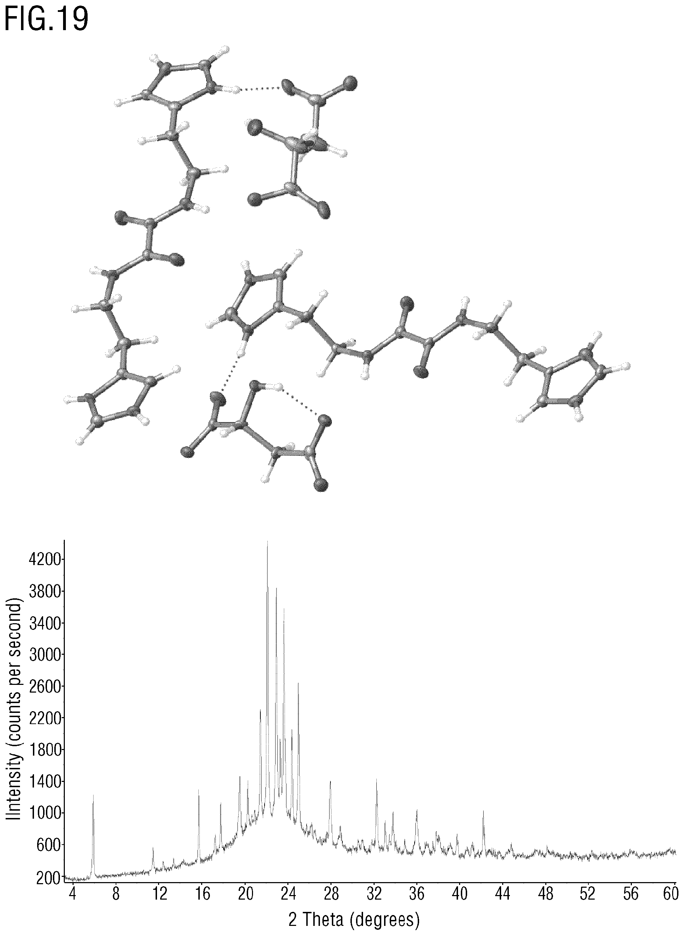

Next, an X-ray diffraction pattern of the crystalline powder was recorded. Table 21 lists the 2θ positions and their relative intensities. Figure 19 shows the powder X-ray diffraction pattern of the obtained crystalline form. Single-crystal analysis revealed that the compound crystallizes in a monoclinic unit cell with the following parameters: space group P2(1), Å: a = 9.5415(14) Å, b = 11.8563(16) Å, c = 16.0230(16) Å, α = 90°, β = 101.013(3)°, γ = 90°, with a cell volume of V = 1779.3(4) Å³.

Based on the cell volume and symmetry, the crystal composition was determined as C₁₂H₁₆N₆O·C₄H₆O₅.

**Table 21. Intensity and 2θ positions of some peaks with relative intensities greater than 5% for the sample.**

| Position [°2θ] | Relative Intensity [%] |
|---|---|
| 7.05 | 14.00 |
| 11.56 | 11.00 |
| 12.79 | 9.00 |
| 14.13 | 12.00 |
| 17.14 | 17.00 |
| 18.71 | 12.00 |
| 19.41 | 100.00 |
| 21.45 | 22.00 |
| 22.1 | 15.00 |
| 22.96 | 16.00 |
| 23.26 | 19.00 |
| 23.48 | 60.00 |
| 24.06 | 80.00 |
| 24.82 | 35.00 |
| 25.39 | 48.00 |
| 27.61 | 12.00 |
| 28.51 | 15.00 |
| 29.64 | 10.00 |
| 30.7 | 11.00 |
| 32.25 | 12.00 |
| 32.65 | 16.00 |
| 33.29 | 16.00 |
| 36.3 | 8.00 |
| 38.83 | 11.00 |
| 40.11 | 11.00 |

In the X-ray diffraction pattern of C₁₂H₁₆N₆O·C₄H₆O₅, the following intense peaks (maxima) were observed at diffraction angles 2θ (± 0.2°): 17.1, 19.4, 21.5, 22.1, 23.0, 23.3, 23.5, 24.1, 24.8, 25.4 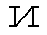 28.5. The most intense maxima were displayed at 22.1, 23.5, 24.1.

### Example 23. Synthesis of N,N'-bis[2-(1H-imidazol-4-yl)ethyl]oxalamide disulfate (molar ratio 1:2)

To a solution of 2.32 mL (0.04 mol) of sulfuric acid (96%) in 6.3 mL of water, 5.00 g (0.02 mol) of N,N'-bis[2-(1H-imidazol-4-yl)ethyl]oxalamide was added with stirring. The resulting suspension was stirred at 50°C until a solution was obtained. The reaction mixture was cooled to 20-25 °C, 33 mL of acetone was added, and the mixture was stirred for 2 hours. The precipitate was filtered, washed with acetone (2 × 10.0 mL), and dried under vacuum at 60°C to give a white crystalline powder.

Yield: 7.7 g (89.8%).

Retention time of the individual peak for C₁₂H₁₆N₆O: 4.1 min.

Quantitative determination of the base C₁₂H₁₆N₆O: 58.5% calculated, 58.6% determined by HPLC (internal normalization method), recalculated on an anhydrous basis.

Quantitative determination of the acid residue in the salt: 41.5% calculated, 41.6% determined by acid-base titration, recalculated on an anhydrous basis.

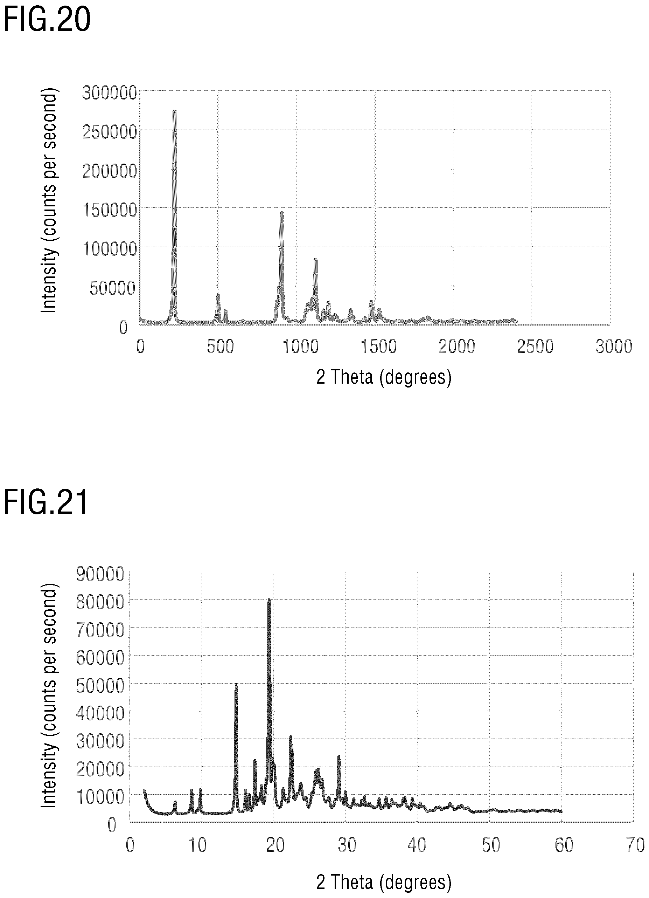

Next, an X-ray diffraction pattern of the crystalline powder was recorded. Table 22 lists the 2θ positions and their relative intensities. Figure 20 shows the powder X-ray diffraction pattern of the obtained crystalline form.

**Table 22. Intensity and 2θ positions of some peaks with relative intensities greater than 5% for the sample.**

| Position [°2θ] | Relative Intensity [%] |
|---|---|
| 6.4823 | 100 |
| 12.0252 | 17.51 |
| 12.968 | 5.65 |
| 19.5001 | 8.29 |
| 19.7958 | 20.62 |
| 20.0908 | 54.26 |
| 23.4242 | 8.92 |
| 23.5768 | 5.94 |
| 23.72 | 6.45 |
| 23.9866 | 10.97 |
| 24.4325 | 38.68 |
| 25.4515 | 5.22 |
| 26.096 | 6.21 |
| 28.8972 | 9.67 |
| 31.5232 | 11.65 |

In the X-ray diffraction pattern of C₁₂H₁₆N₆O·2H₂SO₄, the following intense peaks (maxima) were observed at diffraction angles 2θ (± 0.2°): 6.5, 12.0, 19.8, 20.1, 24.0, 24.4, and 31.5. The most intense maxima were displayed at 6.5, 20.1, and 24.4.

### Example 24. Synthesis of N,N'-bis[2-(1H-imidazol-4-yl)ethyl]oxalamide L-tartrate (molar ratio 1:1)

To a solution of 2.62 g (0.02 mol) of L-tartaric acid in 13.8 mL of water, 5.00 g (0.02 mol) of N,N'-bis[2-(1H-imidazol-4-yl)ethyl]oxalamide was added with stirring. The resulting suspension was stirred at 60 °C until a clear solution was obtained. The reaction mixture was cooled to 20-25°C, 33 mL of acetone was added, and the mixture was stirred for 2 hours. The precipitate was filtered, washed with acetone (2 × 15.0 mL), and dried under vacuum at 60°C to give a white crystalline powder.

Yield: 6.9 g (89.1%).

Retention time of the individual peak for C₁₂H₁₆N₆O: 3.7 min.

Quantitative determination of the base C₁₂H₁₆N₆O: 64.8% calculated, 63.6% determined by HPLC (internal normalization method), recalculated on an anhydrous basis.

Quantitative determination of the acid residue in the salt: 35.2% calculated, 34.6% determined by acid-base titration, recalculated on an anhydrous basis.

Next, an X-ray diffraction pattern of the crystalline powder was recorded. Table 23 lists the 2θ positions and their relative intensities. Figure 21 shows the powder X-ray diffraction pattern of the obtained crystalline form.

**Table 23. Intensity and 2θ positions of some peaks with relative intensities greater than 5% for the sample.**

| Position [°2θ] | Relative Intensity [%] |
|---|---|
| 6.4157 | 6.95 |
| 8.6591 | 9.01 |
| 9.8743 | 10.11 |
| 14.8252 | 45.83 |
| 17.4244 | 15.77 |
| 17.8019 | 13.38 |
| 18.9367 | 8.62 |
| 19.3727 | 100.00 |
| 20.0544 | 20.66 |
| 21.3212 | 9.98 |
| 22.3573 | 8.44 |
| 22.5311 | 29.39 |
| 23.7801 | 16.10 |
| 25.8339 | 18.85 |
| 26.1628 | 23.35 |
| 28.5848 | 6.65 |
| 29.0786 | 15.85 |
| 29.6355 | 5.03 |
| 31.5079 | 2.14 |
| 32.6116 | 3.22 |
| 32.9693 | 0.90 |
| 34.6641 | 3.34 |
| 35.6451 | 4.14 |
| 36.3994 | 3.24 |
| 38.2213 | 5.78 |
| 39.2088 | 8.66 |
| 40.3628 | 4.24 |
| 42.5381 | 1.09 |
| 43.9069 | 5.43 |
| 44.5138 | 3.07 |
| 45.6942 | 4.64 |
| 46.1111 | 2.80 |
| 47.0222 | 3.27 |
| 49.8374 | 1.18 |
| 50.6088 | 2.66 |
| 52.8314 | 1.85 |
| 58.0262 | 0.48 |
| 59.2772 | 1.19 |

In the X-ray diffraction pattern of C₁₂H₁₆N₆O₂·C₄H₆O₆, the following intense peaks (maxima) were observed at diffraction angles 2θ (± 0.2°): 9.9, 14.8, 17.4, 17.8, 19.4, 20.1, 22.5, 23.8, 25.8, 26.2, and 29.1. The most intense maxima were displayed at 19.4, 14.8, and 22.5.

### Example 25. Synthesis of N,N'-bis[2-(1H-imidazol-4-yl)ethyl]oxalamide D-tartrate (molar ratio 1:1)

To a solution of 2.73 g (0.02 mol) of D-tartaric acid in 15.8 mL of water, 5.00 g (0.02 mol) of N,N'-bis[2-(1H-imidazol-4-yl)ethyl]oxalamide was added with stirring. The resulting suspension was stirred at 50 °C until a clear solution was obtained. The reaction mixture was cooled to 20-25°C, 50 mL of acetone was added, and the mixture was stirred for 2 hours. The formed precipitate was filtered, washed with acetone (2 × 10.0 mL), and dried under vacuum at 60°C to give a white crystalline powder.

Yield: 6.3 g (81.2%).

Retention time of the individual peak for C₁₂H₁₆N₆O₂: 4.0 min.

Quantitative determination of the base C₁₂H₁₆N₆O₂: 64.8% calculated, 64.5% determined by HPLC (internal normalization method), recalculated on an anhydrous basis.

Quantitative determination of the acid residue in the salt: 35.2% calculated, 35.1% determined by acid-base titration, recalculated on an anhydrous basis.

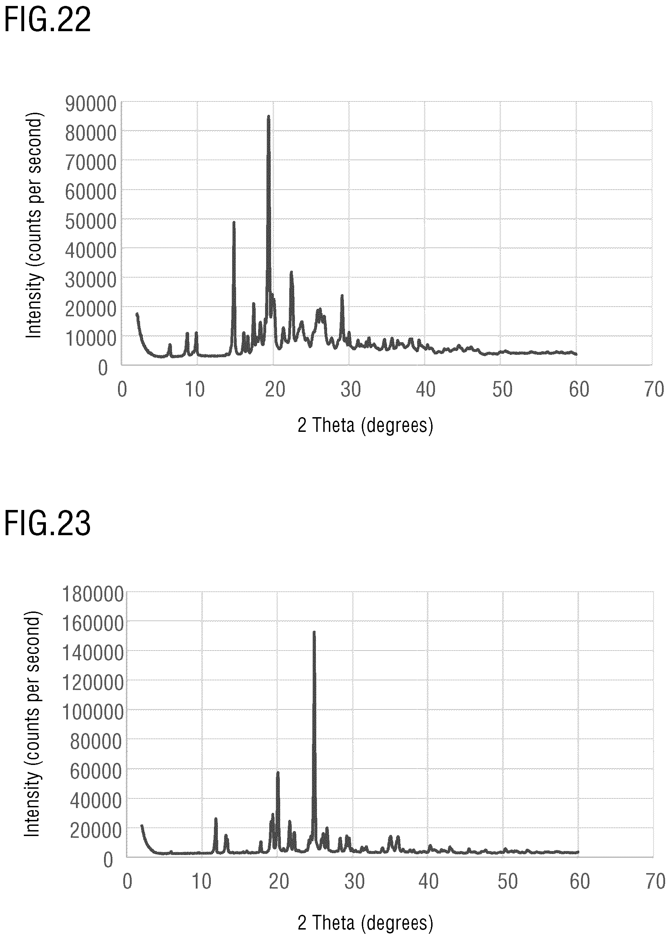

Next, an X-ray diffraction pattern of the crystalline powder was recorded. Table 24 lists the 2θ positions and their relative intensities. Figure 22 shows the powder X-ray diffraction pattern of the obtained crystalline form.

**Table 24. Intensity and 2θ positions of some peaks with relative intensities greater than 5% for the sample.**

| Position [°2θ] | Relative Intensity [%] |
|---|---|
| 8.6836 | 9.76 |
| 9.3797 | 1.01 |
| 14.8324 | 58.16 |
| 16.1398 | 9.64 |
| 17.4436 | 20.07 |
| 17.8035 | 5.06 |
| 18.3196 | 9.05 |
| 19.3877 | 100 |
| 19.8478 | 17.59 |
| 20.0848 | 19.72 |
| 21.2971 | 6.97 |
| 22.3642 | 14.3 |
| 23.4003 | 4.4 |
| 23.8165 | 5.36 |
| 25.8549 | 10.07 |
| 26.1932 | 13.62 |
| 26.7235 | 8.42 |
| 28.6449 | 3.34 |
| 29.1125 | 21.9 |
| 29.6682 | 3.39 |
| 31.1884 | 3.75 |
| 31.5198 | 1.7 |
| 32.2833 | 3.1 |
| 32.6218 | 5.2 |
| 32.9638 | 1.24 |
| 33.2384 | 2.01 |
| 35.6755 | 4.57 |
| 36.3942 | 4.06 |
| 36.7341 | 1.63 |
| 38.2337 | 4.55 |
| 40.3724 | 2.54 |
| 40.8034 | 1.74 |
| 43.8601 | 1.17 |
| 44.4944 | 2.14 |
| 44.8707 | 1.07 |
| 46.0933 | 1.86 |
| 47.0636 | 1.31 |
| 50.5767 | 1.24 |

In the X-ray diffraction pattern of C₁₂H₁₆N₆O₂·C₄H₆O₆, the following intense peaks (maxima) were observed at diffraction angles 2θ (± 0.2°): 8.7, 14.8, 17.4, 19.4, 19.9, 20.1, 22.4, 25.9, 26.2, and 29.1. The most intense maxima were displayed at 14.8, 19.4, and 29.1.

### Example 26. Synthesis of N,N'-bis[2-(1H-imidazol-4-yl)ethyl]oxalamide diglycolate (molar ratio 1:2)

To a solution of 2.75 g (0.04 mol) of glycolic acid in 15.8 mL of water, 5.00 g (0.02 mol) of N,N'-bis[2-(1H-imidazol-4-yl)ethyl]oxalamide was added with stirring. The resulting suspension was stirred at 70 °C until a clear solution was obtained. The reaction mixture was cooled to 20-25°C, 53 mL of acetone was added, and the mixture was stirred for 2 hours. The formed precipitate was filtered, washed with acetone (2 × 10.0 mL), and dried under vacuum at 60°C to give a white crystalline powder.

Yield: 5.7 g (73.5%).

Retention time of the individual peak for C₁₂H₁₆N₆O₂: 4.0 min.

Quantitative determination of the base C₁₂H₁₆N₆O₂: 64.5% calculated, 63.8% determined by HPLC (internal normalization method), recalculated on an anhydrous basis.

Quantitative determination of the acid residue in the salt: 35.5% calculated, 35.1% determined by acid-base titration, recalculated on an anhydrous basis.

Next, an X-ray diffraction pattern of the crystalline powder was recorded. Table 25 lists the 2θ positions and their relative intensities. Figure 23 shows the powder X-ray diffraction pattern of the obtained crystalline form.

**Table 25. Intensity and 2θ positions of some peaks with relative intensities greater than 5% for the sample.**

| Position [°2θ] | Relative Intensity [%] |
|---|---|
| 5.9002 | 5.41 |
| 9.5187 | 3.46 |
| 11.8491 | 19.83 |
| 13.1962 | 9.79 |
| 14.1124 | 0.75 |
| 14.6668 | 0.83 |
| 15.9624 | 2.22 |
| 16.6228 | 1.26 |
| 19.1792 | 22.01 |
| 19.4107 | 16.32 |
| 20.0851 | 52.41 |
| 21.6446 | 22.9 |
| 22.2642 | 11.27 |
| 23.8554 | 1.42 |
| 24.2374 | 5.14 |
| 24.4625 | 29.09 |
| 24.923 | 100 |
| 25.8867 | 13.07 |
| 26.1084 | 4.33 |
| 26.5992 | 12.19 |
| 28.3534 | 9.87 |
| 28.9723 | 2.71 |
| 29.2227 | 8.51 |
| 29.9227 | 2.02 |
| 34.9225 | 11.63 |
| 35.0952 | 5.45 |
| 35.3703 | 2.38 |
| 35.8389 | 6.46 |
| 36.0315 | 10.57 |
| 36.6791 | 1.98 |
| 37.6379 | 1.89 |
| 38.1121 | 1.71 |
| 40.3615 | 7.49 |
| 40.8591 | 2.21 |
| 42.1279 | 1.54 |
| 42.3958 | 0.54 |
| 43.1762 | 3.48 |
| 45.4971 | 2.58 |
| 46.2883 | 1.86 |
| 47.4038 | 2.74 |
| 50.3211 | 3.82 |
| 50.9177 | 0.78 |
| 53.2868 | 3.23 |
| 54.5205 | 0.87 |
| 55.6423 | 4.61 |

In the X-ray diffraction pattern of C₁₂H₁₆N₆O₂·2C₂H₄O₃, the following intense peaks (maxima) were observed at diffraction angles 2θ (± 0.2°): 11.9, 19.4, 20.0, 22.3, 24.5, 24.9, 25.9, 26.6, and 34.9. The most intense maxima were displayed at 20.0, 24.5 and 24.9.

### Example 27. Synthesis of N,N'-bis[2-(1H-imidazol-4-yl)ethyl]oxalamide glutarate dihydrate (molar ratio 1:1)

To a solution of 4.78 g (0.04 mol) of glutaric acid in 6.3 mL of water, 5.00 g (0.02 mol) of N,N'-bis[2-(1H-imidazol-4-yl)ethyl]oxalamide was added with stirring. The resulting suspension was stirred at 50 °C until a clear solution was obtained. The reaction mixture was cooled to 20-25°C, 33 mL of acetone was added, and the mixture was stirred for 2 hours. The precipitate was filtered, washed with acetone (2×10.0 mL), and dried under vacuum at 60°C to give a white crystalline powder.

Yield: 6.7 g (84.1%).

Retention time of the individual peak for C₁₂H₁₆N₆O₂: 3.9 min.

Quantitative determination of the base C₁₂H₁₆N₆O₂: 62.2% calculated, 63.1% determined by HPLC (internal normalization method), recalculated on an anhydrous basis.

Quantitative determination of the acid residue in the salt: 29.7% calculated, 30.1% determined by acid-base titration, recalculated on an anhydrous basis.

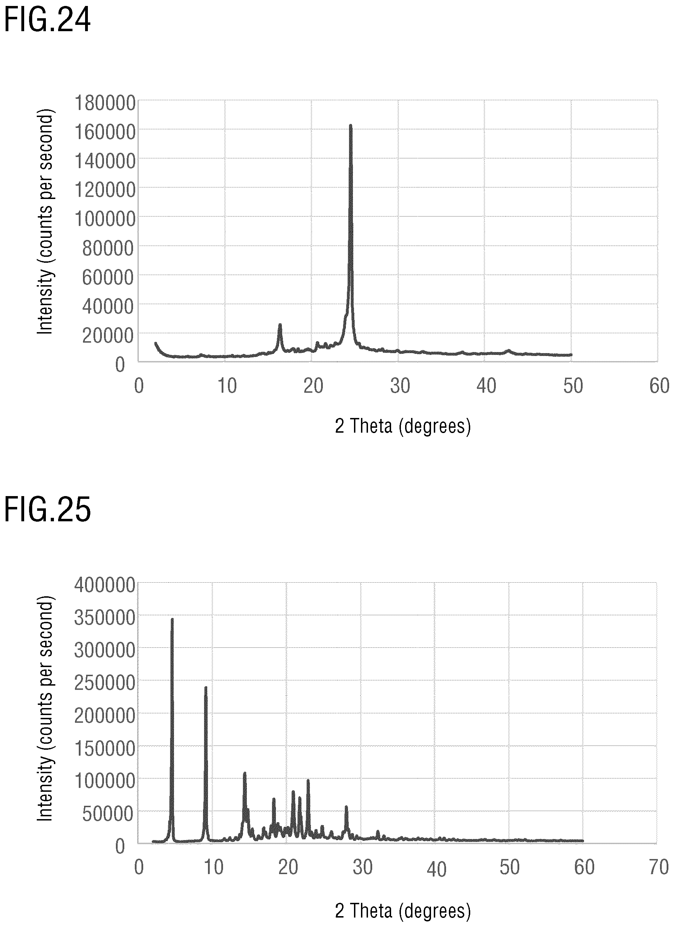

Table 26 lists the 2θ positions and their relative intensities. Figure 24 shows the powder X-ray diffraction pattern of the obtained crystalline form.

**Table 26. Intensity and 2θ positions of some peaks with relative intensities greater than 5% for the sample.**

| Position [°2θ] | Relative Intensity [%] |
|---|---|
| 7.2351 | 0.50 |
| 7.5407 | 0.23 |
| 10.4286 | 2.04 |
| 10.8609 | 0.78 |
| 11.4719 | 0.93 |
| 12.1906 | 0.65 |
| 13.9762 | 0.41 |
| 14.2556 | 0.35 |
| 14.5199 | 0.34 |
| 15.0372 | 0.62 |
| 16.1064 | 3.96 |
| 16.3774 | 16.78 |
| 17.2705 | 0.56 |
| 17.9596 | 3.58 |
| 18.5023 | 1.25 |
| 19.5120 | 4.17 |
| 19.8763 | 0.83 |
| 20.6622 | 5.50 |
| 21.6712 | 1.23 |
| 23.9227 | 29.16 |
| 24.5211 | 100.00 |
| 28.1712 | 1.16 |
| 28.9054 | 1.10 |
| 29.9242 | 1.27 |
| 30.8403 | 1.55 |
| 31.8632 | 0.31 |
| 32.8569 | 1.13 |
| 34.8868 | 1.27 |
| 37.4511 | 1.65 |
| 42.5355 | 0.74 |
| 42.8137 | 3.66 |
| 44.6455 | 1.01 |
| 46.1982 | 0.90 |
| 47.2295 | 0.18 |

In the X-ray diffraction pattern of C₁₂H₁₆N₆O₂·C₅H₈O₄·2H₂O, the following intense peaks (maxima) were observed at diffraction angles 2θ (±0.2°): 10.4, 16.4, 18.0, 19.5, 23.9, 24.5, and 42.8. The most intense maxima were displayed at 16.4, 23.9, and 24.5.

### Example 28. Synthesis of N,N'-bis[2-(1H-imidazol-4-yl)ethyl]oxalamide dicamphorsulfonate (molar ratio 1:2)

To a solution of 8.41 g (0.04 mol) of D,L-camphorsulfonic acid in 6.3 mL of water, 5.00 g (0.02 mol) of N,N'-bis[2-(1H-imidazol-4-yl)ethyl]oxalamide was added with stirring. The resulting suspension was stirred at 75°C until a clear solution was obtained. The reaction mixture was cooled to 20-25°C, 33 mL of acetone was added, and the mixture was stirred for 2 hours. The precipitate was filtered, washed with acetone (2×10.0 mL), and dried under vacuum at 60°C to give a white crystalline powder.

Yield: 12.1 g (90.1%).

Retention time of the individual peak for C₁₂H₁₆N₆O₂: 5.4 min.

Quantitative determination of the base C₁₂H₁₆N₆O₂: 37.3% calculated, 37.6% determined by HPLC (internal normalization method), recalculated on an anhydrous basis.

Quantitative determination of the acid residue in the salt: 62.7% calculated, 63.1% determined by acid-base titration, recalculated on an anhydrous basis.

Next, an X-ray diffraction pattern of the crystalline powder was recorded. Table 27 the 2θ positions and their relative intensities. Figure 25 shows the powder X-ray diffraction pattern of the obtained crystalline form.

**Table 27. Intensity and 2θ positions of some peaks with relative intensities greater than 5% for the sample.**

| Position [°2θ] | Relative Intensity [%] |
|---|---|
| 4.314 | 9.84 |
| 4.5505 | 100 |
| 9.1128 | 91.4 |
| 11.6631 | 4.86 |
| 12.3147 | 3.9 |
| 13.1096 | 2.14 |
| 14.0245 | 13.19 |
| 14.3337 | 42.09 |
| 14.7343 | 27.79 |
| 15.3807 | 12.48 |
| 16.2411 | 1. 94 |
| 16.9464 | 5.95 |
| 18.3059 | 18.99 |
| 18.8274 | 19.1 |
| 19.7949 | 3.77 |
| 20.9207 | 38.76 |
| 21.7936 | 21.48 |
| 21.9873 | 7.05 |
| 22.7253 | 6.21 |
| 22.924 | 28.54 |
| 23.4142 | 3.67 |
| 23.9926 | 5.17 |
| 26.6976 | 0.9 |
| 27.6015 | 10.7 |
| 28.0527 | 15.53 |
| 28.3466 | 5.57 |
| 28.8376 | 2.53 |
| 29.4651 | 1.74 |
| 31.0958 | 0.74 |
| 31.3536 | 1.15 |
| 32.3113 | 5.44 |
| 33.1296 | 2.34 |
| 35.1785 | 1.11 |
| 36.0767 | 1.62 |
| 38.9084 | 0.78 |
| 41.2919 | 1. 68 |
| 44.1915 | 0.12 |
| 45.4386 | 0.17 |
| 46.3413 | 0.6 |
| 46.8694 | 1.17 |
| 51.3327 | 0.92 |
| 51.7181 | 0.8 |
| 59.1845 | 0.15 |

In the X-ray diffraction pattern of C₁₂H₁₆N₆O₂·2C₁₀H₁₆O₄S, the following intense peaks (maxima) were observed at diffraction angles 2θ (±0.2°): 4.6, 9.1, 14.0, 14.3, 14.7, 15.4, 18.3, 18.3, 21.8, 22.9, and 28.1. The most intense maxima were displayed at 4.6, 9.1, and 14.3.

### Example 29. Synthesis of N,N'-bis[2-(1H-imidazol-4-yl)ethyl]oxalamide diformiate (molar ratio 1:2)

To a solution of 1.57 mL (0.04 mol) of formic acid in 6.3 mL of water, 5.00 g (0.02 mol) of N,N'-bis[2-(1H-imidazol-4-yl)ethyl]oxalamide was added with stirring. The resulting suspension was stirred at 50 °C until a clear solution was obtained. The reaction mixture was cooled to 20-25°C, 33 mL of acetone was added, and the mixture was stirred for 2 hours. The precipitate was filtered, washed with acetone (2×15.0 mL), and dried under vacuum at 60°C to give a white crystalline powder.

Yield: 5.0 g (75.0%).

Retention time of the individual peak for C₁₂H₁₆N₆O₂: 4.1 min.

Quantitative determination of the base C₁₂H₁₆N₆O₂: 75.0% calculated, 74.9% determined by HPLC (internal normalization method), recalculated on an anhydrous basis.

Quantitative determination of the acid residue in the salt: 25.0% calculated, 24.9% determined by acid-base titration, recalculated on an anhydrous basis.

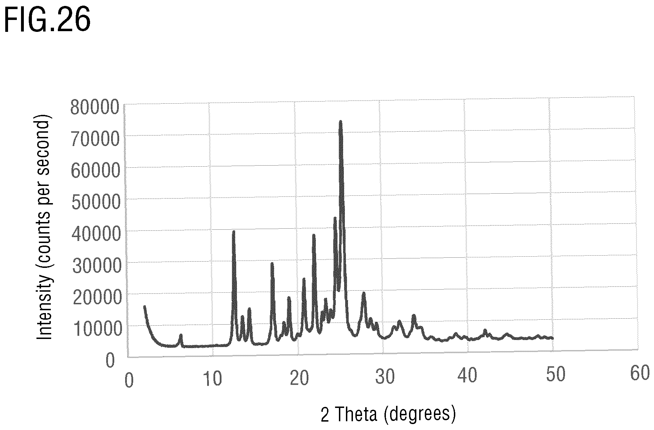

Next, an X-ray diffraction pattern of the crystalline powder was recorded. Table 28 lists the 2θ positions and their relative intensities. Figure 26 shows the powder X-ray diffraction pattern of the obtained crystalline form.

**Table 28. Intensity and 2θ positions of some peaks with relative intensities greater than 5% for the sample.**

| Position [°2θ] | Relative Intensity [%] |
|---|---|
| 6.3091 | 9.80 |
| 12.6477 | 35.17 |
| 13.5279 | 9.57 |
| 14.3277 | 14.15 |
| 17.1063 | 28.24 |
| 17.9528 | 6.65 |
| 18.4126 | 4.16 |
| 19.0406 | 12.19 |
| 20.7958 | 19.94 |
| 22.0155 | 30.71 |
| 22.8199 | 4.88 |
| 23.2930 | 11.76 |
| 23.9029 | 5.04 |
| 24.5797 | 33.77 |
| 25.4011 | 100.00 |
| 27.5925 | 8.86 |
| 27.8314 | 13.14 |
| 28.6239 | 8.81 |
| 29.2676 | 4.57 |
| 31.3399 | 5.87 |
| 31.9581 | 7.71 |
| 33.6935 | 5.26 |
| 34.4093 | 12.62 |
| 34.6018 | 1.40 |
| 35.7548 | 1.55 |
| 37.8484 | 1.37 |
| 38.5438 | 3.10 |
| 39.5707 | 1.37 |
| 41.5869 | 1.86 |
| 42.0391 | 2.70 |
| 42.5493 | 2.19 |
| 44.6064 | 2.38 |
| 45.0382 | 0.95 |
| 48.2785 | 0.96 |
| 49.0346 | 0.35 |

In the X-ray diffraction pattern of C₁₂H₁₆N₆O₂·2CH₂O₂, the following intense peaks (maxima) were observed at diffraction angles 2θ (±0.2°): 6.3, 12.7, 14.3, 17.1, 19.0, 20.8, 22.0, 23.3, 24.6, 25.4, 27.8, and 34.4. The most intense maxima were displayed at 12.7, 24.6, and 25.4.

### Example 30. Synthesis of N,N'-bis[2-(1H-imidazol-4-yl)ethyl]oxalamide diethanesulfonate (molar ratio 1:2)

To a solution of 3.98 g (0.06 mol) of ethanesulfonic acid in 15.8 mL of water, 7.00 g (0.03 mol) of N,N'-bis[2-(1H-imidazol-4-yl)ethyl]oxalamide was added with stirring. The resulting suspension was stirred at 60 °C until a clear solution was obtained. The solvent was removed under vacuum, and the residue was dissolved in 40 mL of isopropanol and left at 5°C for 12 hours. The formed precipitate was filtered, washed with isopropanol (2×10.0 mL), and dried under vacuum at 60°C to give a white crystalline powder.

Yield: 11.1 g (88.2%).

Retention time of the individual peak for C₁₂H₁₆N₆O₂: 4.0 min.

Quantitative determination of the base C₁₂H₁₆N₆O₂: 55.6% calculated, 56.0% determined by HPLC (internal normalization method), recalculated on an anhydrous basis.

Quantitative determination of the acid residue in the salt: 44.4% calculated, 44.6% determined by acid-base titration, recalculated on an anhydrous basis

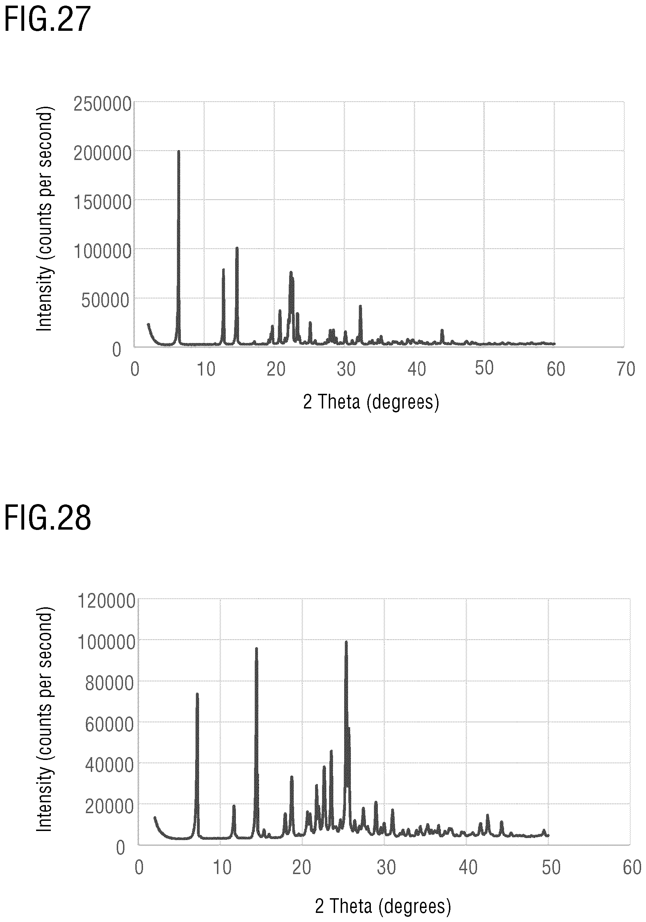

Next, an X-ray diffraction pattern of the crystalline powder was recorded. Table 29 lists the 2θ positions and their relative intensities. Figure 27 shows the powder X-ray diffraction pattern of the obtained crystalline form.

**Table 29. Intensity and 2θ positions of some peaks with relative intensities greater than 5% for the sample.**

| Position [°2θ] | Relative Intensity [%] |
|---|---|
| 6.3844 | 100 |
| 11.5831 | 7.44 |
| 12.7787 | 44.33 |
| 14.706 | 61.93 |
| 17.0043 | 0.89 |
| 17.2213 | 1.59 |
| 19.2283 | 3.67 |
| 19.4916 | 12.39 |
| 20.7941 | 26.91 |
| 21.5276 | 4.48 |
| 22.0189 | 16.49 |
| 22.2456 | 53.55 |
| 22.3921 | 19.77 |
| 22.6186 | 38.71 |
| 23.3016 | 21.01 |
| 23.5618 | 5.55 |
| 25.1369 | 16.03 |
| 25.8583 | 3.81 |
| 26.4255 | 3.58 |
| 27.2388 | 1.23 |
| 27.6331 | 3.92 |
| 28.4339 | 9.86 |
| 28.8216 | 5.32 |
| 29.4567 | 1.93 |
| 31.1388 | 3.12 |
| 31.939 | 5.78 |
| 32.3175 | 26.18 |
| 33.6133 | 2.28 |
| 34.0175 | 3.63 |
| 34.8383 | 4.34 |
| 35.2738 | 6.52 |
| 36.3045 | 1.32 |
| 37.2508 | 2.24 |
| 37.5034 | 1.65 |
| 37.8379 | 0.87 |
| 38.1754 | 2.01 |
| 39.8447 | 2.31 |
| 40.0641 | 1.24 |
| 40.7109 | 2.43 |
| 41.4234 | 0.93 |
| 42.9456 | 2.66 |
| 44.0007 | 8.35 |
| 44.1153 | 4.09 |
| 44.5505 | 0.49 |
| 45.4553 | 2.83 |
| 45.7451 | 1.78 |
| 51.5241 | 1.35 |
| 52.6398 | 2.92 |
| 53.3748 | 1.63 |
| 54.1818 | 0.87 |
| 56.6799 | 1.54 |

In the X-ray diffraction pattern of C₁₂H₁₆N₆O₂·2C₂H₆O₃S, the following intense peaks (maxima) were observed at diffraction angles 2θ (±0.2°): 6.4, 12.8, 14.7, 19.49, 20.8, 22.0, 22.3, 22.4, 22.6, 23.3, 25.1, and 32.3. The most intense maxima were displayed at 6.4, 14.7, and 22.3.

### Example 31. Synthesis of N,N'-bis[2-(1H-imidazol-4-yl)ethyl]oxalamide malonate dihydrate (molar ratio 1:1)

To a solution of 1.88 g (0.02 mol) of malonic acid in 50.0 mL of water, 5.00 g (0.02 mol) of N,N'-bis[2-(1H-imidazol-4-yl)ethyl]oxalamide was added with stirring. The resulting suspension was stirred at 92 °C until a clear solution was obtained. The reaction mixture was cooled to 20-25°C, 33 mL of acetone was added, and the mixture was stirred for 2 hours. The formed precipitate was filtered, washed with acetone (2×10.0 mL), and dried under vacuum at 60°C to give a white crystalline powder.

Yield: 11.1 g (88.2%).

Retention time of the individual peak for C₁₂H₁₆N₆O₂: 4.1 min.

Quantitative determination of the base C₁₂H₁₆N₆O₂: 66.4% calculated, 66.0% determined by HPLC (internal normalization method), recalculated on an anhydrous basis.

Quantitative determination of the acid residue in the salt: 25.0% calculated, 25.4% determined by acid-base titration, recalculated on an anhydrous basis.

The X-ray diffraction pattern of the crystalline powder was recorded. Table 30 lists the 2θ positions and their relative intensities. Figure 28 shows the powder X-ray diffraction pattern of the obtained crystalline form.

**Table 30. Intensity and 2θ positions of some peaks with relative intensities greater than 5% for the sample.**

| Position [°2θ] | Relative Intensity [%] |
|---|---|
| 7.2067 | 67.65 |
| 7.6259 | 1.94 |
| 11.6767 | 21.44 |
| 14.4403 | 84.74 |
| 15.3235 | 8.27 |
| 17.8937 | 15.19 |
| 18.6831 | 40.29 |
| 19.5622 | 1.10 |
| 20.6086 | 12.49 |
| 20.9413 | 14.07 |
| 21.2411 | 1.19 |
| 21.7352 | 20.90 |
| 21.9876 | 12.27 |
| 22.6503 | 43.70 |
| 23.5142 | 36.46 |
| 24.0873 | 4.24 |
| 25.3390 | 100.00 |
| 25.6628 | 51.76 |
| 26.3693 | 5.34 |
| 27.4436 | 11.91 |
| 27.6575 | 5.47 |
| 27.9362 | 2.81 |
| 28.9711 | 16.72 |
| 29.5745 | 3.01 |
| 29.9774 | 5.90 |
| 30.9411 | 14.35 |
| 31.8495 | 1.22 |
| 32.2197 | 2.83 |
| 32.8920 | 3.60 |
| 33.8905 | 3.09 |
| 34.3799 | 6.32 |
| 35.0602 | 1.40 |
| 35.2909 | 6.76 |
| 36.1086 | 4.46 |
| 37.3916 | 2.87 |
| 38.1818 | 4.91 |
| 39.4487 | 1.97 |
| 41.6586 | 7.59 |
| 41.8079 | 2.85 |
| 42.6175 | 14.31 |
| 43.1496 | 1.36 |
| 44.3131 | 10.35 |
| 45.4645 | 2.84 |
| 46.4658 | 1.12 |
| 47.9182 | 0.45 |
| 48.8234 | 0.85 |
| 49.4671 | 2.67 |

In the X-ray diffraction pattern of C₁₂H₁₆N₆O₂·C₃H₄O₄·2H₂O, the following intense peaks (maxima) were observed at diffraction angles 2θ (±0.2°): 11.68, 14.44, 17.89, 18.7, 20.6, 20.9, 21.7, 22.7, 23.5, 25.3, 25.7, 29.0, and 30.9. The most intense maxima were displayed at 7.2, 14.4, and 25.3.

### Example 32. Synthesis of N,N'-bis[2-(1H-imidazol-4-yl)ethyl]oxalamide di-L,D-mandelate (molar ratio 1:2)

To a solution of 5.51 g (0.04 mol) of L,D-mandelic acid in 15.8 mL of water, 5.00 g (0.02 mol) of N,N'-bis[2-(1H-imidazol-4-yl)ethyl]oxalamide was added with stirring. The resulting suspension was stirred at 50 °C until a clear solution was obtained. The solvent was then removed under vacuum, the residue was dissolved in a minimum amount of isopropanol, and the solution was kept at 5°C for 12 hours. The precipitate was filtered, washed with isopropanol (3×10.0 mL), and dried under vacuum at 60°C to give a white crystalline powder.

Yield: 8.94 g (85.0%).

Retention time of the individual peak for C₁₂H₁₆N₆O₂: 5.1 min.

Quantitative determination of the base C₁₂H₁₆N₆O₂: 47.6% calculated, 49.5% determined by HPLC (internal normalization method), recalculated on an anhydrous basis.

Quantitative determination of the acid residue in the salt: 52.5% calculated, 54.6% determined by acid-base titration, recalculated on an anhydrous basis.

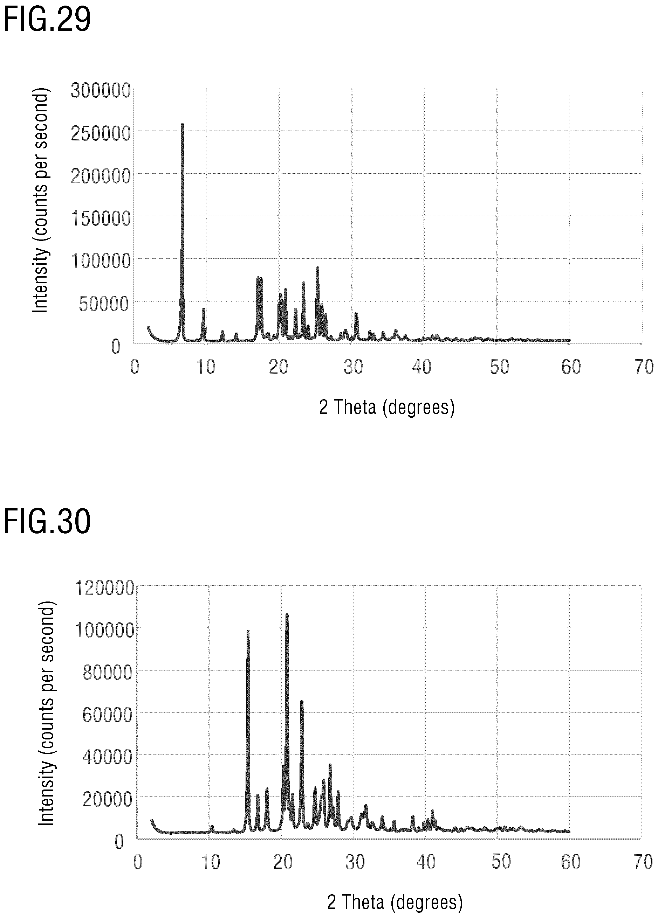

The X-ray diffraction pattern of the crystalline powder was recorded. Table 31 lists the 2θ positions and their relative intensities. Figure 29 shows the powder X-ray diffraction pattern of the obtained crystalline form.

**Table 31. Intensity and 2θ positions of some peaks with relative intensities greater than 5% for the sample.**

| Position [°2θ] | Relative Intensity [%] |
|---|---|
| 6.5235 | 15.39 |
| 6.7366 | 100 |
| 8.7287 | 0.59 |
| 9.617 | 18.94 |
| 12.2474 | 6.64 |
| 14.1495 | 4.84 |
| 14.7567 | 6.68 |
| 17.1113 | 35.05 |
| 17.4973 | 41.41 |
| 17.7509 | 1.64 |
| 18.2651 | 5.4 |
| 18.538 | 4.25 |
| 19.5066 | 1.05 |
| 20.0139 | 16.5 |
| 20.24 | 27.32 |
| 20.8598 | 34.82 |
| 21.6253 | 1.94 |
| 22.2726 | 18.72 |
| 22.8438 | 2.65 |
| 23.3464 | 31.63 |
| 24.0121 | 6.25 |
| 25.2866 | 39.54 |
| 25.4279 | 14.48 |
| 25.8926 | 15.2 |
| 26.3794 | 11.63 |
| 27.1466 | 1.71 |
| 28.5002 | 4.77 |
| 29.1017 | 8.78 |
| 29.3415 | 4.28 |
| 30.0135 | 1.46 |
| 30.6179 | 23.22 |
| 32.4806 | 4.96 |
| 33.0254 | 3.79 |
| 34.1424 | 0.98 |
| 34.3522 | 4.04 |
| 34.6436 | 0.71 |
| 35.0438 | 0.89 |
| 35.699 | 1.06 |
| 36.0267 | 9.02 |
| 36.3021 | 3.26 |
| 40.6784 | 0.71 |
| 41.1815 | 3.76 |
| 44.3972 | 1.77 |
| 45.1868 | 0.85 |
| 47.7659 | 2.55 |
| 49.4016 | 0.58 |
| 51.987 | 2.05 |
| 54.2298 | 0.77 |
| 55.8788 | 0.52 |
| 59.1237 | 0.51 |

In the X-ray diffraction pattern of C₁₂H₁₆N₆O₂·2C₈H₈O₃, the following intense peaks (maxima) were observed at diffraction angles 2θ (±0.2°): 6.5, 6.7, 9.6, 17.1, 17.5, 20.2, 20.9, 22.3, 23.4, 25.3, 25.4, 25.9, 26.4, and 30.6. The most intense maxima were displayed at 6.7, 17.1, and 26.7.

### Example 33. Synthesis of N,N'-bis[2-(1H-imidazol-4-yl)ethyl]oxalamide dinicotinate (molar ratio 1:2)

To a solution of 1.78 g (0.02 mol) of nicotinic acid in 6.3 mL of water, 2.00 g (0.01 mol) of N,N'-bis[2-(1H-imidazol-4-yl)ethyl]oxalamide was added with stirring. The resulting suspension was stirred at 80 °C until a clear solution was obtained. The solvent was removed under vacuum, the residue was dissolved in a minimum amount of isopropanol, and the solution was kept at 5°C for 12 hours. The formed precipitate was filtered, washed with isopropanol (2×4.0 mL), and dried under vacuum at 60°C to give a white crystalline powder.

Yield: 3.38 g (89.4%).

Retention time of the individual peak for C₁₂H₁₆N₆O₂: 4.0 min.

Quantitative determination of the base C₁₂H₁₆N₆O₂: 52.4% calculated, 47.1% determined by HPLC (internal normalization method), recalculated on an anhydrous basis.

Quantitative determination of the acid residue in the salt: 52.5% calculated, 47.4% determined by acid-base titration, recalculated on an anhydrous basis.

The X-ray diffraction pattern of the crystalline powder was recorded. Table 32 lists the 2θ positions and their relative intensities. Figure 30 shows the powder X-ray diffraction pattern of the obtained crystalline form.

**Table 32. Intensity and 2θ positions of some peaks with relative intensities greater than 5% for the sample.**

| Position [°2θ] | Relative Intensity [%] |
|---|---|
| 4.8322 | 25.9 |
| 10.4488 | 3.75 |
| 13.5133 | 6.93 |
| 15.4168 | 85.76 |
| 16.7575 | 22.11 |
| 18.0208 | 32.1 |
| 20.287 | 23.3 |
| 20.5236 | 2.07 |
| 20.7987 | 100 |
| 21.1404 | 10.46 |
| 21.5388 | 18.49 |
| 22.8639 | 67.01 |
| 23.6961 | 3.06 |
| 24.5996 | 15.21 |
| 24.7459 | 9.28 |
| 25.8884 | 19.44 |
| 26.7907 | 32.97 |
| 27.1733 | 12.08 |
| 27.8867 | 19.11 |
| 29.6974 | 15.32 |
| 31.0605 | 12.42 |
| 31.7388 | 18.35 |
| 32.5702 | 6.07 |
| 34.017 | 7.76 |
| 34.4237 | 1.91 |
| 35.6387 | 4.33 |
| 36.6413 | 1.47 |
| 38.2565 | 7.87 |
| 39.1157 | 1.14 |
| 40.3921 | 5.05 |
| 41.0371 | 9.32 |
| 41.7856 | 1.29 |
| 42.0485 | 1.31 |
| 44.1605 | 1.56 |
| 44.9961 | 0.96 |
| 45.7441 | 2.78 |
| 46.43 | 4.62 |
| 48.2695 | 2.21 |
| 49.8232 | 1.57 |
| 51.7564 | 0.85 |
| 54.7233 | 0.44 |
| 55.7284 | 2.87 |
| 57.7959 | 1.92 |

In the X-ray diffraction pattern of C₁₂H₁₆N₆O₂·2C₆H₅NO₂, the following intense peaks (maxima) were observed at diffraction angles 2θ (±0.2°): 4.8, 15.4, 18.0, 20.3, 20.8, 21.5, 22.9, 24.6, 25.9, 26.8, 27.1, 27.9, 29.7, 31.0, and 31.7. The most intense maxima were displayed at 15.4, 20.8, and 22.9.

### Example 34. Analysis of Pharmacopoeial properties of N,N'-bis[2-(1H-imidazol-4-yl)ethyl]oxalamide dihydrochloride dihydrate

A sample of the product from Example 2 was analyzed to verify compliance with the general requirements for a pharmaceutical substance (Ph. Eur. 2.4.14).

The following results were obtained:
- Foreign impurities (HPLC): Single unidentified impurity (not more than 0.1%) - 0.07%; Total impurities (not more than 0.5%) - 0.18%.
- Sulphated ash (not more than 0.1%) - 0.028% (Ph. Eur. 2.4.14).
- Heavy metals (not more than 0.001%) - compliant (Ph. Eur. 2.4.8 Method C).
- Residual organic solvents (HPLC): acetone (not more than 5000 ppm) - 190 ppm; methanol (not more than 3000 ppm) - not detected; isopropyl alcohol (not more than 5000 ppm) - not detected.
- Total aerobic microbial count (not more than 10³ CFU/g) - compliant (Ph. Eur. 2.6.12).
- Total yeast and mold count (not more than 10² CFU/g) - compliant (Ph. Eur. 2.6.12).
- Absence of *Escherichia coli* in 1 g - compliant (Ph. Eur. 2.6.13).

Chromatographic analysis was performed using a Gemini C18 column (4.6 × 250 mm, 5 µm) at a flow rate of 1.0 mL/min and a temperature of 25 °C.

### Example 35. Study of water solubility, stability in solid state and in solution, and biological activity of N,N'-bis[2-(1H-imidazol-4-yl)ethyl]oxalamide and salts thereof

**Table 33. Water solubility of N,N'-bis[2-(1H-imidazol-4-yl)ethyl]oxalamide and salts thereof**

| Compound Name | Water solubility (Ph.Eur. 5.11) |
|---|---|
| N,N'-bis[2-(1H-imidazol-4-yl)ethyl]oxalamide | Practically insoluble |
| N,N'-bis[2-(1H-imidazol-4-yl)ethyl]oxalamide dhydrochloride dihydrate (1) | Freely soluble |
| N,N'-bis[2-(1H-imidazol-4-yl)ethyl]oxalamide diphosphate (2) | Freely soluble |
| N,N'-bis[2-(1H-imidazol-4-yl)ethyl]oxalamide disulfate trihydrate (8) | Freely soluble |
| N,N'-bis[2-(1H-imidazol-4-yl)ethyl]oxalamide disulfate | Freely soluble |
| N,N'-bis[2-(1H-imidazol-4-yl)ethyl]oxalamide dimaleinate dihydrate (3) | Sparingly soluble |
| N,N'-bis[2-(1H-imidazol-4-yl)ethyl]oxalamide malonate dihydrate | Soluble |
| N,N'-bis[2-(1H-imidazol-4-yl)ethyl]oxalamide dimalonate (4) | Freely soluble |
| N,N'-bis[2-(1H-imidazol-4-yl)ethyl]oxalamide fumarate (5) | Sparingly soluble |
| N,N'-bis[2-(1H-imidazol-4-yl)ethyl]oxalamide dihydroiodide | Freely soluble |
| N,N'-bis[2-(1H-imidazol-4-yl)ethyl]oxalamide dihydroiodide dihydrate (6) | Freely soluble |
| N,N'-bis[2-(1H-imidazol-4-yl)ethyl]oxalamide dihydrobromide | Freely soluble |
| N,N'-bis[2-(1H-imidazol-4-yl)ethyl]oxalamide dihydrobromide dihydrate (7) | Freely soluble |
| N,N'-bis[2-(1H-imidazol-4-yl)ethyl]oxalamide dibenzenesulfonate (9) | Freely soluble |
| N,N'-bis[2-(1H-imidazol-4-yl)ethyl]oxalamide tartrate (10) | Freely soluble |
| N,N'-bis[2-(1H-imidazol-4-yl)ethyl]oxalamide malate (11) | Freely soluble |
| N,N'-bis[2-(1H-imidazol-4-yl)ethyl]oxalamide di-p-toluenesulfonate (12) | Soluble |
| N,N'-bis[2-(1H-imidazol-4-yl)ethyl]oxalamide oxalate (13) | Soluble |
| N,N'-bis[2-(1H-imidazol-4-yl)ethyl]oxalamide dioxalate (14) | Soluble |
| N,N'-bis[2-(1H-imidazol-4-yl)ethyl]oxalamide disulfate | Freely soluble |
| N,N'-bis[2-(1H-imidazol-4-yl)ethyl]oxalamide tartrate (starting L-tartaric acid) | Freely soluble |
| N,N'-bis[2-(1H-imidazol-4-yl)ethyl]oxalamide tartrate (starting D-tartaric acid) | Freely soluble |
| N,N'-bis[2-(1H-imidazol-4-yl)ethyl]oxalamide diglycolate | Freely soluble |
| N,N'-bis[2-(1H-imidazol-4-yl)ethyl]oxalamide glutarate dihydrate | Freely soluble |
| N,N'-bis[2-(1H-imidazol-4-yl)ethyl]oxalamide dicamphorsulfonate | Freely soluble |
| N,N'-bis[2-(1H-imidazol-4-yl)ethyl]oxalamide dimethanesulfonate | Freely soluble |
| N,N'-bis[2-(1H-imidazol-4-yl)ethyl]oxalamide diformiate | Freely soluble |
| N,N'-bis[2-(1H-imidazol-4-yl)ethyl]oxalamide diethanesulfonate | Freely soluble |
| N,N'N,N'-bis[2-(1H-imidazol-4-yl)ethyl]oxalamide malonate dihydrate | Soluble |
| N,N'-bis[2-(1H-imidazol-4-yl)ethyl]oxalamide dimandelate | Soluble |
| N,N'-bis[2-(1H-imidazol-4-yl)ethyl]oxalamide dinicotinate | Soluble |
| N,N'-bis[2-(1H-imidazol-4-yl)ethyl]oxalamide succinate | Soluble |

### Stability of the salt forms of N,N'-bis[2-(1H-imidazol-4-yl)ethyl]oxalamide in the solid state and in solution

For a preliminary assessment of the stability of the salt forms of N,N'-bis[2-(1H-imidazol-4-yl)ethyl]oxalamide, isothermal stress testing was conducted at 75°C in a sealed container for 21 days. This isothermal stress testing confirmed the stability of the salts of formula (I) during storage. In particular, the dihydrate of the hydrochloric acid salt of N,N'-bis[2-(1H-imidazol-4-yl)ethyl]oxalamide demonstrated stability, with a total impurity content less than 1% and an individual impurity content of less than 0.15%).

Further, the stability of the different salts of of N,N'-bis[2-(1H-imidazol-4-yl)ethyl]oxalamide was studied under long-term storage conditions. The results of the study demonstrated that the salts are stable: single unidentified impurity content is not more than 0.15%.

Thus, storage-stable salts were obtained in the dry form, suitable for the production of stable liquid formulations as well as solid formulations with the necessary and acceptable pharmacokinetic characteristics.

Stability data were also obtained for a 1% (10 mg/mL) solutions of certain salts of N,N'-bis[2-(1H-imidazol-4-yl)ethyl]oxalamide at temperature +25 °C and 60% humidity, with the results presented in Table 34.

**Table 34. Stability Data for 1% Solutions.**

| Acid name | Molar Ratio of Free Base to Acid | Stability Study Time Point (months) | Foreign impurities (HPLC) | |
|---|---|---|---|---|
| | | | Single unidentified impurity, (%) | Total impurities (%) |
| Free base | Not possible to prepare a 1% solution* | | | |
| Hydrochloric acid | 1:2 | 0 | <0.05 | <0.05 |
| | | 3 | <0.05 | <0.05 |
| | | 6 | <0.05 | <0.05 |
| | | 12 | <0.05 | <0.05 |
| | | 18 | <0.05 | <0.05 |
| | | 24 | <0.05 | <0.05 |
| Benzenesulfonic Acid | 1:2 | 0 | 0.062 | 0.115 |
| | | 6 | 0.111 | 0.111 |
| p-Toluenesulfonic Acid | 1:2 | 0 | 0.051 | 0.051 |
| | | 6 | 0.097 | 0.143 |
| Oxalic Acid | 1:2 | 0 | 0.089 | 0.139 |
| | | 6 | 0.325 | 0.435 |
| Sulfuric Acid | 1:2 | 0 | 0.099 | 0.099 |
| | | 6 | 0.060 | 0.060 |
| Glycolic Acid | 1:2 | 0 | 0.069 | 0.069 |
| | | 6 | 0.128 | 0.128 |
| D,L-Camphorsulfonic Acid | 1:2 | 0 | 0.062 | 0.062 |
| | | 6 | 0.053 | 0.053 |
| Methanesulfonic Acid | 1:2 | 0 | 0.127 | 0.182 |
| | | 6 | 0.214 | 0.293 |
| Formic acid | 1:2 | 0 | 0.125 | 0.169 |
| | | 6 | 0.335 | 0.465 |
| Ethanesulfonic Acid | 1:2 | 0 | 0.051 | 0.057 |
| | | 6 | 0.171 | 0.171 |
| D,L-Mandelic Acid | 1:2 | 0 | 0.057 | 0.057 |
| | | 6 | 0.129 | 0.129 |
| Nicotinic Acid | 1:2 | 0 | 0.076 | 0.076 |
| | | 6 | 0.199 | 0.199 |
| D,L-tartaric acid | 1:1 | 0 | 0.067 | 0.067 |
| | | 6 | 0.285 | 0.420 |
| L-tartaric acid | (1:1) | 0 | 0.131 | 0.182 |
| | | 6 | 0.284 | 0.284 |
| D-tartaric acid | (1:1) | 0 | 0.057 | 0.057 |
| | | 6 | 0.386 | 0.468 |
| Glutaric acid | (1:1) | 0 | 0.122 | 0.186 |
| | | 6 | 0.273 | 0.415 |
| Succinic acid | (1:1) | 0 | 0.118 | 0.186 |
| | | 6 | 0.397 | 0.534 |

| | | | | |
|---|---|---|---|---|
| * The free base is practically insoluble in water, with a solubility of 0.32 g/L. | | | | |

Further studies unexpectedly revealed that salts of the general formula (I) exhibit high water solubility and crystallinity. Moreover, it was unexpectedly found that water solutions of N,N'-bis[2-(1H-imidazol-4-yl)ethyl]oxalamide dihydrochloride dihydrate and other salts of formula (I) possess high stability during storage at +25 °C and 60% humidity.

### Characterization of the biological activity of N,N'-bis[2-(1H-imidazol-4-yl)ethyl]oxalamide and salts thereof

The biological activity of N,N'-bis[2-(1H-imidazol-4-yl)ethyl]oxalamide, as well as N,N'-bis[2-(1H-imidazol-4-yl)ethyl]oxalamide dihydrochloride dihydrate, was studied in various *in vitro* and *in vivo* experiments.

### Biological Example 1. Study of the pharmacokinetics of N,N'-bis[2-(1H-imidazol-4-yl)ethyl]oxalamide after a single oral administration in Sprague-Dawley rats

The pharmacokinetics of N,N'-bis[2-(1H-imidazol-4-yl)ethyl]oxalamide in the blood plasma of Sprague-Dawley rats after a single intragastric administration of 13 mg/kg of the free base was studied in three independent experiments. In all studies, the concentration of N,N'-bis[2-(1H-imidazol-4-yl)ethyl]oxalamide were determined using high-performance liquid chromatography with tandem mass spectrometric detection (HPLC-MS/MS).

It was found that the free base exhibits high intra-group variability of key pharmacokinetic parameters (Table 35). In the table, the mean values for each time point were determined based on individual data obtained from six animals.

**Table 35. Key pharmacokinetic parameters of N,N'-bis[2-(1H-imidazol-4-yl)ethyl]oxalamide in the blood plasma of Sprague-Dawley rats after oral administration of the free base at a dose of 13 mg/kg.**

| Experiment | T_{1/2} | Tₘₐₓ | Cₘₐₓ | AUC₀₋₈ |
|---|---|---|---|---|
| No. | (h) | (h) | (ng/mL) | (ng ·h/mL) |
| 1 | 1.9 | 2.0 | 139 [84-271] | 381 [232-633] |
| 2 | 5.2 | 2.0 | 319 [230-425] | 1180 [895-1466] |
| 3 | 6.3 | 4.0 | 443 [305-773] | 1499 [1064-2327] |

After a single administration of the free base at a dose of 13 mg/kg to Sprague-Dawley rats, the peak plasma concentration of the compound ranged from 84 to 773 ng/mL, corresponding to 0.25-2.25 µmol/L, while the exposure of N,N'-bis[2-(1H-imidazol-4-yl)ethyl]oxalamide ranged from 900 to 8950 nmol ·h. In other words, the oral administration of the same dose of the free base to two different experimental animals resulted in a tenfold difference in both peak concentrations and exposures of N,N'-bis[2-(1H-imidazol-4-yl)ethyl]oxalamide.

It is important to note that this tenfold difference in the maximum and mean daily concentrations of N,N'-bis[2-(1H-imidazol-4-yl)ethyl]oxalamide makes the selection of an effective and safe dose of the free base extremely challenging.

Similar results were obtained in two independent studies of the distribution of N,N'-bis[2-(1H-imidazol-4-yl)ethyl]oxalamide in the organs and tissues of experimental animals following a single oral administration of the free base (Table 36).

In the table, the mean values for each time point were determined based on individual data obtained from six animals.

**Table 36. Key pharmacokinetic parameters of N,N'-bis[2-(1H-imidazol-4-yl)ethyl]oxalamide in the lungs and kidneys of Sprague-Dawley rats after oral administration of the free base at a dose of 13 mg/kg.**

| Experiment | T_{1/2} | Tₘₐₓ | Cₘₐₓ | AUC₀₋₈ |
|---|---|---|---|---|
| No. | (h) | (h) | (ng/mL) | (ng ·h/mL) |
| 2 (Lung) | 1.9 | 2.0 | 139 [84-271] | 381 [232-633] |
| 3 (Lung) | 5.2 | 2.0 | 187 [116-233] | 913 [741-1037] |
| 2 (Kidney) | 5.0 | 2.0 | 585 [325-1140] | 2020 [1266-2745] |
| 3 (Kidney) | 3.3 | 2.7 | 4030 [2096-7352] | 12761 [7868-17310] |

The therapeutic use of a drug with high variability in pharmacokinetic parameters requires individualized dose selection, which precludes the use of the free base of N,N'-bis[2-(1H-imidazol-4-yl)ethyl]oxalamide for the treatment of fibrosis.

Thus, the pharmacokinetic characteristics of N,N'-bis[2-(1H-imidazol-4-yl)ethyl]oxalamide prevent its clinical use for the treatment of life-threatening diseases such as pulmonary fibrosis.

### Biological Example 2. Pharmacokinetic study after a single oral administration of N,N'-bis[2-(1H-imidazol-4-yl)ethyl]oxalamide dihydrochloride dihydrate in Sprague-Dawley rats

In the course of the pharmacokinetic study of N,N'-bis[2-(1H-imidazol-4-yl)ethyl]oxalamide after the oral administration of its dihydrochloride dihydrate in Sprague-Dawley rats, it was unexpectedly found that this salt form exhibits superior pharmacokinetic parameters compared to the free base. Table 37 presents the results of the pharmacokinetic study of N,N'-bis[2-(1H-imidazol-4-yl)ethyl]oxalamide dihydrochloride dihydrate after a single oral administration at a dose of 18 mg/kg (equivalent to 13 mg/kg in terms of the free base). For each time point, the mean values were determined based on individual data obtained from six animals.

The peak plasma concentration of the compound in Sprague-Dawley rats ranged from 165 to 282 ng/mL, corresponding to approximately 1 µmol/L, while the exposure after a single administration was in the range of 2500 to 3500 nmol·h.

**Table 37. Key pharmacokinetic parameters after administration of N,N'-bis[2-(1H-imidazol-4-yl)ethyl]oxalamide dihydrochloride dihydrate at a dose of 18 mg/kg (13 mg/kg in terms of the Free Base) in Sprague-Dawley rats.**

| Experiment | T_{1/2} | Tₘₐₓ | Cₘₐₓ | AUC₀₋₈ |
|---|---|---|---|---|
| No. | (h) | (h) | (ng/mL) | (ng ·h/mL) |
| 1 (blood plasma) | 5.0 | 2.0 | 213 [165-272] | 894 [666-1123] |
| 2 (blood plasma) | 4.8 | 2.0 | 201 [101-282] | 663 [503-974] |

As can be seen from the data presented, the use of N,N'-bis[2-(1H-imidazol-4-yl)ethyl]oxalamide dihydrochloride dihydrate is characterized by significantly lower variability in pharmacokinetic parameters. In particular, the difference in the mean Cₘₐₓ values after administration of the dihydrochloride dihydrate is less than 50%, whereas for the free base, the difference in peak concentrations among individual animals is approximately tenfold (i.e., 1000%).

A similar situation trend is observed in exposure comparisons. After administration of the free base, mean exposure values (Table 37, column AUC₀₋₈) differ by more than one order of magnitude, whereas after administration of N,N'-bis[2-(1H-imidazol-4-yl)ethyl]oxalamide dihydrochloride dihydrate, the difference in exposure is approximately 30%.

### Biological Example 3. Tissue Bioavailability

Studies of tissue bioavailability after oral administration of N,N'-bis[2-(1H-imidazol-4-yl)ethyl]oxalamide dihydrochloride dihydrate have shown that administration of the salt form leads to preferential distribution of the drug into the target organs of fibrosis (lungs, liver, kidneys, pancreas, and heart) (see Table 39 and 40).

Moreover, a comparison of the pharmacokinetics of the salt form and the free base shows that the use of N,N'-bis[2-(1H-imidazol-4-yl)ethyl]oxalamide dihydrochloride dihydrate is not only associated with significantly lower variability in pharmacokinetic parameters but also ensures higher drug concentrations in the lungs compared to the free base (see Table 38). For example, peak concentrations and exposure levels of N,N'-bis[2-(1H-imidazol-4-yl)ethyl]oxalamide in the lungs of experimental animals after administration of the dihydrochloride dihydrate were three times higher than the concentrations produced by an equivalent dose of the free base.

**Table 38. Comparison of the pharmacokinetic characteristics of N,N'-bis[2-(1H-imidazol-4-yl)ethyl]oxalamide after administration of equivalent doses of its dihydrochloride dihydrate or the free base in Sprague-Dawley rats**

| Experiment | T_{1/2} | Tₘₐₓ | Cₘₐₓ | AUC₀₋₈ |
|---|---|---|---|---|
| No. | (h) | (h) | (ng/mL) | (ng ·h/mL) |
| Free base (blood plasma) | 5.0 | 2.0 | 443 [305-773] | 1499 [1064-2327] |
| Salt form (blood plasma) | 5.0 | 2.0 | 213 [165-272] | 894 [666-1123] |
| Free base (lung) | 5.5 | 3.0 | 1163 [743-1633] | 4662 [3404-5625] |
| Salt form (lung) | 4.8 | 3.0 | **3528** [2484-4571] | **14333** [12244-16278] |
| Free base (pancreas) | 3.9 | 4.0 | 915 [719-1140] | 4440 [3780-5235] |
| Salt form (pancreas) | 4.0 | 2.0 | 1294 [635-1990] | 4129 [3122-4799] |

Thus, based on the pharmacokinetic studies of N,N'-bis[2-(1H-imidazol-4-yl)ethyl]oxalamide and its dihydrochloride dihydrate, it was unexpectedly found that administration of the salt form is characterized by preferential distribution to the lungs (tissue bioavailability, defined as the exposure in the organ divided by the exposure in plasma, is approximately 25 for the salt form and about 7 for the free base).In addition, it was unexpectedly found that N,N'-bis[2-(1H-imidazol-4-yl)ethyl]oxalamide dihydrochloride dihydrate is characterized by low variability in key pharmacokinetic parameters compared to the free base. It should be noted that the differences in tissue availability parameters between the free base and the salt form of the drug cannot be predicted based on the known pharmacokinetic and physicochemical properties of the free base.

Due to the potential use of N,N'-bis[2-(1H-imidazol-4-yl)ethyl]oxalamide dihydrochloride dihydrate in the treatment of fibrosis of the liver, kidney, pancreas, and heart, its distribution was further analyzed after oral administration at dose of 18 mg/kg in the liver, kidneys, pancreas, and heart. The tissue factors of compound 1 for the lungs, pancreas, heart, and kidneys exceeded the tissue factor for the free base (Table 39).

**Table 39. Analysis of the distribution of the free base N,N'-bis[2-(1H-imidazol-4-yl)ethyl]oxalamide and N,N'-bis[2-(1H-imidazol-4-yl)ethyl]oxalamide dihydrochloride dihydrate in the lungs, liver, kidneys, pancreas, and heart after oral administration at a dose of 18 mg/kg to Sprague-Dawley rats**

| | Tₘₐₓ | Cₘₐₓ | T_{1/2} | AUCₜ, | AUC_{INF} | MRT_{INF} | MRTₜ | AUCₜ tissue /AUCₜplasma |
|---|---|---|---|---|---|---|---|---|
| | h | ng/mL | h | ng·h/mL | ng·h/mL | h | h | |
| Free base N,N'-bis [2-(1H-imidazol-4-yl) ethyl ] oxalamide | | | | | | | | |
| Plasma | 2.0 | 319 | 5.2 | 1180 | 1220 | 5.5 | 4.7 | |
| Lungs | 4.0 | 816 | 3.7 | 8062 | 8103 | 5.9 | 5.8 | 6.8 |
| Kidneys | 1.0 | 2612 | 4.1 | 14007 | 14182 | 4.8 | 4.5 | 11.9 |
| Pancreas | 4.0 | 791 | 3.9 | 4480 | 6691 | 7,2 | 4.1 | 3.8 |
| Heart | 4.0 | 1879 | 2.1 | 10061 | 11611 | 4.6 | 3.6 | 8.5 |

| N,N'-bis[2-(1H-imidazol-4-yl)ethyl]oxalamide dihydrochloride dihydrate | | | | | | | | |
|---|---|---|---|---|---|---|---|---|
| Plasma | 2.0 | 194 | 4.8 | 758 | 783 | 5.1 | 4.2 | |
| Lungs | 4.0 | 3033 | 3.9 | 19256 | 195444 | 6.0 | 5.6 | 25.4 |
| Kidneys | 2.0 | 7470 | 3.3 | 372335 | 37566 | 4.8 | 4.6 | 49.1 |
| Pancreas | 2.0 | 885 | 4.0 | 5181 | 5290 | 5.3 | 4.8 | 6.8 |
| Heart | 2.0 | 1777 | 4.5 | 9365 | 9488 | 4.9 | 4.6 | 12.4 |

As part of the *in vivo* studies, the antifibrotic activity of N,N'-bis[2-(1H-imidazol-4-yl)ethyl]oxalamide dihydrochloride dihydrate was evaluated under both therapeutic administration (after the development of fibrosis, with treatment initiation on day 14 of the study) and prophylactic administration (before the development of fibrosis, with treatment initiation on day 0 of the study).

### Biological Example 4. Study of the effect of N,N'-bis[2-(1H-imidazol-4-yl)ethyl]oxalamide dihydrochloride dihydrate on the development of bleomycin-induced pulmonary fibrosis in comparison with the Pirfenidone, therapeutic treatment regimen

In this experiment (therapeutic treatment regimen), pulmonary fibrosis was induced by a single intratracheal administration of the cytostatic bleomycin, dissolved in PBS, at a dose of 16 units/kg in a volume of 200 µL. The therapeutic treatment regimen involved the administration of N,N'-bis[2-(1H-imidazol-4-yl)ethyl]oxalamide dihydrochloride dihydrate from day 14 to day 55 after the induction of pulmonary fibrosis in male Agouti guinea pigs, aged 4-5 weeks and weighing 180-200 grams. The test compound was administered intragastrically at doses of 0.9 and 3.5 mg/kg once daily. The administration volume was 10 mL/kg of body weight. Control animals received the solvent (distilled water) in an equivalent volume. As a comparator, a suspension of Pirfenidone (64 mg/kg) was used, which was administered intragastrically, daily, three times a day, from day 14 to day 55.

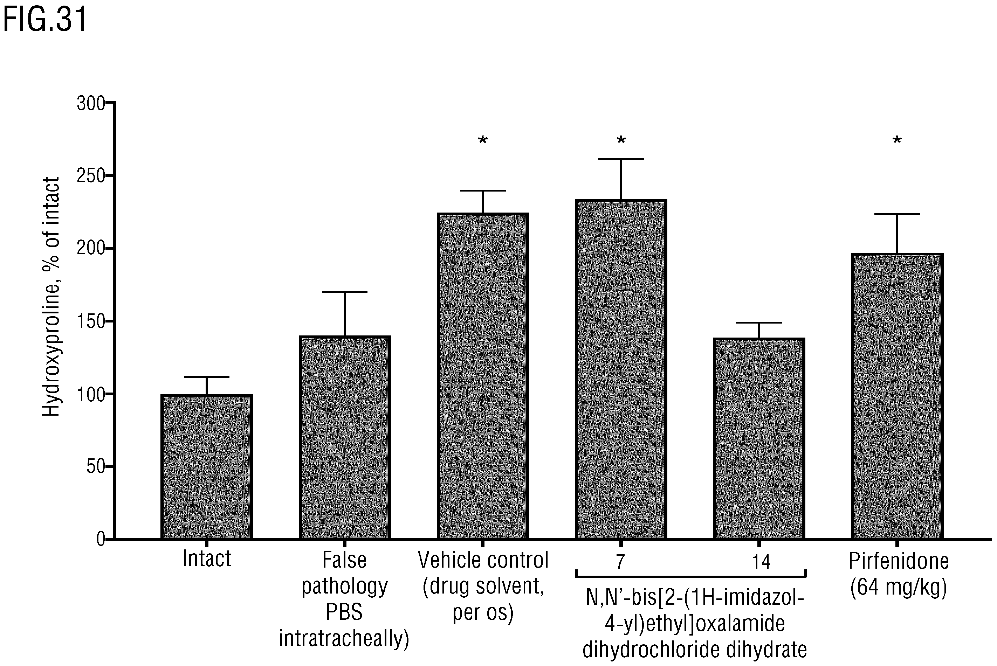

Under the influence of bleomycin, animals in the pathological control group exhibited a statistically significant increase in hydroxyproline levels compared to the intact group. The administration of N,N'-bis[2-(1H-imidazol-4-yl)ethyl]oxalamide dihydrochloride dihydrate resulted in a statistically significant reduction in hydroxyproline levels (see Fig. 31), while the comparator drug Pirfenidone did not show a statistically significant effect.

Thus, the use of N,N'-bis[2-(1H-imidazol-4-yl)ethyl]oxalamide dihydrochloride dihydrate under the therapeutic treatment regimen in a model of bleomycin-induced pulmonary fibrosis led to a statistically significant decrease in hydroxyproline levels, one of the key parameters evaluated in the study of fibrotic changes in the lungs.

### Biological Example 5. Study of the effect of N,N'-bis[2-(1H-imidazol-4-yl)ethyl]oxalamide dihydrochloride dihydrate on the development of bleomycin-induced pulmonary fibrosis, prophylactic treatment regimen

Pulmonary fibrosis was induced by a single intratracheal administration of the cytostatic bleomycin, dissolved in PBS, at a dose of 16 units/kg in a volume of 200 µL. The prophylactic treatment regimen involved the administration of N,N'-bis[2-(1H-imidazol-4-yl)ethyl]oxalamide dihydrochloride dihydrate from day 0 to day 27 after the induction of pulmonary fibrosis in male Agouti guinea pigs, aged 4-5 weeks and weighing 180-200 g. The test compound was administered intragastrically at doses of 0.9 or 3.5 mg/kg once daily.

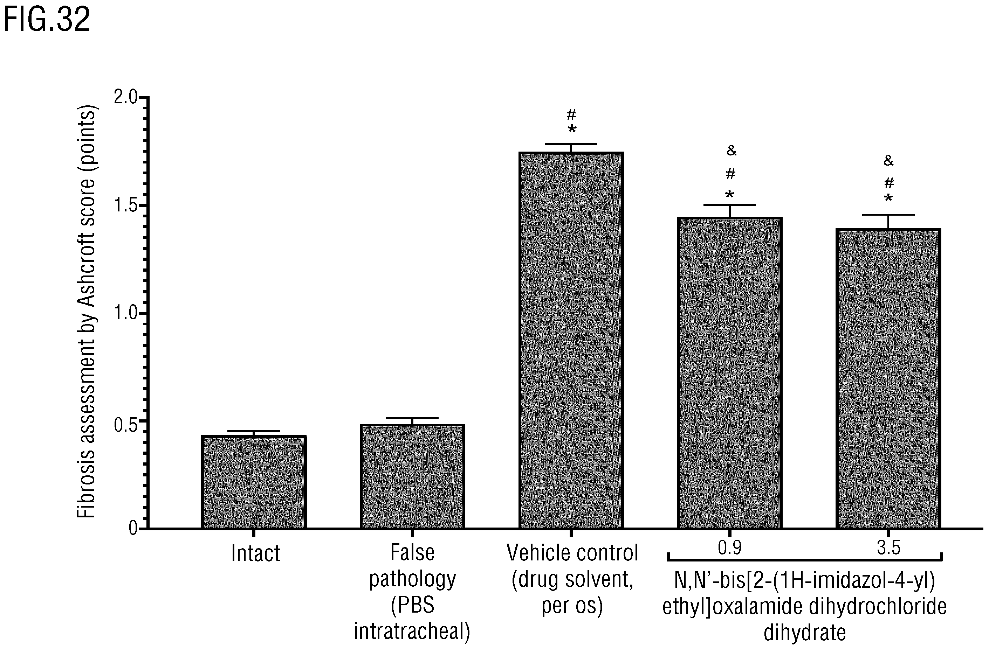

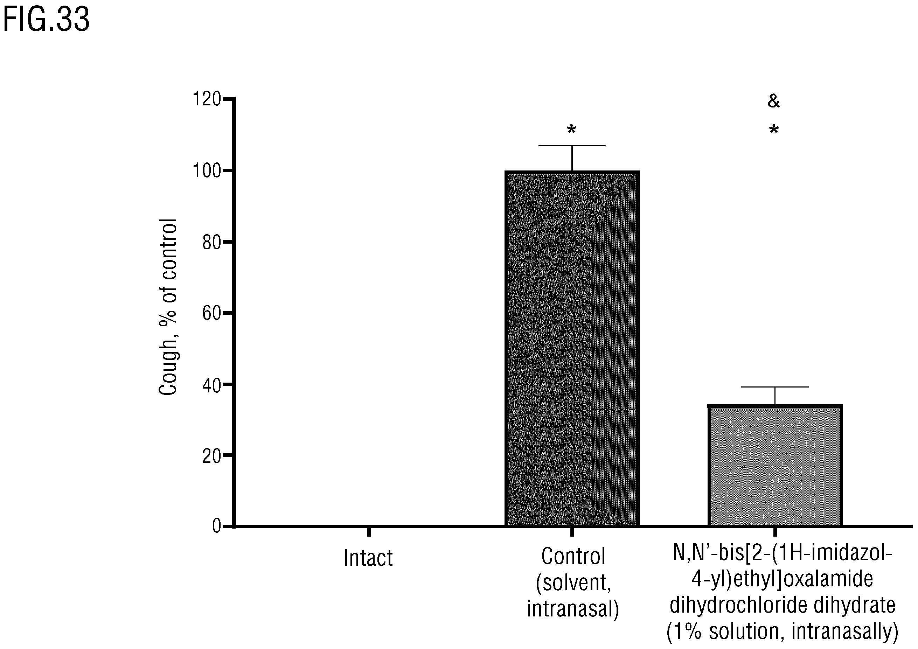

The modified Ashcroft scale was used for the semiquantitative assessment of the severity of pulmonary fibrosis [J Clin Pathol 1988; 41:467-70; Eur Respir J 1999; 13:71-77]. Histological sections from animals in the pathological control group showed fibrotic changes in the lungs, corresponding to high scores on the Ashcroft scale (Fig. 33). Administration of N,N'-bis[2-(1H-imidazol-4-yl)ethyl]oxalamide dihydrochloride dihydrate resulted in a statistically significant reduction in the Ashcroft score (Fig. 32).

Thus, the use of N,N'-bis[2-(1H-imidazol-4-yl)ethyl]oxalamide dihydrochloride dihydrate in the bleomycin-induced pulmonary fibrosis model under the prophylactic treatment regimen significantly reduced the severity of pulmonary fibrosis compared to the pathological control group.

### Biological Example 6. Study of the activity of N,N'-bis[2-(1H-imidazol-4-yl)ethyl]oxalamide dihydrochloride dihydrate in a capsaicin-induced cough model in guinea pigs

The study was conducted on male Agouti guinea pigs. Groups were formed using a selection method based on body weight as the primary criterion (the variation in initial body weight between and within groups did not exceed ±10%). Each group consisted of 10 animals.

The animals were inhaled with a capsaicin solution (30 µM) prepared in a mixture of ethanol and Tween-80, using a compression nebulizer for 5 minutes. The drug candidate, N,N'-bis[2-(1H-imidazol-4-yl)ethyl]oxalamide dihydrochloride dihydrate, was administered as a 1% solution intranasally, once, at 10 µL per nostril 5 minutes before capsaicin inhalation and once into the throat using a spray bottle with a metered-dose nozzle at a volume of 50 µL 5 minutes before capsaicin inhalation. Control animals received the solvent, and the intact group received no treatment. Antitussive activity was evaluated by counting the number of coughs within 15 minutes from the start of capsaicin inhalation, with results expressed as a percentage relative to the control group.

Descriptive statistics were applied to all data, including the calculation of the arithmetic mean (Mean) and the standard error of the mean (SEM). The normality of the data distribution obtained in the experiment was assessed using the Shapiro-Wilk test. In cases where the data did not follow a normal distribution, intergroup differences were evaluated using one-way ANOVA with Dunn's post hoc test. In cases of normal distribution, one-way ANOVA with Tukey's post hoc test was used for multiple group comparisons. Statistical analysis was performed using GraphPad Prism, and differences were considered statistically significant at p < 0.05.

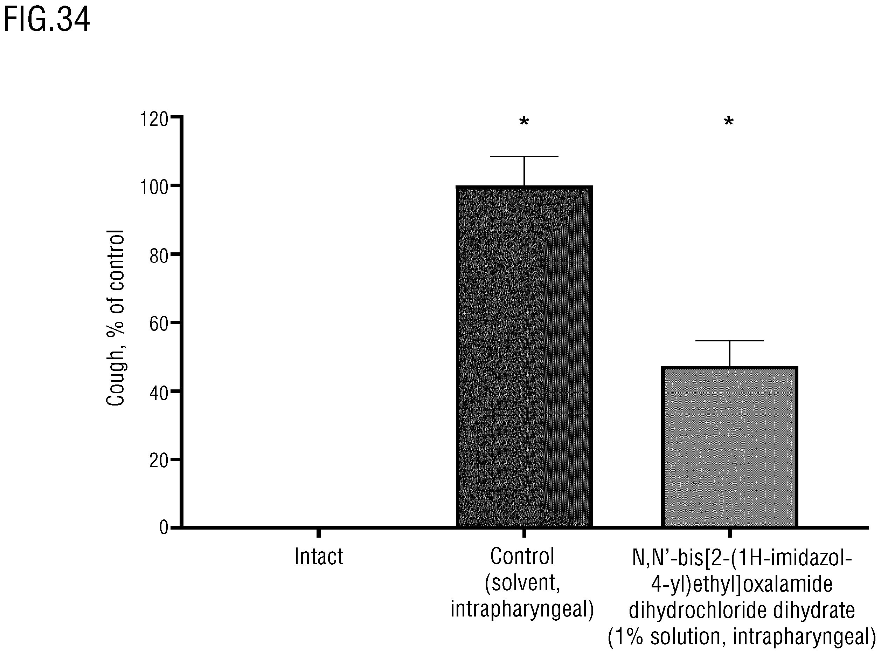

The results are shown in Figs 33 and 34, where Fig. 33 illustrates intranasal inhalation, as described above, and Fig. 34 shows administration into the throat.

The results demonstrate that a single administration of N,N'-bis[2-(1H-imidazol-4-yl)ethyl]oxalamide dihydrochloride dihydrate as a 1% solution results in a statistically significant reduction in coughing episodes in guinea pigs.

### Biological Example 7. Study of the activity of N,N'-bis[2-(1H-imidazol-4-yl)ethyl]oxalamide dihydrochloride dihydrate in an acute cough model induced by citric acid inhalation in guinea pigs

The study was conducted on male Agouti guinea pigs. The animals were exposed to inhalation of a citric acid solution (0.4 M in physiological saline) for 10 minutes. The test compound was administered intragastrically at doses of 14 and 28 mg/kg, 15 minutes before citric acid inhalation. Antitussive activity was evaluated by counting the number of coughs during over 15 minutes: during the 10-minute inhalation of citric acid and for 5 minutes after inhalation.

The results demonstrated that intragastric administration of the compound dose-dependently reduced the number of coughs in the acute cough model induced by citric acid inhalation in guinea pigs (Table 40).

**Table 40. Number of coughs in the acute cough model induced by citric acid inhalation in guinea pigs (Mean ± SEM, n = 10)**

| Group | Drug dose, mg/kg | Number of Coughs | |
|---|---|---|---|
| | | 0-10 min | 11-15 min |
| Control (Substance Solvent, per os) | | 21.9±0.8 | 1.2±0.3 |
| N,N'-bis[2-(1H-imidazol-4-yl)ethyl]oxalamide dihydrochloride dihydrate | 14 | 5.4±0.5 & | 0.0±0.0 & |
| | 28 | 3.3±0.8 & | 0.0±0.0 & |

| | | | |
|---|---|---|---|
| Notes: & -difference from the control group (one-way ANOVA at p<0.05). | | | |

### Biological Example 8. Study of the activity of N,N'-bis[2-(1H-imidazol-4-yl)ethyl]oxalamide dihydrochloride dihydrate of in a liver fibrosis model induced by intraperitoneal administration of CCl₄

The study was conducted on male C57Bl/6 mice. The animals received intraperitoneal injections of CCl₄ three times per week, dissolved in olive oil at a 1:9 ratio (dose of 2 mL/kg). The test compound was administered intragastrically at doses of 0.3, 3, 30, and 90 mg/kg, once daily, strictly every 24 hours, with the first administration occurring immediately after the first injection of CCl₄. On day 28, body weight, liver weight, and histological analysis of the liver were assessed. Body weight was also measured on day 0.

The study results demonstrated that intragastric administration of the test compound reduced liver fibrosis, decreased liver weight, and had a positive effect on body weight gain in the liver fibrosis model induced by intraperitoneal administration of CCl₄ (Table 41).

**Table 41. Body weight gain, liver weight, and percentage of liver fibrosis in the liver fibrosis model induced by intraperitoneal administration of CCl4 in mice (Mean ± SEM, n = 15)**

| Group | Dose, mg/kg | Body Weight Gain, % | Liver Weight, % of Body Weight | % Liver fibrosis |
|---|---|---|---|---|
| Intact | | 115.59±0.52 | 4.41±0.06 | 9.60±0.50 |
| False pathology (Solvent, per os) | | 116.32±0.46 | 4.20±0.12 | 9.47+0.29 |
| Control (Substance Solvent, per os) | | 103.29±0.73*# | 6.16±0.12*# | 29.97±0.47*# |
| Test compound | 0.3 | 106.02±0.93*# | 6.04±0.13*# | 26.93±0.75*# |
| | 3 | 107.72±0.80*#& | 5.84±0.17*# | 25.93±0.49*#& |
| | 30 | 107.79±1.02*#& | 6.16±0.29*# | 25.27±0.63*#& |
| | 90 | 107.22±0.97*#& | 5.37±0.17*#& | 25.93±0.43*#& |

| | | | | |
|---|---|---|---|---|
| Notes: * - Difference from the intact group (one-way ANOVA, p < 0.05). # - Difference from the false pathology group (one-way ANOVA, p < 0.05). & - Difference from the control group (one-way ANOVA, p < 0.05). | | | | |

### Examples of Pharmaceutical Compositions

The salts of N,N'-bis[2-(1H-imidazol-4-yl)ethyl]oxalamide of formula (I) described in this invention can be used for the prevention and/or treatment of diseases in humans or animals in the following formulations:
Tablet I (mg/tablet)
   N,N'-bis[2-(1H-imidazol-4-yl)ethyl]oxalamide dihydrochloride dihydrate - 0.5
   Microcrystalline cellulose - 66.5
   Sodium carboxymethyl starch - 2.3
   Magnesium stearate - 0.7
Tablet II (mg/tablet)
   N,N'-bis[2-(1H-imidazol-4-yl)ethyl]oxalamide dihydrochloride dihydrate - 5.0
   Microcrystalline cellulose - 62.0
   Sodium carboxymethyl starch - 2.3
   Magnesium stearate - 0.7
Tablet III (mg/tablet)
   N,N'-bis[2-(1H-imidazol-4-yl)ethyl]oxalamide dihydrochloride dihydrate - 50
   Microcrystalline cellulose - 620
   Sodium carboxymethyl starch 23
   Magnesium stearate - 7
Tablet IV (mg/tablet)
   N,N'-bis[2-(1H-imidazol-4-yl)ethyl]oxalamide dihydrochloride dihydrate - 50
   Lactose, Ph. Eur. - 223.75
   Sodium croscarmellose - 6.0
   Corn starch - 15
   Polyvinylpyrrolidone (5% w/v paste) - 2.25
   Magnesium stearate - 3.0
Tablet V (mg/tablet)
   N,N'-bis[2-(1H-imidazol-4-yl)ethyl]oxalamide dihydrochloride dihydrate - 200
   Lactose, Ph. Eur. - 182.75
   Sodium croscarmellose - 12.0
   Corn starch (5% w/v paste) - 2.25
   Magnesium stearate - 3.0
Tablet VI (mg/tablet)
   N,N'-bis[2-(1H-imidazol-4-yl)ethyl]oxalamide dihydrochloride dihydrate - 100
   Anhydrous lactose, Ph. Eur. - 116.82
   Pregelatinized starch, Ph. Eur. - 24.50
   Hydrogenated castor oil - 3.68

Capsule I (mg/capsule)
   N,N'-bis[2-(1H-imidazol-4-yl)ethyl]oxalamide dihydrochloride dihydrate - 10
   Lactose, Ph. Eur. - 488.5
   Magnesium oxide - 1.5
Capsule II (mg/capsule)
   N,N'-bis[2-(1H-imidazol-4-yl)ethyl]oxalamide dihydrochloride dihydrate - 100
   Anhydrous lactose, Ph. Eur. - 116.82
   Pregelatinized starch, Ph. Eur. - 24.50
   Hydrogenated castor oil - 3.68

   Injection Composition I (50 mg/mL)
   N,N'-bis[2-(1H-imidazol-4-yl)ethyl]oxalamide dihydrochloride dihydrate - 5.0% w/v
   1M sodium hydroxide solution - 15.0% w/v
   1M hydrochloric acid solution - to pH 7.6
   Polyethylene glycol 400 - 4.5% w/v
   Water for injections - to 100%

   Ointment
   N,N'-bis[2-(1H-imidazol-4-yl)ethyl]oxalamide dihydrochloride dihydrate - 40 mg
   Ethanol - 300 µL
   Water - 300 µL
   1-dodecylazacycloheptanone - 50 µL
   Propylene glycol - to 1 mL
Capsule III, Hard Gelatin Capsule, Size 2 (mg/capsule)
   N,N'-bis[2-(1H-imidazol-4-yl)ethyl]oxalamide dihydrochloride dihydrate - 100 mg
   Anhydrous lactose - 85.0
   Pregelatinized starch (Ph. Eur. 1500) - 12.00
   Magnesium stearate - 3.0
   Capsule weight - 260.73
Capsule IV, Hard Gelatin Capsule, Size 2 (mg/capsule)
   N,N'-bis[2-(1H-imidazol-4-yl)ethyl]oxalamide dihydrochloride dihydrate - 100 mg
   Lactose monohydrate - 85.0
   Pregelatinized starch (Ph. Eur. 1500) - 12.00
   Magnesium stearate - 3.0
   Total capsule weight - 260.73

These formulations can be prepared according to standard pharmaceutical methods. Tablets (I)-(II) may be coated with an enteric coating using, for example, cellulose acetate phthalate.

### Stability of pharmaceutical compositions during storage

The stability of the pharmaceutical compositions in the capsules described above was evaluated under storage conditions. The results are summarized in Table 42. The second row of Table 42 presents the standard stability requirements that the compositions must meet.

**Table 42. Stability study of pharmaceutical compositions**

| Storage period | Storage conditions | Description | Dissolution | Related impurities | Quantitative determination |
|---|---|---|---|---|---|
| Specifi cation limits | - | Hard gelatin capsules. Contents - white or nearly white powder | Not less than 80% (Q) (C₁₂H₁₆N₆O₂ ·2HCl ·2 H₂O) N,N'-bis[2-(1H-imidazol-4-yl) ethyl] oxalami de dihydrochloride dihydrate after 30 min | Single unidentified impurity ≤ 0.20%; Total impurities ≤ 1.0% | 95.0 mg to 105.0 mg of compound 1 in the form of dihydrochloride dihydrate in capsule |

| The composition corresponds to the composition of Capsule II given above | | | | | |
|---|---|---|---|---|---|
| **0 months** | +25°C/60% | Complies | Complies 98.85%. | Complies (Single impurity below Limit of Quantificatio n (LOQ) ; Total impurities below LOQ) | Corresponds to the composition of Capsule II, 99.65 mg |
| **18 months** | +25°C/60% | Complies | Complies 100.08%. | Complies (Single impurity below LOQ; Total impurities below LOQ) | Corresponds to the composition of Capsule II, 103.49 mg |
| **24 months** | +25°C/60% | Complies | Complies 101.71%. | Complies (Single impurity below LOQ; Total impurities below LOQ) | Corresponds to the composition of Capsule II, 99.70 mg |
| **36 months** | +25°C/60% | Complies | Complies 99.79%. | Complies (Single impurity below LOQ; Total impurities below LOQ) | Corresponds to the composition of Capsule II, 101.68 mg |

| The composition corresponds to the composition of Capsule II given above | | | | | |
|---|---|---|---|---|---|
| **0 months** | | Complies | Complies 100.5% | Complies Single impurity below 0.10%; Total impurities below 0.01% | Corresponds, 100.9% |
| **3 months** | +25°C/60% | Complies | Complies 98.5% | Complies Single impurity below 0.09%; Total impurities below 0.09% | Corresponds, 101.2% |
| | +30°C/65% | Complies | Complies 99.6% | Complies Single impurity below 0.09%; Total impurities below 0.09% | Corresponds, 99.1% |
| | +40°C/75% | Complies | Complies 99.0% | Complies Single impurity below 0.09%; Total impurities below 0.09% | Corresponds, 99.0% |
| **6 months** | +25°C/60% | Complies | Complies 98.6% | Complies Single impurity below 0.09%; Total impurities below 0.16% | Corresponds, 100.1% |
| | +30°C/65% | Complies | Complies 98.3% | Complies Single impurity below 0.09%; Total impurities below 0.17% | Corresponds, 99.6% |
| | +40°C/75% | Complies | Complies 98.5% | Complies | Corresponds, 98.1% |
| | | | | Single impurity below 0.09%; Total impurities below 0.16% | |

| The composition corresponds to the composition of Capsule IV given above | | | | | |
|---|---|---|---|---|---|
| **0 months** | | Complies | Complies 102.2% | Complies Single impurity below 0.10%; Total impurities below 0.01% | Corresponds, 104.3% |
| **3 months** | +25°C/60% | Complies | Complies 103.7% | Complies Single impurity below 0.09%; Total impurities below 0.09% | Corresponds, 99.2% |
| | +30°C/65% | Complies | Complies 98.2% | Complies Single impurity below 0.90%; Total impurities below 0.09% | Corresponds, 101.1% |
| | +40°C/75% | Complies | Complies 101.5% | Complies Single impurity below 0.09%; Total impurities below 0.09% | Corresponds, 100.3% |
| **6 months** | +25°C/60% | Complies | Complies 100.5% | Complies Single impurity below 0.09%; Total impurities below 0.16% | Corresponds, 99.6% |
| | +30°C/65% | Complies | Complies 103.5% | Complies Single impurity below 0.08%; Total impurities below 0.15% | Corresponds, 101.1% |
| | +40°C/75% | Complies | Complies 104.5% | Complies Single impurity below 0.09%; Total impurities below 0.16% | Corresponds, 99.8% |

Thus, the experiments demonstrated that N,N'-bis[2-(1H-imidazol-4-yl)ethyl]oxalamide dihydrochloride dihydrate exhibits both therapeutic and prophylactic efficacy against fibrotic diseases, including pulmonary fibrosis. Moreover, in contrast to the free base, this compound possesses pharmacokinetic characteristic properties that make it suitable for use as a pharmaceutical agent for the treatment and/or prevention of fibrosis in the liver, kidneys, pancreas, and heart (myocardial fibrosis), as well as for the prevention and treatment of pulmonary fibrosis, preferably of idiopathic origin. The salts of general formula (I) have also been tested and demonstrate similar results

Although the invention has been described with reference to the disclosed embodiments, it will be apparent to those skilled in the art that the detailed experiments are provided solely for illustrative purposes and should not be construed as limiting the scope of the invention in any way. It should be understood that various modifications can be made without departing from the essence of the present invention.

## Claims

1. A compound of Formula (I) 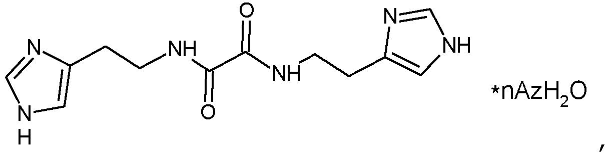
wherein A is selected from the group including HCl, H₃PO₄, maleic acid, malonic acid, fumaric acid, HI, HBr, H₂SO₄, benzenesulfonic acid, tartaric acid, malic acid, p-toluenesulfonic acid, oxalic acid, glycolic acid, glutaric acid, camphorsulfonic acid, methanesulfonic acid, formic acid, ethanesulfonic acid, mandelic acid, nicotinic acid, succinic acid, and adipic acid;
n represents 1 or 2; and
z represents an integer from 0 to 5.

2. The compound of claim 1 selected from the group including:
N,N'-bis[2-(1H-imidazol-4-yl)ethyl]oxalamide dihydrochloride dihydrate of Formula 1 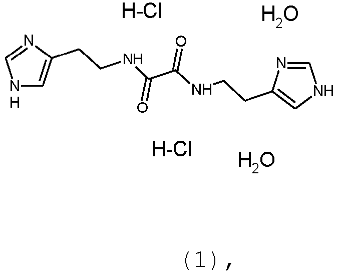
N,N'-bis[2-(1H-imidazol-4-yl)ethyl]oxalamide diphosphate of Formula 2 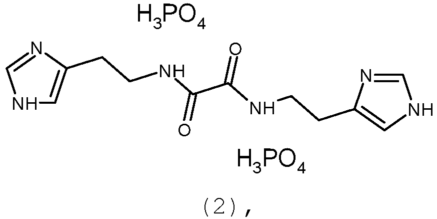
N,N'-bis[2-(1H-imidazol-4-yl)ethyl]oxalamide dimaleinate dihydrate of Formula 3 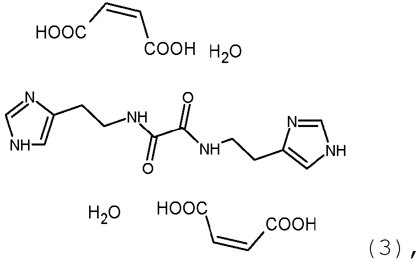
N,N'-bis[2-(1H-imidazol-4-yl)ethyl]oxalamide dimalonate of Formula 4 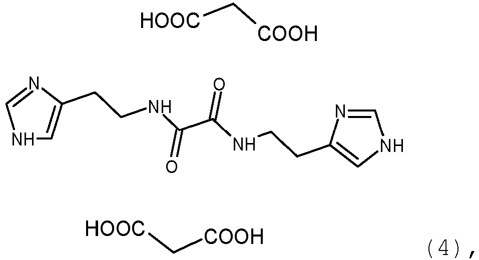
N,N'-bis[2-(1H-imidazol-4-yl)ethyl]oxalamide fumarate of Formula 5 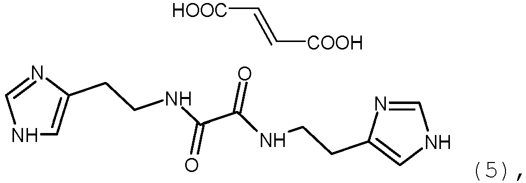
N,N'-bis[2-(1H-imidazol-4-yl)ethyl]oxalamide dihydroiodide dihydrate of Formula 6 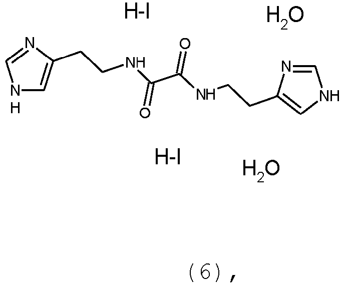
N,N'-bis[2-(1H-imidazol-4-yl)ethyl]oxalamide dihydrobromide dihydrate of Formula 7 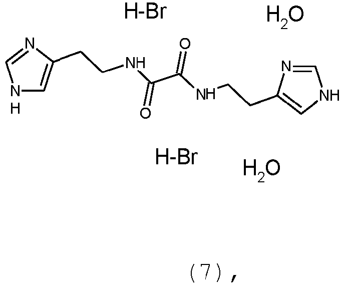
N,N'-bis[2-(1H-imidazol-4-yl)ethyl]oxalamide sulfate trihydrate of Formula 8 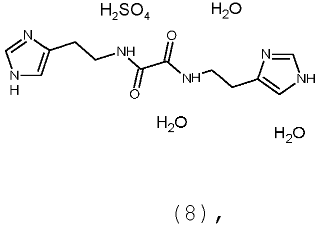
N,N'-bis[2-(1H-imidazol-4-yl)ethyl]oxalamide dibenzenesulfonate of Formula 9 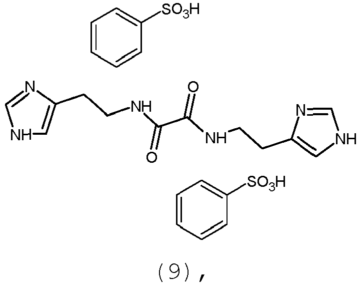
N,N'-bis[2-(1H-imidazol-4-yl)ethyl]oxalamide L,D-tartrate of Formula 10 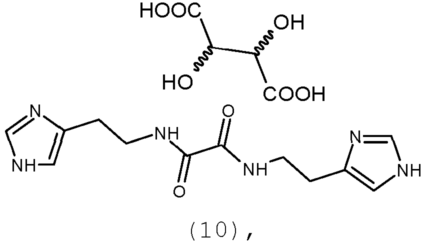
N,N'-bis[2-(1H-imidazol-4-yl)ethyl]oxalamide L-malate of Formula 11 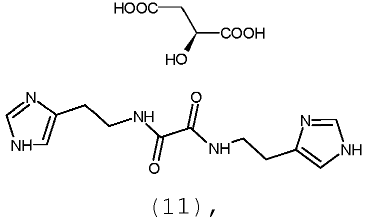
N,N'-bis[2-(1H-imidazol-4-yl)ethyl]oxalamide di-p-toluenesulfonate of Formula 12 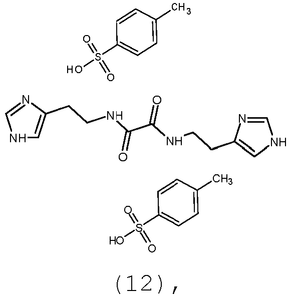
N,N'-bis[2-(1H-imidazol-4-yl)ethyl]oxalamide oxalate of Formula 13 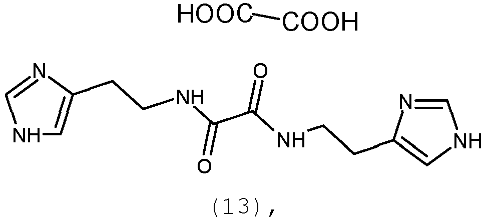
N,N'-bis[2-(1H-imidazol-4-yl)ethyl]oxalamide dioxalate of Formula 14 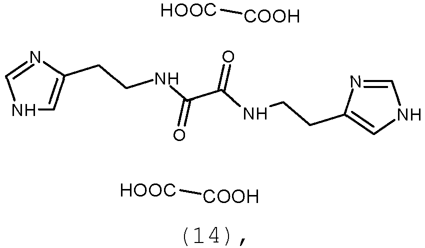
N,N'-bis[2-(1H-imidazol-4-yl)ethyl]oxalamide diadipinate monohydrate of Formula 15: 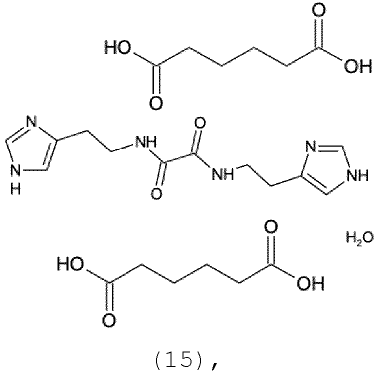
N,N'-bis[2-(1H-imidazol-4-yl)ethyl]oxalamide dimetansulfonate dihydrate of Formula 16: 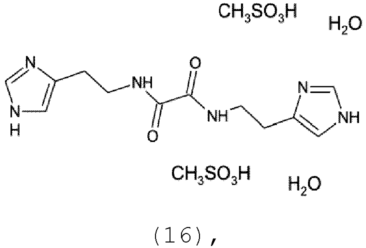
N,N'-bis[2-(1H-imidazol-4-yl)ethyl]oxalamide succinate of Formula 17: 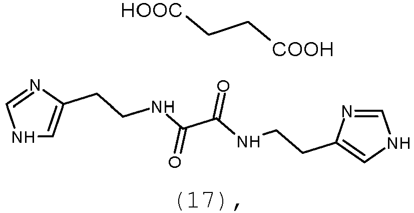 N,N'-bis[2-(1H-imidazol-4-yl)ethyl]oxalamide di-D-malate of Formula 18: 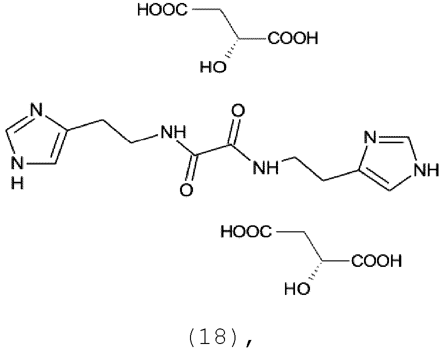
N,N'-bis[2-(1H-imidazol-4-yl)ethyl]oxalamide D-malate of Formula 19: 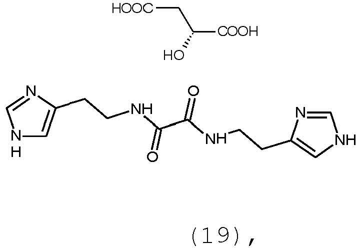
N,N'-bis[2-(1H-imidazol-4-yl)ethyl]oxalamide diglycolate of Formula 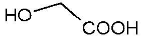 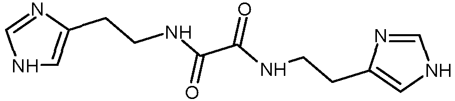 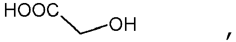
N,N'-bis[2-(1H-imidazol-4-yl)ethyl]oxalamide glutarate dihydrate of Formula 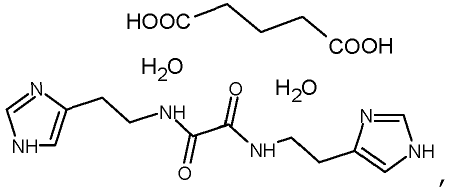
N,N'-bis[2-(1H-imidazol-4-yl)ethyl]oxalamide dicamphorsulfonate of Formula 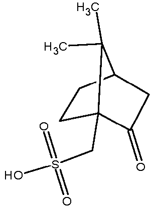 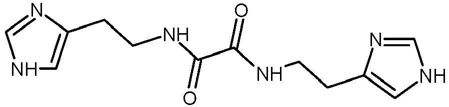 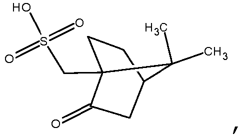
N,N'-bis[2-(1H-imidazol-4-yl)ethyl]oxalamide diformiate of Formula 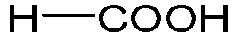 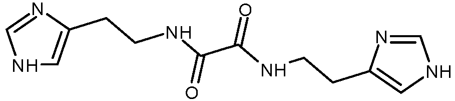 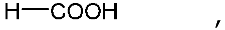
N,N'-bis[2-(1H-imidazol-4-yl)ethyl]oxalamide diethansulfonate of Formula 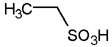 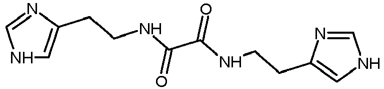 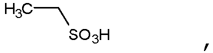
N,N'N,N'-bis[2-(1H-imidazol-4-yl)ethyl]oxalamide malonate dihydrate of Formula 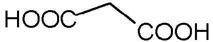 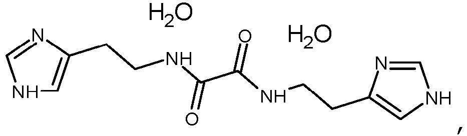
N,N'-bis[2-(1H-imidazol-4-yl)ethyl]oxalamide dimandelate of Formula 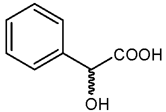 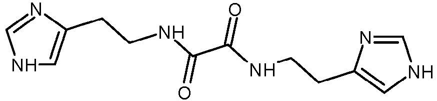 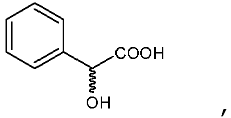
N,N'-bis[2-(1H-imidazol-4-yl)ethyl]oxalamide dinicotinate of Formula 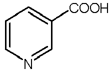 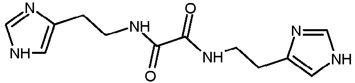 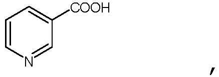
N,N'-bis[2-(1H-imidazol-4-yl)ethyl]oxalamide disulfate of Formula 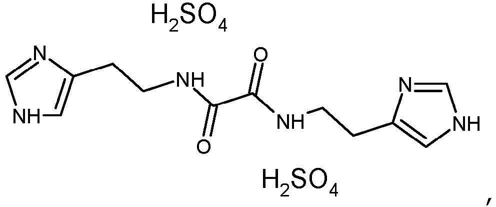
N,N'-bis[2-(1H-imidazol-4-yl)ethyl]oxalamide D-tartrate of Formula 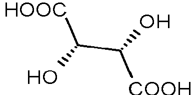 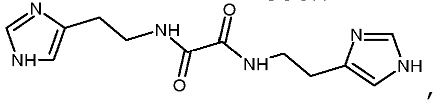
N,N'-bis[2-(1H-imidazol-4-yl)ethyl]oxalamide L-tartrate of Formula 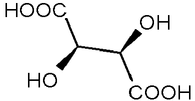 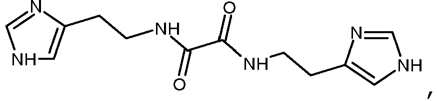
N,N'-bis[2-(1H-imidazol-4-yl)ethyl]oxalamide dihydroiodide of Formula 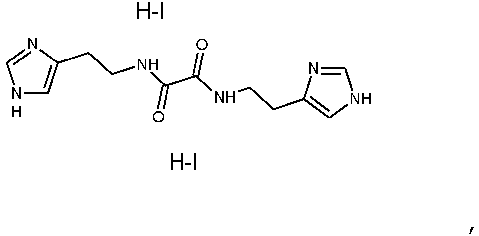 and
N,N'-bis[2-(1H-imidazol-4-yl)ethyl]oxalamide dihydrobromide of Formula

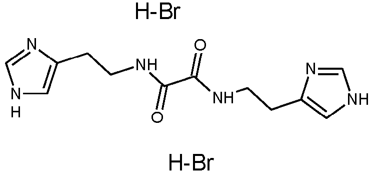

3. A crystalline form selected from the following table
| Number | name | peak positions in the powder X-ray diffraction pattern at 2θ, deg. (± 0.2°) |
|---|---|---|
| 1.1 | N,N'-bis[2-(1H-imidazol-4-yl)ethyl]oxalamide dihydrochloride dihydrate | 21.7, 23.3, and 24.2, preferably 14.6, 21.1, 21.7, 23.3, 24.2, 27.3, 29.0 and 34.7 |
| 1.2 | N,N'-bis[2-(1H-imidazol-4-yl)ethyl]oxalamide diphosphate | 20.4, 23.3, and 24.7, preferably 6.5, 12.5, 20.2, 20.4, 23.3, 23.4, 23.8, and 24.7 |
| 1.3 | N,N'-bis[2-(1H-imidazol-4-yl)ethyl]oxalamide dimaleinate dihydrate | 9.3, 21,2, and 25.4, preferably 9.3, 18.4, 18.6, 21.2, 22.2, 25.1, 25.4, and 29.9 |
| 1.4 | N,N'-bis[2-(1H-imidazol-4-yl)ethyl]oxalamide dimalonate | 6.5, 23.3, and 31.1, preferably 6.5, 13.9, 19.7, 23.3, 23.5, 25.5, 26.2, 31.1, and 37.3 |
| 1.5 | N,N'-bis[2-(1H-imidazol-4-yl)ethyl]oxalamide fumarate | 21.3, 23.6, and 27.5, preferably 16.5, 18.8, 18.9, 2.3, 21.4, 22.0, 22.5, 23.6, 24.3, 27.5, 28.1, and 28.6 |
| 1.6 | N,N'-bis[2-(1H-imidazol-4-yl)ethyl]oxalamide dihydroiodide dihydrate | 20.4, 22.6, and 26.2, preferably 18.2, 20.4, 22.6, 23.6, 26.2, 31.3, 33.3, and 36.5 |
| 1.7 | N,N'-bis[2-(1H-imidazol-4-yl)ethyl]oxalamide dihydrobromide dihydrate | 22.0, 22.8, and 25.0, preferably 12.0, 20.5, 21.2, 22.0, 22.8, 23.2, 23.7, 24.3, 25.0, 26.8, and 31.8 |
| 1.8 | N,N'-bis[2-(1H-imidazol-4-yl)ethyl]oxalamide sulfate trihydrate | 6.6, 21.3, and 24.52, preferably 6.3, 6.6, 11.9, 13.2, 15.9, 18.2, 21.1, 21.3, 22.9, 23.6, 24.5, 26.0, and 27.4 |
| 1.9 | N,N'-bis[2-(1H-imidazol-4-yl)ethyl]oxalamide dibenzenesulfonate | 20.9, 21.6, and 22.4, preferably 13.3, 16.2, 17.9, 18.8, 20.9, 21.6, 22.4, 22.6, 25.0, 25.1, and 26.6. |
| 1.10 | N,N'-bis[2-(1H-imidazol-4-yl)ethyl]oxalamide tartrate | 6.9, 19.9, and 23.6, preferably 6.9, 10.8, 18.4, 19.9, 21.1, 21.5, and 27.2. |
| 1.11 | N,N'-bis[2-(1H-imidazol-4-yl)ethyl]oxalamide L-malate | 19.3, 24.0, and 25.3, preferably 7.0, 11.5, 17.1, 19.3, 21.4, 23.9, 24.8, 25.32, and 32.6. |
| 1.12 | N,N'-bis[2-(1H-imidazol-4-yl)ethyl]oxalamide di-p-toluenesulfonate | 7.4, 15.7, and 23.1, preferably 7.4, 12.3, 13.2, 15.0, 15.7, 18.4, 18.5, 21.4, 22.3, 23.1, 24.6, 25.4, 25.7, 25.9, 26.7, 27.0, 27.6, 28.2, 30.2, 32.2, and 33.3 |
| 1.13 | N,N'-bis[2-(1H-imidazol-4-yl)ethyl]oxalamide | 16.5, 16.6, and 18.7, preferably 10.4, 16.5, 16.6, 18.7, 20.8, 22.0, 25.1, 25.3, 26.4, 33.6, and 43.2 |
| 1.14 | N,N'-bis[2-(1H-imidazol-4-yl)ethyl]oxalamide dioxalate | 24.1, 25.4, and 27.5, preferably **characterized by** peak positions in the powder X-ray diffraction pattern at 2θ, deg. (± 0.2°): 19.3, 22.4, 24.1, 25.4, 27.5, and 30.0 |
| 1.15 | N,N'-bis[2-(1H-imidazol-4-yl)ethyl]oxalamide diadipinate monohydrate | 19.5, 23.7, and 25.8, preferably **characterized by** peak positions in the powder X-ray diffraction pattern at 2θ, deg. (± 0.2°): 14.1, 18.9, 19.5, 20.9, 22.7, 23,7, 25.8, and 36.2 |
| 1.16 | N,N'-bis[2-(1H-imidazol-4-yl)ethyl]oxalamide dimetansulfonate dihydrate | 6.4, 23.9, and 22.5, preferably 6.4, 12.8, 19.0, 19.3, 22.3, 22.5, 22.9, 23.1, 23.9, 25.8, and 33.1 |
| 1.17 | N,N'-bis[2-(1H-imidazol-4-yl)ethyl]oxalamide succinate | 6.2, 21.2 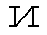 26.32, preferably 6.2, 16.9, 18.4, 18.6, 19.4, 20.4, 21.2, 26.3, 24.4, 25.62. |
| 1.18 | N,N'-bis[2-(1H-imidazol-4-yl)ethyl]oxalamide di-D-malate | 22.1, 22.9, and 23.6, preferably 5.9, 15.7, 19,5, 20.3, 21.4, 22.1, 22.9, 23.3, 23.6, 24.4, 28.0, and 32.3 |
| 1.19 | N,N'-bis[2-(1H-imidazol-4-yl)ethyl]oxalamide D-malate | 19.4, 23.5 and 24.1, preferably 17.1, 19.4, 21.5, 22.1, 23.0, 23.3, 23.5, 24.1, 24.8, 25.4 and 28.5 |

4. A crystalline form selected from the following table
| Number | name | Cell parameters |
|---|---|---|
| 2.1 | N,N'-bis[2-(1H-imidazol-4-yl)ethyl]oxalamide dihydrochloride dihydrate | monoclinic syngony, with unit cell edge lengths of 5.72 ± 0.50 Å, 24.20 ± 0.50 Å, and 6.95 ± 0.50 Å, and a unit cell angle of 104.04° ± 5.00° |
| 2.2 | N,N'-bis[2-(1H-imidazol-4-yl)ethyl]oxalamide diphosphate | triclinic syngony, with unit cell edge lengths of 4.90 ± 0.50 Å, 7.43 ± 0.50 Å, 14.02 ± 0.50 Å, and a unit cell angle of 103.84° ± 5.00°, 94.02° ± 5.00°, 97.90° ± 5.00° |
| 2.3 | N,N'-bis[2-(1H-imidazol-4-yl)ethyl]oxalamide dimaleinate dihydrate | triclinic syngony, with unit cell edge lengths of 4.97 ± 0.50 Å, 10.32 ± 0.50 Å, 13.53 ± 0.50 Å, and a unit cell angle of 109.72° ± 5.00°, 96.62° ± 5.00°, 98.80° ± 5.00° |
| 2.4 | N,N'-bis[2-(1H-imidazol-4-yl)ethyl]oxalamide dimalonate | triclinic syngony, with unit cell edge lengths of 4.59 ± 0.50 Å, 8.53 ± 0.50 Å, 14.01 ± 0.50 Å, and a unit cell angle of 103.91° ± 5.00°, 92.18° ± 5.00°, 96.82° ± 5.00° |
| 2.5 | N,N'-bis[2-(1H-imidazol-4-yl)ethyl]oxalamide fumarate | monoclinic syngony, with unit cell edge lengths of 12.87 ± 0.50 Å, 5.10 ± 0.50 Å, 14.45 ± 0.50 Å, and a unit cell angle of 104.36° ± 5.00° |
| 2.6 | N,N'-bis[2-(1H-imidazol-4-yl)ethyl]oxalamide dihydroiodide dihydrate | triclinic syngony, with unit cell edge lengths of 7.76 ± 0.50 Å, 8.04 ± 0.50 Å, 8.99 ± 0.50 Å, and a unit cell angle of 88.05° ± 5.00°, 74.66° ± 5.00°, 70.66° ± 5.00° |
| 2.7 | N,N'-bis[2-(1H-imidazol-4-yl)ethyl]oxalamide dihydrobromide dihydrate | triclinic syngony, with unit cell edge lengths of 5.11 ± 0.50 Å, 7.80 ± 0.50 Å, 12.65 ± 0.50 Å, and a unit cell angle of 104.78° ± 5.00°, 96.31° ± 5.00°, 100.38° ± 5.00° |
| 2.8 | N,N'-bis[2-(1H-imidazol-4-yl)ethyl]oxalamide sulfate trihydrate | monoclinic syngony, with unit cell edge lengths of 8.22 ± 0.50 Å, 8.77 ± 0.50 Å, 27.13 ± 0.50 Å, and a unit cell angle of 97.61° ± 5.00° |
| 2.9 | N,N'-bis[2-(1H-imidazol-4-yl)ethyl]oxalamide dibenzenesulfonate | monoclinic syngony, with unit cell edge lengths of 9.61 ± 0.50 Å, 10.74 ± 0.50 Å, 12.99 ± 0.50 Å, and a unit cell angle of 101.92° ± 5.00° |
| 2.10 | N,N'-bis[2-(1H-imidazol-4-yl)ethyl]oxalamide L,D-tartrate | triclinic syngony, with unit cell edge lengths of 9.00 ± 0.50 Å, 9.16 ± 0.50 Å, 13.54 ± 0.50 Å, and a unit cell angle of 108.59° ± 5.00°, 91.87° ± 5.00°, 112.10° ± 5.00° |
| 2.11 | N,N'-bis[2-(1H-imidazol-4-yl)ethyl]oxalamide L-malate | triclinic syngony, with unit cell edge lengths of 4.68 ± 0.50 Å, 7.81 ± 0.50 Å, 12.74 ± 0.50 Å, and a unit cell angle of 81.64° ± 5.00°, 81.79° ± 5.00°, 80.66° ± 5.00° |
| 2.12 | N,N'-bis[2-(1H-imidazol-4-yl)ethyl]oxalamide di-p-toluenesulfonate | monoclinic syngony, with unit cell edge lengths of 7.25 ± 0.50 Å, 8.06 ± 0.50 Å, 23.86 ± 0.50 Å, and a unit cell angle of 95.81° ± 5.00° |
| 2.13 | N,N'-bis[2-(1H-imidazol-4-yl)ethyl]oxalamide oxalate | monoclinic syngony, with unit cell edge lengths of 14.91 ± 0.50 Å, 21.31 ± 0.50 Å, 22.43 ± 0.50 Å, and a unit cell angle of 22.43° ± 5.00° |
| 2.14 | N,N'-bis[2-(1H-imidazol-4-yl)ethyl]oxalamide dioxalate | monoclinic syngony, with unit cell edge lengths of 10.84 ± 0.50 Å, 5.64 ± 0.50 Å, 16.10 ± 0.50 Å, and a unit cell angle of 100.51° ± 5.00° |
| 2.15 | N,N'-bis[2-(1H-imidazol-4-yl)ethyl]oxalamide diadipinate monohydrate | monoclinic syngony, with unit cell edge lengths of 4.99 ± 0.50 Å, 7.92 ± 0.50 Å, 18.33 ± 0.50 Å, and a unit cell angle of 82.98° ± 5.00°, 88.26° ± 5.00° 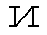 77.81° ± 5.00° |
| 2.16 | N,N'-bis[2-(1H-imidazol-4-yl)ethyl]oxalamide dimetansulfonate dihydrate | monoclinic syngony, with unit cell edge lengths of 5.85 ± 0.50 Å, 7.19 ± 0.50 Å, 14.02 ± 0.50 Å, and a unit cell angle of 97.43° ± 5.00°, 98.30° ± 5.00°, and 102.48° ± 5.00° |
| 2.17 | N,N'-bis[2-(1H-imidazol-4-yl)ethyl]oxalamide succinate | monoclinic syngony, with unit cell edge lengths of 13.02 ± 0.50 Å, 5.08 ± 0.50 Å, 14.51 ± 0.50 Å, and a unit cell angle of 104.87° ± 5.00° |
| 2.18 | A crystalline form of N,N'-bis[2-(1H-imidazol-4-yl)ethyl]oxalamide di-D-malate | monoclinic syngony, with unit cell edge lengths of 5.03 ± 0.50 Å, 7.83 ± 0.50 Å, 15.05 ± 0.50 Å, and a unit cell angle of 83.89° ± 5.00°, 84.35° ± 5.00°, 78.31° ± 5.00° |
| 2.19 | N,N'-bis[2-(1H-imidazol-4-yl)ethyl]oxalamide D-malate | monoclinic syngony, with unit cell edge lengths of 9.54 ± 0.50 Å, 11.86 ± 0.50 Å, 16.02 ± 0.50 Å, and a unit cell angle of 101.01° ± 5.00° |

5. A method for preparing a crystalline form of the compound of Formula (I) according to claims 1 to 4, comprising the steps of:
a) providing N,N'-bis[2-(1H-imidazol-4-yl)ethyl]oxalamide;
b) preparing a solution of an acid in water and/or an organic solvent and/or a mixture of solvents;
c) mixing the components from steps (a) and (b) to obtain a solution of a salt of N,N'-bis[2-(1H-imidazol-4-yl)ethyl]oxalamide;
d) crystallizing the obtained salt by diluting the reaction mixture with an organic solvent;
e) isolating the obtained salt
by removing the solvent from the reaction mixture under vacuum, dissolving the residue in isopropanol, and maintaining the resulting solution at +5°C until salt crystals precipitate; or
by crystalizing from water;
and
f) separating salt crystals from the solvent.

6. The method according to claim 5, wherein the solvent for the acid in step (b) is water.

7. The method according to claims 5 or 6, wherein the N,N'-bis[2-(1H-imidazol-4-yl)ethyl]oxalamide in step (a) is provided in solid form or as a suspension or solution in an organic solvent, preferably in acetone.

8. The method according to any one of claims 5 to 7, wherein the temperature in step (c) is between 50 and 92°C.

9. The method according to any one of claims 5 to 8, wherein in step (d) during crystallization, the mixture is cooled to a temperature of 5 to 25°C and stirred for 30 to 120 minutes.

10. The compound according to any one of claims 1 to 4 for use as a medicament.

11. The compound according to claim 10 for use as a medicament in the treatment and/or prevention of fibrosis, preferably of idiopathic fibrosis.

12. The compound according to claim 11, wherein the fibrosis is selected from pulmonary fibrosis, hepatic fibrosis, pancreatic fibrosis, cardiac fibrosis (myocardial fibrosis), and renal fibrosis.

13. The compound according to claim 10 for use as a medicament in the treatment and/or prevention of cough, preferably the cough associated with idiopathic pulmonary fibrosis.

14. A pharmaceutical composition comprising an effective amount of the compound according to any one of claims 1 to 4 and a pharmaceutically acceptable carrier.

15. The pharmaceutical composition according to claim 14, wherein the composition is provided in the form of a solid gelatin capsule, comprising N,N'-bis[2-(1H-imidazol-4-yl)ethyl]oxalamide dihydrochloride dihydrate, lactose, starch, and magnesium stearate.

16. The pharmaceutical composition according to claim 15, which comprises the following components (mg per capsule):
N,N'-bis[2-(1H-imidazol-4-yl)ethyl]oxalamide dihydrochloride dihydrate: 80-120 mg,
Anhydrous lactose: 75-90 mg,
Pregelatinized starch 1500: 7-15 mg, and
Magnesium stearate: 1-5 mg.

17. The pharmaceutical composition according to claim 15, which comprises the following components (mg per capsule):
N,N'-bis[2-(1H-imidazol-4-yl)ethyl]oxalamide dichlorhydrate dihydrate: 80-120 mg,
Lactose monohydrate: 75-90 mg,
Pregelatinized starch 1500: 7-15 mg, and
Magnesium stearate: 1-5 mg.
